(19) **Europäisches Patentamt / European Patent Office / Office européen des brevets**

(11) **EP 4 378 577 A2**

(12) # EUROPEAN PATENT APPLICATION

(43) Date of publication:
05.06.2024   Bulletin 2024/23

(21) Application number: 24165451.6

(22) Date of filing: 07.11.2014

(51) International Patent Classification (IPC):
*B01J 20/10* *(2006.01)*

(52) Cooperative Patent Classification (CPC):
**A61K 33/24; A61K 9/2054; A61P 3/12; A61P 7/08; A61P 7/10; A61P 9/04; B01J 20/10; B01J 39/14**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: 08.11.2013   US 201361901886 P
10.12.2013   US 201361914354 P
22.01.2014   US 201461930328 P
22.01.2014   US 201461930336 P
30.05.2014   US 201462005484 P
20.06.2014   US 201462015215 P
22.09.2014   US 201462053732 P

(62) Document number(s) of the earlier application(s) in accordance with Art. 76 EPC:
**14860387.1 / 3 065 710**

(71) Applicant: **ZS Pharma, Inc**
**Coppell, TX 75019-7609 (US)**

(72) Inventors:
• **KEYSER, Donald Jeffrey**
**Southlake, 76092 (US)**
• **GUILLEM, Alvaro F**
**Lantana, 76226 (US)**

(74) Representative: **AstraZeneca Intellectual Property**
**Eastbrook House**
**Shaftesbury Road**
**Cambridge CB2 8BF (GB)**

Remarks:
This application was filed on 22.03.2024 as a divisional application to the application mentioned under INID code 62.

(54)    **MICROPOROUS ZIRCONIUM SILICATE FOR THE TREATMENT OF HYPERKALEMIA**

(57)    The present invention relates to novel microporous zirconium silicate compositions that are formulated to remove toxins, e.g. potassium ions, from the gastrointestinal tract at an elevated rate without causing undesirable side effects. The preferred composition has at least 95% ZS-9. These compositions are particularly useful in the therapeutic treatment of hyperkalemia. These compositions are also useful in the treatment of chronic kidney disease, coronary vascular disease, diabetes mellitus, and transplant rejection.

**Description**

## CROSS-REFERENCE TO RELATED APPLICATIONS

This application claims priority to U.S. Provisional Application Nos. 61/901,886, filed November 8, 2013, 61/914,354, filed December 10, 2013, 61/930,328 filed January 22, 2014, 61/930,336 filed January 22, 2014, 62/005,484 filed May 30, 2014, 62/015,215 filed June 20, 2014, and 62/053,732 filed September 22, 2014 the disclosures of each are hereby incorporated by reference in their entirety.

## BACKGROUND OF THE INVENTION

### Field of the Invention

The present invention relates to novel zirconium silicate ("ZS") compositions which are preferably sodium zirconium cyclosilicates having an elevated level of ZS-9 crystalline form relative to other forms of zirconium cyclosilicates (*i.e.,* ZS-7) and zirconium silicates (*i.e.,* ZS-8, ZS-11). The ZS compositions are preferably sodium zirconium cyclosilicate compositions where the crystalline form has at least 95% ZS-9 relative to other crystalline forms of zirconium silicate. The ZS compositions of the present invention unexpectedly exhibit a markedly improved *in vivo* potassium ion absorption profile and rapid reduction in elevate levels of serum potassium.

Preferably ZS compositions of the present invention are specifically formulated at particular dosages to remove select toxins, *e.g.*, potassium ions or ammonium ions, from the gastrointestinal tract at an elevated rate without causing undesirable side effects. The preferred formulations are designed to remove and avoid potential entry of particles into the bloodstream and potential increase in pH of urine in patients. The formulation is also designed to release less sodium into the blood. These compositions are particularly useful in the therapeutic treatment of hyperkalemia and kidney disease. The present invention also relates to pharmaceutical granules, tablets, pill, and dosage forms comprising the microporous ZS as an active ingredient. In particular, the granules, tablets, pills or dosage forms are compressed to provide immediate release, delayed release, or specific release within the subject. Also disclosed are microporous ZS compositions having enhanced purity and potassium exchange capacity ("KEC"). Methods of treating acute, sub-acute, and chronic hyperkalemia have also been investigated. Disclosed herein are particularly advantageous dosing regimens for treating different forms of hyperkalemia using the microporous ZS compositions noted above. In addition, the present invention relates to methods of co-administering microporous ZS compositions in combination with other pharmacologic drugs that are known to induce, cause, or exacerbate the hyperkalemic condition.

### Description of the Related Art

Acute hyperkalemia is a serious life threatening condition resulting from elevated serum potassium levels. Potassium is a ubiquitous ion, involved in numerous processes in the human body. It is the most abundant intracellular cation and is critically important for numerous physiological processes, including maintenance of cellular membrane potential, homeostasis of cell volume, and transmission of action potentials. Its main dietary sources are vegetables (tomatoes and potatoes), fruit (oranges, bananas) and meat. The normal potassium levels in plasma are between 3.5-5.0 mmol/L with the kidney being the main regulator of potassium levels. The renal elimination of potassium is passive (through the glomeruli) with active reabsorption in the proximal tubule and the ascending limb of the loop of Henle. There is active excretion of potassium in the distal tubules and the collecting duct, both of these processes are controlled by aldosterone.

Increased extracellular potassium levels result in depolarization of the membrane potential of cells. This depolarization opens some voltage-gated sodium channels, but not enough to generate an action potential. After a short period of time, the open sodium channels inactivate and become refractory, increasing the threshold to generate an action potential. This leads to impairment of the neuromuscular-, cardiac- and gastrointestinal organ systems, and this impairment is responsible for the symptoms seen with hyperkalemia. Of greatest concern is the effect on the cardiac system, where impairment of cardiac conduction can lead to fatal cardiac arrhythmias such as asystole or ventricular fibrillation. Because of the potential for fatal cardiac arrhythmias, hyperkalemia represents an acute metabolic emergency that must be immediately corrected.

Hyperkalemia may develop when there is excessive production of serum potassium (oral intake, tissue breakdown). Ineffective elimination, which is the most common cause of hyperkalemia, can be hormonal (as in aldosterone deficiency), pharmacologic (treatment with ACE-inhibitors or angiotensin-receptor blockers) or, more commonly, due to reduced kidney function or advanced cardiac failure. The most common cause of hyperkalemia is renal insufficiency, and there is a close correlation between degree of kidney failure and serum potassium ("S-K") levels. In addition, a number of different commonly used drugs cause hyperkalemia, such as, but not limited to, ACE-inhibitors, angiotensin receptor blockers, potassium-sparing diuretics (such as, but not limited to, amiloride), NSAIDs (such as, but not limited to, ibuprofen, naproxen, celecoxib), heparin and certain cytotoxic, immunosuppressants (such as, but not limited to, cyclosporin and tacrolimus) and/or antibiotic drugs (such as, but not limited to, trimethoprim). Finally, beta-receptor blocking agents, digoxin or succinylcholine are other well-known causes of hyperkalemia. In addition, advanced degrees of congestive heart disease, massive injuries, burns or intravascular hemolysis cause hyperkalemia, as can metabolic acidosis, most often as part of diabetic ketoacidosis.

Symptoms of hyperkalemia are somewhat non-specific and generally include malaise, palpitations and muscle weakness or signs of cardiac arrhythmias, such as palpitations, brady- tachycardia or dizziness/fainting. Often, however, the hyperkalemia is detected during routine screening blood tests for a medical disorder or after severe complications have developed, such as cardiac arrhythmias or sudden death. Diagnosis is obviously established by S-K measurements.

Treatment depends on the S-K levels. In milder cases (S-K between 5-6.5 mmol/l), acute treatment with a potassium binding resin (Kayexalate®), combined with dietary advice (low potassium diet) and possibly modification of drug treatment (if treated with drugs causing hyperkalemia) is the standard of care; if S-K is above 6.5 mmol/l or if arrhythmias are present, emergency lowering of potassium and close monitoring in a hospital setting is mandated. The following treatments are typically used:

- Kayexalate®, a resin that binds potassium in the intestine and hence increases fecal excretion, thereby reducing S-K levels. However, as Kayexalate® has been shown to cause intestinal obstruction and potential rupture. Further, diarrhea needs to be simultaneously induced with treatment. These factors have reduced the palatability of treatment with Kayexalate®.
- Insulin IV (+ glucose to prevent hypoglycemia), which shifts potassium into the cells and away from the blood.
- Calcium supplementation. Calcium does not lower S-K, but it decreases myocardial excitability and hence stabilizes the myocardium, reducing the risk for cardiac arrhythmias.
- Bicarbonate. The bicarbonate ion will stimulate an exchange of K+ for Na+, thus leading to stimulation of the sodium-potassium ATPase.
- Dialysis (in severe cases).

The only commercial pharmacologic modality that actually increases elimination of potassium from the body is Kayexalate®; however, due to the need to induce diarrhea, Kayexalate® cannot be administered on a chronic basis, and even in the acute setting, with the accompanying need to induce diarrhea, combined with only marginal efficacy and a foul smell and taste, reduces its usefulness.

The use of ZS or titanium silicate microporous ion exchangers to remove toxic cations and anions from blood or dialysate is described in U.S. Patent Nos. 6,579,460, 6,099,737, and 6,332,985, each of which is incorporated herein in their entirety. Additional examples of microporous ion exchangers are found in U.S. Patent Nos. 6,814,871, 5,891,417, and 5,888,472, each of which is incorporated herein in their entirety.

The inventors have found that known ZS compositions may exhibit undesirable effects when utilized *in vivo* for the removal of potassium in the treatment of hyperkalemia. Specifically, the administration of ZS molecular sieve compositions has been associated with an incidence of mixed leukocyte inflammation, minimal acute urinary bladder inflammation and the observation of unidentified crystals in the renal pelvis and urine in animal studies, as well as an increase in urine pH. Further, known ZS compositions have had issues with crystalline impurities and undesirably low cation exchange capacity.

The inventors disclosed novel ZS molecular sieves to address the problem associated with existing hyperkalemia treatments, and novel methods of treatment for hyperkalemia utilizing these novel compositions. *See* U.S. Patent Application No. 13/371,080 (U.S. Pat. Application Pub. No. 2012-0213847 A1). In addition, the present inventors have disclosed novel processes for producing ZS absorbers with an improved particles-size distribution that can be prepared with methods avoid and/or reduce the need to screen ZS crystals. *See* U.S. Patent Application 13/829,415 (U.S. Pat. Application Pub. No. 2013-0334122).

The inventors have discovered that delivery of ZS in the treatment of hyperkalemia can be improved by the use of novel dosage forms. Specifically, the inventors have found that specific dosages of the ZS, when administered to a subject suffering from elevated levels of potassium, are capable of significantly decreasing the serum potassium levels in patients with hyperkalemia to normal levels. The inventors have also found that these specific dosages are capable of sustaining the lower potassium levels in patients for an extended period of time.

The inventors have also discovered that administering and/or co-administering microporous ZS is also beneficial to those patients currently undergoing treatment with pharmacologic drugs that are known to cause hyperkalemia. For example, patients with kidney dysfunction, cardiovascular or heart disease, or organ transplantation receiving ACE or ARB inhibitors and/or immunosuppressants typically develop hyperkalemia. One possible solution to the development of hyperkalemia in these patients is to suspend treatment of the drug until potassium levels normalize. The inventors have discovered that the co-administration or administration of ZS to these patients will normalize or reduce excess potassium levels so as to allow the continued administration of the pharmacologic drug that is causing hyperkalemia.

The role of aldosterone in kidney function has been extensively studied. *See* Remuzzi et al., "The role of renin-angiotensin-aldosterone system in the progression of chronic kidney disease," Kidney Int'l, Vol. 68 Supp. 99, pp. S57-S65 (2005); Zhang et al., "Aldosterone induces epithelial-mesenchymal transition via ROS of mitochondrial origin," Am J Physiol Renal Physiol 293 (2007); Ponda et al., "Aldosterone Antagonism in Chronic Kidney Disease," Clin J Am Soc Nephol 1:668-677 (2006); U. Wenzel, "Aldosterone and Progression of Renal Disease," Current Opinion in Nephrology and Hypertension 17:44-50 (2008); Remuzzi et al., "The Aggravating Mechanisms of Aldosterone on Kidney Fibrosis," J Am Soc Nephrol 19:1459-1462 (2008); Navaneethan et al., "Aldosterone Antagonists for Preventing the Progression of Chronic Kidney Disease: A Systematic Review and Meta-analysis," Am Soc Neph (2008); Briet et al., "Aldosterone: effects on the kidney and cardiovascular system," Nature Reviews: Nephrology 6:261-273 (2010); R Toto, "Aldosterone blockade in chronic kidney disease: can it improve outcome?" Current Opinion in Nephrology and Hypertension 19:444-449 (2010); Turner et al., "Treatment of chronic kidney disease," Kidney Int'l 81:351-362 (2012). As noted by Turner et al., recognition of the deleterious effects of aldosterone has led to attempts to selectively block it using the mineralocorticoid receptor blockers. A large number of animal studies support this approach, and human studies have shown a reduction in proteinuria when aldosterone blockade was added to an ACE inhibitor or ARB. However, this approach has frequently led to hyperkalemia. Thus, there exists a need to treat CKD by lowering aldosterone levels in a way that leads to improved GFR without the onset of hyperkalemia.

The role of aldosterone in cardiovascular disease (CVD) has been extensively studied. Rocha et al., "Selective Aldosterone Blockade Prevents Angiotensin II/Salt-Induced Vascular Inflammation in the Rat Heart," Endocrinology 143(12):4828-4836 (2002); Rocha et al., "Aldosterone Induces a Vascular Inflammatory Phenotype in the Rat Heart," Am J Phsiol Heat Circ Physiol 283:H1802-H1810 (2002); Briet et al., "Aldosterone: effects on the kidney and cardiovascular system," Nature Reviews: Nephrology 6:261-273 (2010); Tomaschitz et al., "Plasma aldosterone levels are associated with increased cardiovascular mortality: the Ludwigshafen Risk and Cardiocascular Health (LURIC) study," European Heart Journal 31:1237-1247 (2010). Notably, CVD is well known to be common and often fatal in people with CKD. As discussed by Tomachitz et al., plasma aldosterone levels are associated with increased cardiovascular morality. Accordingly, reduction of aldosterone levels without side effects associated with aldo blockers would be desirably in the treatment of patients diagnosed with CKD and/or CVD.

Patients suffering from moderate to severe heart failure and/or renal failure are often administered a combination therapy of ACE inhibitors or ARB and a diuretic (e.g., potassium sparing). The administration of this combination has been shown to increase the risk of developing hyperkalemia, especially in patients with diabetes mellitus and renal impairment. Horn and Hansten, "Hyperkalemia Due to Drug Interactions," Pharmacy Times, pp. 66-67, January 2004; Desai "Hyperkalemia Associated with Inhibitors of the Renin-Angiotensin-Aldosterone System: Balancing Risk and Benefit," Circulation, 118:1609-1611 (2008) Therefore, there is a need to provide patients who are currently on this combination therapy with a means of lower the serum potassium levels without halting the treatment.

Patients who have undergone organ replacement or transplantation are typical prescribed immunosuppressants to help reduce the risk of organ rejection by the immune system. However, the use of immunosuppressants is known to increase the risk of developing hyperkalemia. Therefore, there is a need to provide patients who are currently undergoing immunosuppressant therapy with a means to reduce or lower serum potassium levels without halting the use of these drugs.

Hyperkalemia is also common in patients with diabetes mellitus who may or may not have renal impairment. Because there is a risk of developing hyperkalemia or the presence of hyperkalemia in diabetic patients, the use of renin-angiotensin-aldosterone system inhibitors, which is also associated with increasing the risk of hyperkalemia, is limited these patients. The inventors of the present invention have found that the administration of microporous ZS to diabetic patients will allow the continued administration or co-administration of renin-angiotensin-aldosterone system inhibitors useful for the treatment of diabetes mellitus.

## SUMMARY OF THE EMBODIMENTS OF THE INVENTION

Cation exchange compositions or products comprising ZS, when formulated and administered at a particular pharmaceutical dose, are capable of significantly reducing the serum potassium levels in patients exhibiting elevated potassium levels. In one embodiment, the patients exhibiting elevated potassium levels are patients with chronic or acute kidney diseases. In another embodiment, the patients exhibiting elevated potassium levels have acute or chronic hyperkalemia.

In one embodiment, the dosage of the composition may range from approximately 1-20 grams of ZS, preferably 8-15 grams, more preferably 10 grams. In another embodiment, the composition is administered at a total dosage range of approximately 1-60 gram, preferably 24-45 grams, more preferably 30 grams.

In another embodiment, the composition comprises molecular sieves having a microporous structure composed of $ZrO_3$ octahedral units and at least one $SiO_2$ tetrahedral units and $GeO_2$ tetrahedral units. These molecular sieves have the empirical formula:

$$A_pM_xZr_{1-x}Si_nGe_yO_m$$

where A is an exchangeable cation selected from potassium ion, sodium ion, rubidium ion, cesium ion, calcium ion, magnesium ion, hydronium ion or mixtures thereof, M is at least one framework metal selected from the group consisting of hafnium (4+), tin (4+), niobium (5+), titanium (4+), cerium (4+), germanium (4+), praseodymium (4+), and terbium (4+), "p" has a value from about 0 to about 20, "x" has a value from 0 to less than 1, "n" has a value from about 0 to about 12, "y" has a value from 0 to about 12, "m" has a value from about 3 to about 36 and $1 \leq n + y \leq 12$. The germanium can substitute for the silicon, zirconium or combinations thereof. Since the compositions are essentially insoluble in bodily fluids (at neutral or basic pH), they can be orally ingested in order to remove toxins in the gastrointestinal system.

In an alternative embodiment, the molecular sieve is provided which has an elevated cation exchange capacity, particularly potassium exchange capacity. The elevated cation exchange capacity is achieved by a specialized process and reactor configuration that lifts and more thoroughly suspends crystals throughout the reaction as described in U.S. Patent Application No. 13/371,080 (U.S. Pat. Application Pub. No. 2012-0213847 A1). In an embodiment of the invention, the improved ZS-9 crystal compositions (*i.e.,* compositions where the predominant crystalline form is ZS-9) had a potassium exchange capacity of greater than 2.5 meq/g, more preferably between 2.7 and 3.7 meq/g, more preferably between 3.05 and 3.35 meq/g. ZS-9 crystals with a potassium exchange capacity of 3.1 meq/g have been manufactured on a commercial scale and have achieved desirable clinical outcomes. It is expected that ZS-9 crystals with a potassium exchange capacity of 3.2 meq/g will also achieve desirable clinical outcomes and offer improved dosing forms. The targets of 3.1 and 3.2 meq/g may be achieved with a tolerance of $\pm$ 15%, more preferably $\pm$ 10%, and most preferably $\pm$ 5%. Higher capacity forms of ZS-9 are desirable although are more difficult to produce on a commercial scale. Such higher capacity forms of ZS-9 have elevated exchange capacities of greater than 3.5 meq/g, more preferably greater than 4.0 meq/g, more preferably between 4.3 and 4.8 meq/g, even more preferably between 4.4 and 4.7 meq/g, and most preferably approximately 4.5 meq/g. ZS-9 crystals having a potassium exchange capacity in the range of between 3.7 and 3.9 meq/g were produced in accordance with Example 14 below.

In one embodiment, the composition exhibits median particle size of greater than 3 microns and less than 7% of the particles in the composition have a diameter less than 3 microns. Preferably, less than 5% of the particles in the composition have a diameter less than 3 microns, more preferably less than 4% of the particles in the composition have a diameter less than 3 microns, more preferably less than 3% of the particles in the composition have a diameter of less than 3 microns, more preferably less than 2% of the particles in the composition have a diameter of less than 3 microns, more preferably less than 1 % of the particles in the composition have a diameter of less than 3 microns, more preferably less than 0.5% of the particles in the composition have a diameter of less than 3 microns. Most preferably, none of the particles or only trace amounts have a diameter of less than 3 microns.

The median and average particle size is preferably greater than 3 microns and particles reaching a sizes on the order of 1,000 microns are possible for certain applications. Preferably, the median particle size ranges from 5 to 1000 microns, more preferably 10 to 600 microns, more preferably from 15 to 200 microns, and most preferably from 20 to 100 microns.

In one embodiment, the composition exhibiting the median particle size and fraction of particles in the composition having a diameter less than 3 micron described above also exhibits a sodium content of below 12% by weight. Preferably, the sodium contents is below 9% by weight, more preferably the sodium content is below 6% by weight, more preferably the sodium content is below 3% by weight, more preferably the sodium content is in a range of between 0.05 to 3% by weight, and most preferably 0.01% or less by weight or as low as possible.

In one embodiment, the invention involves an individual pharmaceutical dosage comprising the composition in capsule, tablet, pill or powdered form. In another embodiment of the invention, the pharmaceutical product is packaged in a kit in individual unit dosages sufficient to maintain a lowered serum potassium level. The dosage may range from approximately 1-60 grams per day or any whole number or integer interval therein. Such dosages can be individual capsules, tablets, or packaged powdered form of 1.25-20 grams of the ZS, preferably 2.5-15 grams of ZS, more preferably 5-10 grams of ZS. In another embodiment, the ZS may be a single unit dose of approximately 1.25-45 gram capsule, tablet or powdered package. In another embodiment, the product may be consumed once a day, three times daily, every other day, or weekly.

The compositions of the present invention may be used in the treatment of kidney disease (*e.g*., chronic or acute) or symptoms of kidney diseases, such as hyperkalemia (*e.g*., chronic or acute) comprising administering the composition to a patient in need thereof. The administered dose may range from approximately 1.25-20 grams of ZS, preferably 2.5-15 grams, more preferably 10 grams. In another embodiment, the total administered dose of the composition may range from approximately 1-60 gram (14-900 mg/Kg/day), preferably 24-36 grams (350-520 mg/Kg/day), more preferably 30 grams (400 mg/Kg/day).

The present inventors have discovered that administration of preferred forms of microporous ZS is associated with an improved glomerular filtration rates (GFR) and when co administered with therapies that include diuretics desirably reduced the risk of developing hyperkalemia. These data demonstrate that chronic kidney disease (CKD) and/or cardiovascular disease (CVD) may be treated by administration of microporous zirconium silicate along with standard therapies that include diuretic according to the present invention.

In one embodiment, the present invention involves administration of a suitable dose of microporous zirconium silicate to a patient who has been diagnosed with CKD. In another embodiment, the present invention involves administration of a suitable dose of microporous zirconium silicate to a patient who has been diagnosed with CVD or after a myocardial infarction. In one aspect of this embodiment, the patient is diagnosed with both CKD and CVD.

In one embodiment, the invention involves administering to a CKD and/or CVD patient a combination comprising a therapy that includes diuretic and a zirconium silicate. In another embodiment, the zirconium silicate can be a ZS-9 as described herein. In yet another embodiment, the diuretic can be a loop diuretic, a thiazine diuretic and/or a potassium sparing diuretic. In still another embodiment, a method of treating a CKD and/or CVD comprises administering therapies that include diuretics and a zirconium silicate of the present invention. In another embodiment, the treatment of CKD and/or CVD using diuretics and zirconium silicate may further comprise angiotensin converting enzyme inhibitors (ACE) or angiotensin receptor blockers (ARB).

In another embodiment, the invention involves administering to a transplant patient or a patient who recently received organ replacement/transplant a combination comprising an immunosuppressant therapy and a microporous ZS. In another embodiment, the ZS is ZS-9 as described herein. In yet another embodiment, the immunosuppressant may include any currently known immunosuppressant drug used on patients who have undergone transplantation or organ replacement. These immunosuppressants may include induction drugs or maintenance drugs.

In yet another embodiment, the invention involves administering to diabetes patients, in a more preferred embodiment diabetes mellitus patients, a combination comprising renin-angiotensin-aldosterone system inhibitors and a microporous ZS. In yet another embodiment, the renin-angiotensin-aldosterone system inhibitors may be ACE or ARB inhibitors. In another embodiment, the ZS is a ZS-9 as described herein.

## BRIEF DESCRIPTION OF THE DRAWINGS

Fig. 1 is a polyhedral drawing showing the structure of microporous ZS $Na_{2.19}ZrSi_{3.01}O_{9.11} \cdot 2.71H_2O$ (MW 420.71)

Fig. 2 shows particle size distribution of ZS-9 lot 5332-04310-A in accordance with Example 8.

Fig. 3 shows particle size distribution of ZS-9 lot 5332-15410-A in accordance with Example 8.

Fig. 4 shows particle size distribution of ZS-9 preclinical lot in accordance with Example 8.

Fig. 5 shows particle size distribution of lot 5332-04310A w/o screening in accordance with Example 9.

Fig. 6 shows particle size distribution of lot 5332-04310A 635 mesh in accordance with Example 9.

Fig. 7 shows particle size distribution of lot 5332-04310A 450 mesh in accordance with Example 9.

Fig. 8 shows particle size distribution of lot 5332-04310A 325 mesh in accordance with Example 9.

Fig. 9 shows particle size distribution of lot 5332-04310A 230 mesh in accordance with Example 9.

Fig. 10: XRD plot for ZS-9 prepared in accordance with Example 12.

Fig. 11: FTIR plot for ZS-9 prepared in accordance with Example 12.

Fig. 12: XRD plot for ZS-9 prepared in accordance with Example 14.

Fig. 13: FTIR plot for ZS-9 prepared in accordance with Example 14.

Fig. 14: Example of the Blank Solution Chromatogram

Fig. 15: Example of the Assay Standard Solution Chromatogram.

Fig. 16: Exemplary Sample Chromatogram.

Fig. 17: Reaction vessel with standard agitator arrangement.

Fig. 18: Reaction vessel with baffles for production of enhanced ZS-9

Fig. 19: Detail of baffle design for 200-L reaction vessel for production of enhanced ZS-9

Fig. 20: Treatment Period of ZS-9 in comparison to placebo over 48 hours after ingestion.

Fig. 21: Comparison of time of serum potassium decrease.

Fig. 22: Comparison of serum potassium increase following treatment.

Fig. 23: Rate of potassium excretion in urine.

Fig. 24: Daily urinary sodium excretion.

Fig. 25: XRD plot for H-ZS-9 prepared according to Example 20 batch 5602-26812.

Fig. 26: XRD plot for H-ZS-9 prepared according to Example 20 batch 5602-28312.

Fig. 27: XRD plot for H-ZS-9 prepared according to Example 20 batch 5602-29112.

Fig. 28: XRD plot for H-ZS-9 prepared according to Example 20 batch 5602-29812.

Fig. 29: XRD data for ZS crystals produced accoridng to Example 20.

Fig. 30: XRD data showing ZS-8 impurities.

Fig. 31: XRD for ZS having 95% or more ZS-9.

Fig. 32: Particle size distribution for ZS having 95% or more ZS-9.

Fig. 33: Correlation between serum potassium drops and ZS-9%.

Fig. 34: Schematic chemical structure of ZS-9 pore opening.

Fig. 35: Decrease in serum potassium upon administration of ZS-9.

Fig. 36: Statistical significance of Acute Phase.

Fig. 37: Statistical significance of Subacute Phase.

Fig. 38: Graph of dose dependent reduction of K+ over 48 hours on 2.5, 5, and 10 grams of ZS-9 TID.

Fig. 39: Serum potassium levels (mmol/L) measured over 48 hours using ZS-9 vs. placebo .

Fig. 40: Graph measuring the change of potassium serum levels using ZS-9 on patient taking RAASi.

Fig. 41: Serum potassum levels (mmol/l) measured over 48 hrs using ZS-9 vs. placebo.

Fig. 42: Mean change from baseline of serum bicarbinate levels using ZS-9 vs. placebo.

Fig. 43: Mean urniary pH change using ZS-9 vs. placebo.

Fig. 44: Measure of serum potassium (mmol/L) over 21 days of patients on 5 g ZS-9 vs placebo.

Fig. 45: Measure of serum potassium (mmol/L) over 21 days of patients on 10 g ZS-9 vs placebo.

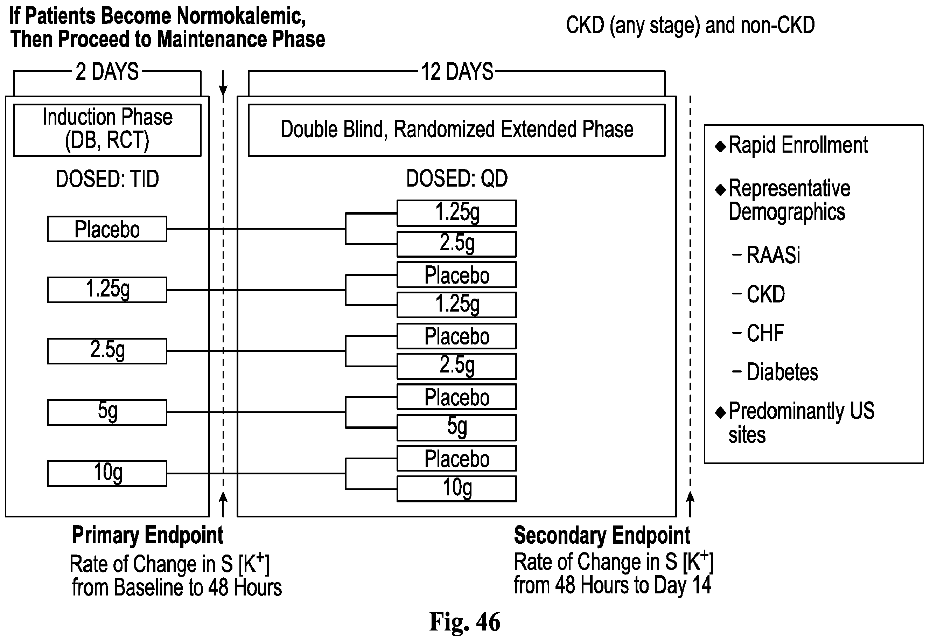

Fig. 46: Schematic of phase 3 study.

Fig. 47: Comparison of ZS-9 dose dependent reduction of potassium over a period of 48 hours in diabetes mellitus patients and overall population.

Fig. 48: Comparison of (a) placebo (b) 5 g , and (c) 10 g adminstration of ZS-9 during acute phase in diabetes mellitus patients, wherein n=96 for placebo, n=96 for 5g ZS-9, and n=81 for 10g ZS-9.

Fig. 49: Comparison of 5 grams and 10 grams of ZS-9 in the reduction in mean potassium at 48 hours in diabetes mellitus vs. overall population.

Fig. 50: Comparison of adverse events in diabetes mellitus populations receiving ZS-9.

Fig. 51. Comp:rison of single QD dosing of ZS-9 (5g and 10g) on normokalemia in extended phase of diabetes mellitus population vs. overall population.

Fig. 52: Comparison of single QD dosing of ZS-9 (10g) to maintain normkalemia in diabetes mellitus populations vs. overall population.

Fig. 53: Mean potassium change in extended phase for 10g of ZS-9 on maintaining potassium levels in comparison to placebo.

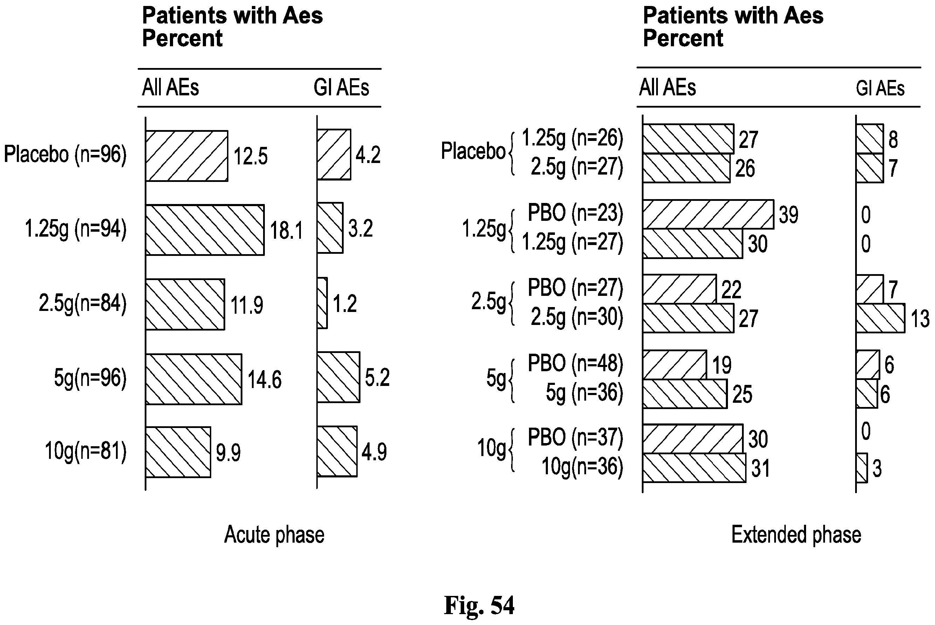

Fig. 54: Rate of adverse events in diabete mellitus population using single QD dosing.

Fig. 55: Mean serum potassium 5g, 10g, and 15g v. placebo.

Fig. 56: 10 g Acute dose 0hr v. 1hr.

Fig. 57: 10 g Acute dose 0hr v. 2hr.

Fig. 58: 10 g Acute dose 0hr v. 4hr.

Fig. 59: 10 g Acute dose 0hr v. 24hr.

Fig. 60: Acute phase proportion of subjects normalkalemia.

Fig. 61: Acute phase time to normalization.

Fig. 62: ZS-9 QD maintains normalkalemia at 5, 10, 15 g doses.

Fig. 63: ZS-9 QD maintains normalkalemia at 10 and 15 g doses in pateitns having 6.0 meq/L or more potassium.

Fig. 64: BUN decline at MP day 15, no change at MP day 29.

Fig. 65: No change in BUN at end of study.

Fig. 66: Significant increase in bicarbonate.

Fig. 67: No difference in GFR.

Fig. 68: Significant decrease in aldosterone.

Fig. 69: Change from maintenance phase renin

Fig. 70: Change from maintenance phase galectine-3.

Fig. 71: Change from maintenance phase BNP.

Fig. 72: Change from maintenance phase insulin.

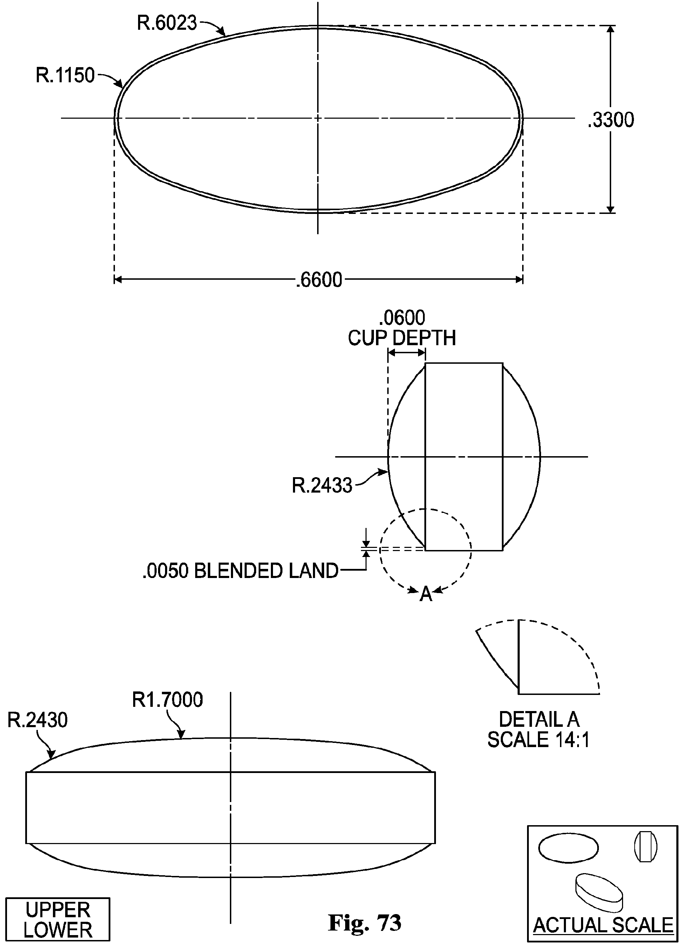

Fig. 73: Schematic of a 500 mg ZS tablet.

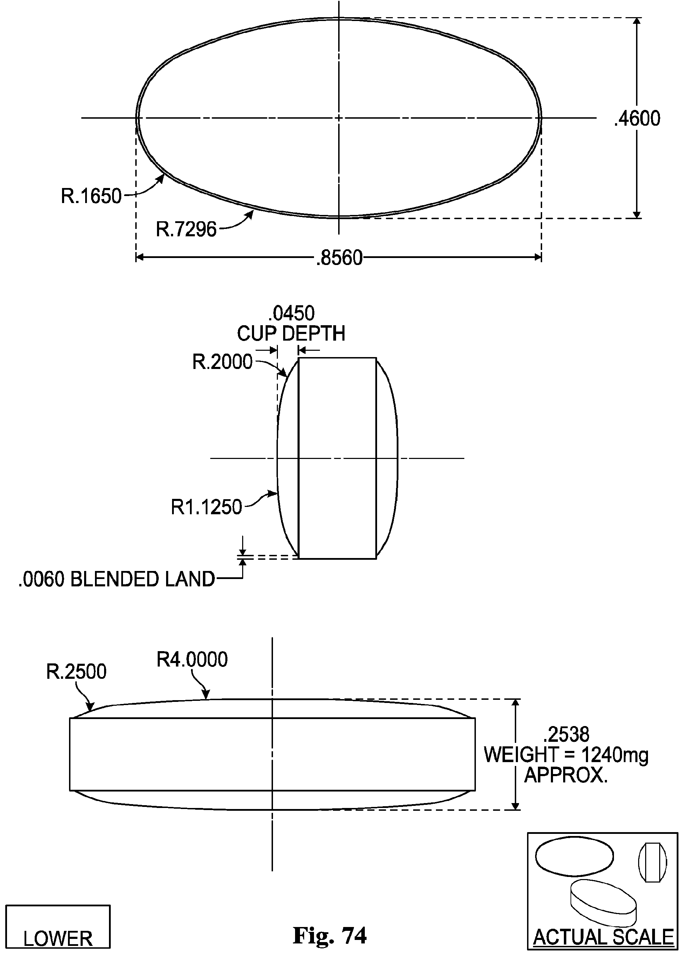

Fig. 74. Schematic of a 1000 mg ZS tablet.

## DETAILED DESCRIPTION OF THE EMBODIMENTS OF THE INVENTION

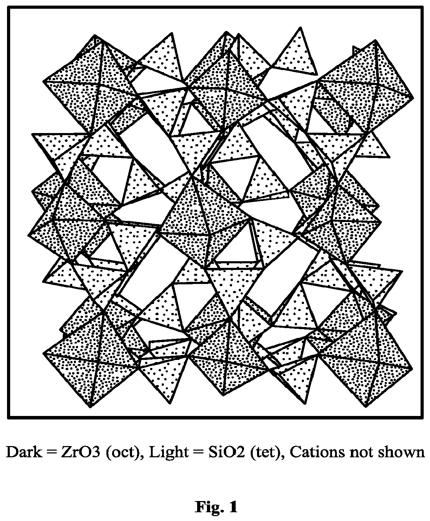

The inventors have discovered novel ZS molecular sieve absorbers that address problems of adverse effects in the therapeutic use of molecular sieve absorbers, *e.g.*, for the treatment of hyperkalemia. ZS has a microporous framework structure composed of $ZrO_2$ octahedral units and $SiO_2$ tetrahedral units. Figure 1 is a polyhedral drawing showing the structure of microporous ZS Na2.19ZrSi3.01O9.11 .• 2.71H2O (MW 420.71) The dark polygons depict the octahedral zirconium oxide units while the light polygons depict the tetrahedral silicon dioxide units. Cations are not depicted in Fig. 1.

The microporous exchanger of the invention has a large capacity and strong affinity, *i.e.,* selectivity, for potassium or ammonium. Eleven types of ZS are available, ZS-1 through ZS-11, each having various affinities to ions have been developed. *See e.g.,* U.S. Patent No. 5,891,417. UZSi-9 (otherwise known as ZS-9) is a particularly effective ZS absorber for absorbing potassium and ammonium. These ZS have the empirical formula:

$$A_pM_xZr_{1-x}Si_nGe_yO_m \qquad (I)$$

where A is an exchangeable cation selected from potassium ion, sodium ion, rubidium ion, cesium ion, calcium ion, magnesium ion, hydronium ion or mixtures thereof, M is at least one framework metal selected from the group consisting of hafnium (4+), tin (4+), niobium (5+), titanium (4+), cerium (4+), germanium (4+), praseodymium (4+), and terbium (4+), "p" has a value from about 0 to about 20, "x" has a value from 0 to less than 1, "n" has a value from about 0 to about 12, "y" has a value from 0 to about 12, "m" has a value from about 3 to about 36 and $1 \leq n + y \leq 12$. The germanium can substitute for the silicon, zirconium or combinations thereof. It is preferred that x and y are zero or both approaching zero, as germanium and other metals are often present in trace quantities. Since the compositions are essentially insoluble in bodily fluids (at neutral or basic pH), they can be orally ingested in order to remove toxins in the gastrointestinal system. The inventors of the present invention have noted that ZS-8 has an increased solubility as compared to other forms of ZS (i.e., ZS-1-ZS-7, and ZSi-9-ZS-11). The presence of soluble forms of ZS including ZS-8 is undesirable since soluble forms of ZS may contribute to elevated levels of zirconium and/or silicates in the urine. Amorphous forms of ZS may also be substantially soluble. Therefore, it is desirable to reduce the proportion of amorphous material to the extent practicable.

The zirconium metallates are prepared by a hydrothermal crystallization of a reaction mixture prepared by combining a reactive source of zirconium, silicon and/or germanium, optionally one or more M metal, at least one alkali metal and water. The alkali metal acts as a templating agent. Any zirconium compound, which can be hydrolyzed to zirconium oxide or zirconium hydroxide, can be used. Specific examples of these compounds include zirconium alkoxide, *e.g.*, zirconium n-propoxide, zirconium hydroxide, zirconium acetate, zirconium oxychloride, zirconium chloride, zirconium phosphate and zirconium oxynitrate. The sources of silica include colloidal silica, fumed silica and sodium silicate. The sources of germanium include germanium oxide, germanium alkoxides and germanium tetrachloride. Alkali sources include potassium hydroxide, sodium hydroxide, rubidium hydroxide, cesium hydroxide, sodium carbonate, potassium carbonate, rubidium carbonate, cesium carbonate, sodium halide, potassium halide, rubidium halide, cesium halide, sodium ethylenediamine tetraacetic acid (EDTA), potassium EDTA, rubidium EDTA, and cesium EDTA. The M metals sources include the M metal oxides, alkoxides, halide salts, acetate salts, nitrate salts and sulfate salts. Specific examples of the M metal sources include, but are not limited to titanium alkoxides, titanium tetrachloride, titanium trichloride, titanium dioxide, tin tetrachloride, tin isopropoxide, niobium isopropoxide, hydrous niobium oxide, hafnium isopropoxide, hafnium chloride, hafnium oxychloride, cerium chloride, cerium oxide and cerium sulfate.

Generally, the hydrothermal process used to prepare the zirconium metallate or titanium metallate ion exchange compositions of this invention involves forming a reaction mixture which in terms of molar ratios of the oxides is expressed by the formulae:

$$aA_2O:bMO_{q/2}:1\text{-}bZrO_2:cSiO_2:dGeO_2:eH_2O$$

where "a" has a value from about 0.25 to about 40, "b" has a value from about 0 to about 1, "q" is the valence of M, "c" has a value from about 0.5 to about 30, "d" has a value from about 0 to about 30 and "e" has a value of 10 to about 3000. The reaction mixture is prepared by mixing the desired sources of zirconium, silicon and optionally germanium, alkali metal and optional M metal in any order to give the desired mixture. It is also necessary that the mixture have a basic pH and preferably a pH of at least 8. The basicity of the mixture is controlled by adding excess alkali hydroxide and/or basic compounds of the other constituents of the mixture. Having formed the reaction mixture, it is next reacted at a temperature of about 100°C to about 250°C for a period of about 1 to about 30 days in a sealed reaction vessel under autogenous pressure. After the allotted time, the mixture is filtered to isolate the solid product which is washed with deionized water, acid or dilute acid and dried. Numerous drying techniques can be utilized including vacuum drying, tray drying, fluidized bed drying. For example, the filtered material may be oven dried in air under vacuum.

To allow for ready reference, the different structure types of the ZS molecular sieves and zirconium germanate molecular sieves have been given arbitrary designations of ZS-1 where the "1" represents a framework of structure type "1". That is, one or more ZS and/or zirconium germanate molecular sieves with different empirical formulas can have the same structure type.

The X-ray patterns presented in the following examples were obtained using standard X-ray powder diffraction techniques and reported in U.S. Patent No. 5,891,417. The radiation source was a high-intensity X-ray tube operated at 45 Kv and 35 ma. The diffraction pattern from the copper K-alpha radiation was obtained by appropriate computer based techniques. Flat compressed powder samples were continuously scanned at 2° (2θ) per minute. Interplanar spacings (d) in Angstrom units were obtained from the position of the diffraction peaks expressed as 2 θ where θ is the Bragg angle as observed from digitized data. Intensities were determined from the integrated area of diffraction peaks after subtracting background, "$I_o$" being the intensity of the strongest line or peak, and "I" being the intensity of each of the other peaks.

As will be understood by those skilled in the art, the determination of the parameter 20 is subject to both human and mechanical error, which in combination can impose an uncertainty of about ±0.4 on each reported value of 2θ. This uncertainty is, of course, also manifested in the reported values of the d-spacings, which are calculated from the θ values. This imprecision is general throughout the art and is not sufficient to preclude the differentiation of the present crystalline materials from each other and from the compositions of the prior art. In some of the X-ray patterns reported, the relative intensities of the d-spacings are indicated by the notations vs, s, m and w which represent very strong, strong, medium, and weak, respectively. In terms of 100xI/ $I_o$, the above designations are defined as w=0-15; m=15-60; s=60-80 and vs=80-100.

In certain instances the purity of a synthesized product may be assessed with reference to its X-ray powder diffraction pattern. Thus, for example, if a sample is stated to be pure, it is intended only that the X-ray pattern of the sample is free of lines attributable to crystalline impurities, not that there are no amorphous materials present.

The crystalline compositions of the instant invention may be characterized by their X-ray powder diffraction patterns and such may have one of the X-ray patterns containing the d-spacings and intensities set forth in the following Tables. The x-ray pattern for ZS-1, ZS-2, ZS-6, ZS-7, ZS-8, and ZS-11 as reported in U.S. Patent No. 5,891,417, is as follows:

| Table 1 - ZS X-Ray powder diffraction patterns | |
| --- | --- |
| ZS-1 | |
| d(Å) | I |
| 7.7-8.6 | m |
| 6.3-7.0 | m |
| 5.5-6.3 | s |
| 4.7-5.5 | m |
| 3.2-4.0 | m |
| 2.6-3.4 | vs |
| ZS-2 | |
| d(Å) | I |
| 5.8-6.6 | m |
| 4.2-5.0 | w |
| 3.9-4.6 | m |
| 2.9-3.7 | m |
| 2.5-3.3 | vs |
| 2.3-3.0 | s |
| ZS-6 | |
| d(Å) | I |
| 6.1-6.9 | m |
| 4.4-5.1 | m |
| 3.4-4.2 | m |
| 3.3-4.1 | m |
| 2.3-3.1 | vs |

(continued)

| ZS-6 | |
|---|---|
| 2.2-3.0 | w |
| **ZS-7** | |
| d(Å) | I |
| 6.8-7.6 | vs |
| 5.6-6.4 | m |
| 3.7-4.5 | m |
| 3.6-4.4 | m |
| 2.6-3.4 | s-vs |
| 2.5-3.3 | m |
| 2.4-3.2 | vs |

| ZS-8 | |
|---|---|
| d(Å) | I |
| 12.0-13.2 | vs |
| 3.9-4.7 | m |
| 2.8-3.6 | m |
| 2.3-3.1 | m |
| 2.2-3.0 | w |
| 2.1-2.9 | w |
| **ZS-11** | |
| d(Å) | I |
| 6.0-6.8 | w-m |
| 5.5-6.3 | m |
| 5.4-6.2 | vs |
| 5.2-6.0 | m |
| 2.7-3.5 | s |
| 2.5-3.3 | m |

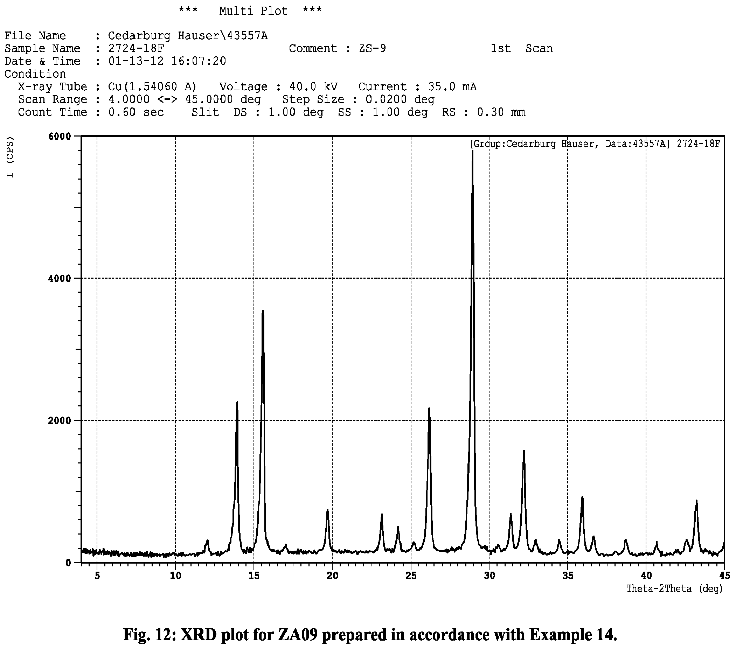

The x-ray diffraction pattern for the high-purity, high KEC ZS-9 as made in accordance with Example 14 herein (XRD shown in Fig. 12), had the following characteristics d-spacing ranges and intensities:

| Table 2 - ZS-9 | |
|---|---|
| d(Å) | I |
| 5.9-6.7 | m |
| 5.3-6.1 | m-s |
| 2.7-3.5 | vs |
| 2.0-2.8 | w-m |
| 1.6-2.4 | w |

The formation of ZS involves the reaction of sodium silicate and zirconium acetate in the presence of sodium hydroxide and water. The reaction has typically been conducted in small reaction vessels on the order of 1-5 Gallons. The smaller reaction vessels have been used to produce various crystalline forms of ZS including ZS-9. The inventors recognized that the ZS-9 being produced in these smaller reactors had an inadequate or undesirably low cation exchange capacity ("CEC").

The inventors have discovered that the use and proper positioning of a baffle-like structure in relation to the agitator within the crystallization vessel produces a ZS-9 crystal product exhibiting crystalline purity (as shown by XRD and FTIR spectra) and an unexpectedly high potassium exchange capacity. In smaller scale reactors (5-gal), cooling coils were positioned within the reactor to provide a baffle-like structure. The cooling coils were not used for heat exchange. Several types of cooling coils are available and the different designs may have some effect on the results presented herein, but the inventors used serpentine-type coils which snake along the inside wall of the reactor vessel.

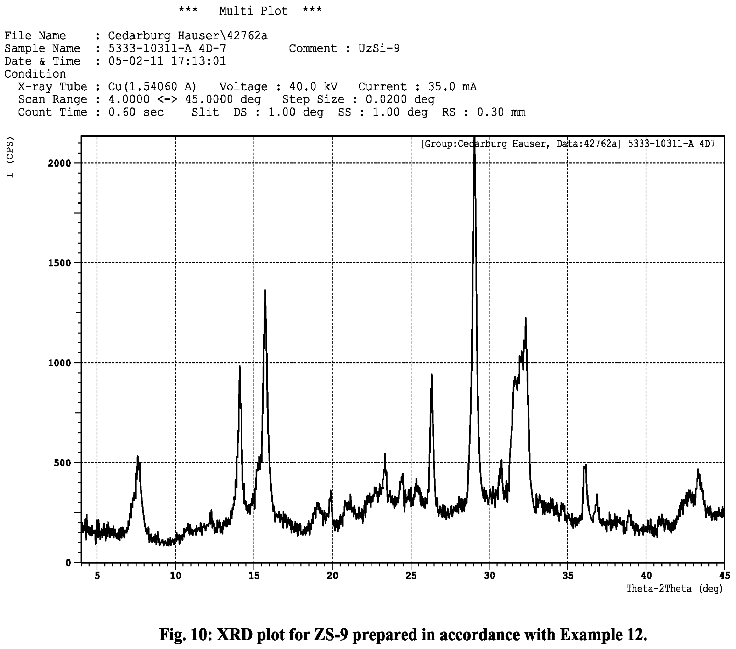

The inventors found that the crystallization reaction used to produce ZS-9 particularly benefitted from baffles that when they are properly positioned relative to the agitator. The inventors initially produced ZS-9 with significant levels of undesirable ZS-11 impurity. See Figs. 10-11. This incomplete reaction is believed to have resulted from significant amounts of solids remaining near the bottom of the reaction vessel. These solids near the bottom of the vessel remain even with conventional agitation. When properly positioned, the baffles and agitator improved the reaction conditions by creating forces within the reactor that lift the crystals within the vessel allowing for the necessary heat transfer and agitation to make a high purity form of ZS-9. In one embodiment, the baffles in combination with the agitator may be configured such that it provides sufficient lift throughout the entire volume regardless of the size of the reactor used. For example, if the reactor size is enlarged (e.g., 200 liter reactor) and the reaction volume is increased, the baffles will also be resized to accommodate the new reactor volume. Figs. 12-13 show XRD and FTIR spectra of high purity ZS-9 crystals. As shown in Table 3 below, these crystals exhibit significantly higher levels of potassium exchange capacity ("KEC") than the less pure ZS-9 compositions. In an embodiment of the invention, the ZS-9 crystals had a potassium exchange capacity of between 2.7 and 3.7 meq/g, more preferably between 3.05 and 3.35 meq/g. ZS-9 crystals with a potassium exchange capacity of 3.1 meq/g have been manufactured on a commercial scale and have achieved desirable clinical outcomes. It is expected that ZS-9 crystals with a potassium exchange capacity of 3.2 meq/g will also achieve desirable clinical outcomes and offer improved dosing forms. The targets of 3.1 and 3.2 meq/g may be achieved with a tolerance of $\pm$ 15%, more preferably $\pm$ 10%, and most preferably $\pm$ 5%. Higher capacity forms of ZS-9 are desirable although are more difficult to produce on a commercial scale. Such higher capacity forms of ZS-9 have elevated exchange capacities of greater than 3.5 meq/g, preferably greater than 4.0 meq/g, more preferably between 4.3 and 4.8 meq/g, even more preferably between 4.4 and 4.7 meq/g, and most preferably approximately 4.5 meq/g. ZS-9 crystals having a potassium exchange capacity in the range of between 3.7 and 3.9 meq/g were produced in accordance with Example 14 below.

Another unexpected benefit that came from using the reactor having a standard agitator in combination with baffles is that the high crystalline purity, high potassium exchange capacity ZS-9 crystals could be produced without utilizing any seed crystals. Prior attempts at making homogenous crystals having high crystalline purity of a single crystalline form have utilized seed crystals. The ability to eliminate the use of seed crystals was therefore an unexpected improvement relative to prior art processes.

As stated the microporous compositions of this invention have a framework structure of octahedral $ZrO_3$ units, at least one of tetrahedral $SiO_2$ units and tetrahedral $GeO_2$ units, and optionally octahedral $MO_3$ units. This framework results in a microporous structure having an intracrystalline pore system with uniform pore diameters, *i.e.*, the pore sizes are crystallographically regular. The diameter of the pores can vary considerably from about 3 angstroms and larger.

As synthesized, the microporous compositions of this invention will contain some of the alkali metal templating agent in the pores. These metals are described as exchangeable cations, meaning that they can be exchanged with other (secondary) A' cations. Generally, the A exchangeable cations can be exchanged with A' cations selected from other alkali metal cations ($K^+$, $Na^+$, $Rb^+$, $Cs^+$), alkaline earth cations ($Mg^{2+}$, $Ca^{2+}$, $Sr^{2+}$, $Ba^{2+}$), hydronium ion or mixtures thereof. It is understood that the A' cation is different from the A cation. The methods used to exchange one cation for another are well known in the art and involve contacting the microporous compositions with a solution containing the desired cation (usually at molar excess) at exchange conditions. Typically, exchange conditions include a temperature of about 25°C to about 100° C and a time of about 20 minutes to about 2 hours. The use of water to exchange ions to replace sodium ions with hydronium ions may require more time, on the order of eight to ten hours. The particular cation (or mixture thereof) which is present in the final product will depend on the particular use and the specific composition being used. One particular composition is an ion exchanger where the A' cation is a mixture of $Na^+$, $Ca^{+2}$ and $H^+$ ions.

When ZS-9 is formed according to these processes, it can be recovered in the Na-ZS-9 form. The sodium content of Na-ZS-9 is approximately 12 to 13% by weight when the manufacturing process is carried out at pH greater than 9. The Na-ZS-9 is unstable in concentrations of hydrochloric acid (HCl) exceeding 0.2 M at room temperature, and will undergo structural collapse after overnight exposure. While ZS-9 is slightly stable in 0.2 M HCl at room temperature, at 37°C the material rapidly loses crystallinity. At room temperature, Na-ZS-9 is stable in solutions of 0.1M HCl and/or a pH of between approximately 6 to 7. Under these conditions, the Na level is decreased from 13% to 2% upon overnight treatment.

The conversion of Na-ZS-9 to H-ZS-9 may be accomplished through a combination of water washing and ion exchange processes, *i.e.,* ion exchange using a dilute strong acid, *e.g.,* 0.1 M HCl or by washing with water. Washing with water will decrease the pH and protonate a significant fraction of the ZS, thereby lowering the weight fraction of Na in the ZS. It may be desirable to perform an initial ion exchange in strong acid using higher concentrations, so long as the protonation of the ZS will effectively keep the pH from dropping to levels at which the ZS decomposes. Additional ion exchange may be accomplished with washing in water or dilute acids to further reduce the level of sodium in the ZS. The ZS made in accordance with the present invention exhibits a sodium content of below 12% by weight. Preferably, the sodium contents is below 9% by weight, more preferably the sodium content is below 6% by weight, more preferably the sodium content is below 3% by weight, more preferably the sodium content is in a range of between 0.05 to 3% by weight, and most preferably 0.01% or less by weight or as low as possible. When protonated (*i.e.*, low sodium) ZS is prepared in accordance with these techniques, the potassium exchange capacity is lowered relative to the un-protonated crystals. The ZS prepared in this way has a potassium exchange capacity of greater than 2.8. In a preferred aspect, the potassium exchange capacity is within the range of 2.8 to 3.5 meq/g, more preferably within the range of 3.05 and 3.35 meq/g, and most preferably about 3.2 meq/g. A potassium exchange capacity target of about 3.2 meq/g includes minor fluctuations in measured potassium exchange capacity that is expected between different batches of ZS crystals.

It has been found that when ZS crystals produced under optimal crystalline conditions are protonated, the protonation can result in a loss in cation exchange capacity. The inventors have discovered during scale up of the manufacturing process for ZS-9 that where crystallization conditions are less than optimal, the protonation of the produced ZS crystals results in an increased cation exchange capacity relative to the unprotonated form. The suboptimal crystallization conditions result for challenges of maintaining thorough agitation in a larger reaction vessel. For example, when increasing the size of the reaction vessel from a 50 gallons to 125 gallons, ZS-9 crystals with a crystalline impurities were produced. However, assessment of the KEC values for the protonated H-ZS-9 crystals utilizing this new method provided for greater than expected KEC's of greater than 3.1 meq/g, more preferably in the range of 3.2 to 3.5 meq/g.

The ion exchanger in the sodium form, *e.g.*, Na-ZS-9, is effective at removing excess potassium ions from a patient's gastrointestinal tract in the treatment of hyperkalemia. When the sodium form is administered to a patient, hydronium ions replace sodium ions on the exchanger leading to an unwanted rise in pH in the patient's stomach and gastrointestinal tract. Through *in vitro* tests it takes approximately twenty minutes in acid to stabilize sodium ion exchanger.

The hydronium form typically has equivalent efficacy as the sodium form for removing potassium ions *in vivo* while avoiding some of the disadvantages of the sodium form related to pH changes in the patient's body. For example, the hydrogenated form has the advantage of avoiding excessive release of sodium in the body upon administration. This can mitigate edema resulting from excessive sodium levels, particularly when used to treat acute conditions. Further, patient who are administered the hydronium form to treat chronic conditions will benefit from the lower sodium levels, particularly patients at risk for congestive heart failure. Further, it is believed that the hydronium form will have the effect of avoiding an undesirable increase of pH in the patient's urine.

The present inventors have found that ZS compositions lacking added calcium can serve to withdraw excess calcium from patients which makes these compositions useful in the treatment of hyperkalemia in hypercalcemic patents as well as for the treatment of hypercalcemia. The calcium content of compositions prepared according to the process described in U.S. Provisional Application 61/670,415, incorporated by reference in its entirety, is typically very low-*i.e.*, below 1 ppm. The present inventors have found that treatment of hyperkalemia with these compositions is also associated with removal of significant quantities of calcium from the patient's body. Therefore, these compositions are particularly useful for the treatment of hypercalcemic patients or hypercalcemic patients suffering from hyperkalemic.

The compositions of the present invention may be prepared by pre-loading the above-described ZS compositions with calcium ions. The pre-loading of the compositions with calcium results in a composition that will not absorb calcium when administered to patients. As an alternative, the ZS compositions may also be pre-loaded with magnesium.

The pre-loading of ZS with calcium (and/or magnesium) is accomplished by contacting the ZS with a dilute solution of either calcium or magnesium ions, preferably having a calcium or magnesium concentration range of about 10-100 ppm. The pre-loading step can be accomplished simultaneously with the step of exchanging hydronium ions with sodium ions as discussed above. Alternatively, the pre-loading step can be accomplished by contacting ZS crystals at any stage of their manufacture with a calcium or magnesium containing solution. Preferably, the ZS compositions comprise calcium or magnesium levels ranging from 1 to 100 ppm, preferably from 1 to 30 ppm, and more preferably between 5 and 25 ppm.

The pre-loading of ZS does not result in a reduction in potassium absorption capacity and therefore does not detract from the use of these compositions in the treatment of hyperkalemia. It is believed that due to their size, calcium and/or magnesium ions do not fully penetrate the pores of the ZS. Rather, the loaded calcium or magnesium remains only on the surface of the ZS. This added calcium or magnesium results in a composition that does not absorb calcium or magnesium from the patient's body and therefore is preferred for clinical use in the treatment of hyperkalemia.

In another embodiment, protonated ZS may be linked to hydroxyl-loaded anion exchanger such as zirconium oxide (OH-ZO), which help in the removal of sodium, potassium, ammonium, hydrogen and phosphate. Without being bound to a theory, the hydrogen released from the protonated ZS and hydroxide released from OH-ZO combine to form water, thus diminishing the concentration of "counter-ions" which diminish binding of other ions. The binding capacity of the cation and anion exchangers should be increased by administering them together. ZS of this form are useful for the treatment of many different types of diseases. In one embodiment, the compositions are used to remove sodium, potassium, ammonium, hydrogen and phosphate from the gut and from the patient with kidney failure.

The ZS-9 crystals have a broad particle size distribution. It has been theorized that small particles, less than 3 microns in diameter, could potentially be absorbed into a patient's bloodstream resulting in undesirable effects such as the accumulation of particles in the urinary tract of the patient, and particularly in the patent's kidneys. The commercially available ZS are manufactured in a way that some of the particles below 1 micron are filtered out. However, it has been found that small particles are retained in the filter cake and that elimination of particles having a diameter less than 3 microns requires the use of additional screening techniques.

The inventors have found that screening can be used to remove particles having a diameter below 3 microns and that removal of such particles is beneficial for therapeutic products containing the ZS compositions of the invention. Many techniques for particle screening can be used to accomplish the objectives of the invention, including hand screening, air jet screening, sifting or filtering, floating or any other known means of particle classification. ZS compositions that have been subject to screening techniques exhibit a desired particle size distribution that avoids potential complications involving the therapeutic use of ZS. In general, the size distribution of particles is not critical, so long as excessively small particles are removed. The ZS compositions of the invention exhibit a median particle size greater than 3 microns, and less than 7% of the particles in the composition have a diameter less than 3 microns. Preferably, less than 5% of the particles in the composition have a diameter less than 3 microns, more preferably less than 4% of the particles in the composition have a diameter less than 3 microns, more preferably less than 3% of the particles in the composition have a diameter of less than 3 microns, more preferably less than 2% of the particles in the composition have a diameter of less than 3 microns, more preferably less than 1 % of the particles in the composition have a diameter of less than 3 microns, more preferably less than 0.5% of the particles in the composition have a diameter of less than 3 microns. Most preferably, none of the particles or only trace amounts have a diameter of less than 3 microns. The median particle size is preferably greater than 3 microns and particles reaching a sizes on the order of 1,000 microns are possible for certain applications. Preferably, the median particle size ranges from 5 to 1000 microns, more preferably 10 to 600 microns, more preferably from 15 to 200 microns, and most preferably from 20 to 100 microns.

The particle screening can be conducted before, during, or after an ion exchange process such as described above whereby the sodium content of the ZS material is lowered below 12%. The lowering of sodium content to below 3% can occur over several steps in conjunction with screening or can occur entirely before or after the screening step. Particles having a sodium content below 3% may be effective with or without screening of particles sizes as described herein.

In addition to screening or sieving, the desired particle size distribution may be achieved using a granulation or other agglomeration technique for producing appropriately sized particles.

In another embodiment, the ZS compositions may further comprise atoms or molecules attached onto their surfaces to produced grafted crystals. The grafted atoms or molecules are attached to the surface of the ZS, preferably through stable covalent bonds. In one embodiment, an organosilicate moiety is grafted onto the surface of the ZS composition through reacting active groups such as silanols ($\equiv$Si-O-H) on the surface of crystals. This may be accomplished, for example by using aprotic solvents. In another embodiment, an alkoxysilane may be grafted and would require the use of a corresponding alcohol to perform the reaction. Identifying free silanol groups on the surface can done through, for example by, Infrared spectroscopy. In another embodiment, if the material to graft lacks of the active groups on their surface, acid washes can be used to promote their formation. Following successful grafting, the ZS compositions may further comprise tagging the composition with radioactive isotopes, such as but not limited to C or Si. In an alternative embodiment, the ZS compositions may also comprise non-exchangeable atoms, such as isotopes of Zr, Si, or O, which may be useful in mass-balance studies.

It is also within the scope of the invention that these microporous ion exchange compositions can be used in powder form or can be formed into various shapes by means well known in the art. Examples of these various shapes include pills, extrudates, spheres, pellets and irregularly shaped particles. It is also envisioned that the various forms can be packaged in a variety of known containers. These might include capsules, plastic bags, pouches, packets, sachets, dose packs, vials, bottles, or any other carrying device that is generally known to one of skill in the art.

The microporous ion exchange crystals of this invention may be combined with other materials to produce a composition exhibiting a desired effect. The ZS compositions may be combined with foods, medicaments, devices, and compositions that are used to treat a variety of diseases. For example, the ZS compositions of the present invention may be combined with toxin reducing compounds, such as charcoal, to expedite toxin and poison removal. In another embodiment, the ZS crystals may exist as a combination of two or more forms of ZS of ZS-1 to ZS-11. In one embodiment, the combination of ZS may comprise ZS-9 and ZS-11, more preferably ZS-9 and ZS-7, even more preferably ZS-9, ZS-11, and ZS-7. In another embodiment of the present invention, the ZS composition may comprise a blend or mixture of ZS-9, wherein ZS-9 is present at greater than at least 40%, more preferably greater than at least 60%, even more preferably greater than or equal 70%, where the remainder may comprise mixtures of other forms of ZS crystals (i.e., ZS-1 to ZS-11) or other amorphous forms. In another embodiment, the blend of ZS-9 may comprise greater than about between 50% to 75% ZS-9 crystals and greater than about 25% to about 50% ZS-7 crystals with the remainder being other forms of ZS crystals, wherein the remainder of the ZS crystals does not include ZS-8 crystals.

As stated, these compositions have particular utility in adsorbing various toxins from fluids selected from bodily fluids, dialysate solutions, and mixtures thereof. As used herein, bodily fluids will include but not be limited to blood and gastrointestinal fluids. Also by bodily is meant any mammalian body including but not limited to humans, cows, pigs, sheep, monkeys, gorillas, horses, dogs, etc. The instant process is particularly suited for removing toxins from a human body.

The zirconium metallates can also be formed into pills, tablets or other shapes which can be ingested orally and pickup toxins in the gastrointestinal fluid as the ion exchanger transits through the intestines and is finally excreted. In one embodiment, the ZS compositions may be made into wafer, a pill, a powder, a medical food, a suspended powder, or a layered structure comprising two or more ZS. In order to protect the ion exchangers from the high acid content in the stomach, the shaped articles may be coated with various coatings which will not dissolve in the stomach, but dissolve in the intestines. In one embodiment, the ZS may be shaped into a form that is subsequently coated with an enteric coating or embedded within a site specific tablet, or capsule for site specific delivery.

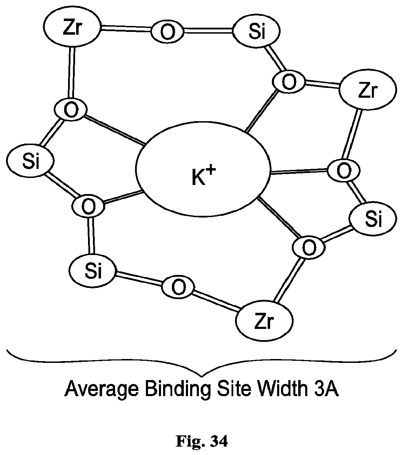

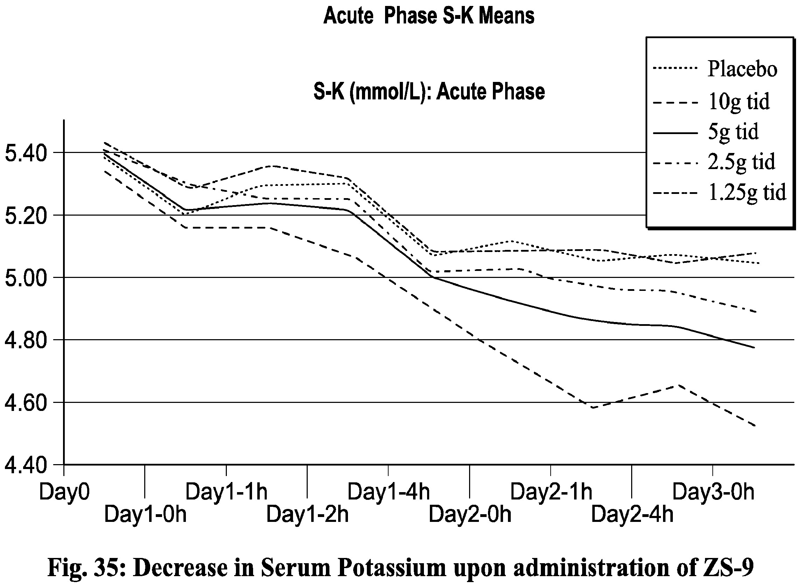

The pills or tablets described herein are produced using a high shear granulation process followed by a blending and compression into a pill, tablet, or any other shape. An example of a compressed tablet can be seen at figures 34 and 35. Those of skill in the art will appreciate that the pills, tablets or other shapes of compression will comprise the usual excipients required for the formation of a compressed composition. These will include controlled delivery components (such as, but not limited to hydroxypropyl metylcellulose HPMC), binders (such as but not limited to microcrystalline cellulose, dibasic calcium phosphate, stearic acid, dextrin, guar gum, gelatin), disintegrants (such as but not limited to, starch, pregelatinized starch, fumed silica or crospovidone), lubrincants or anti-adherent (such as but not limited to magnesium stearate, stearic acid, talc, or ascorbyl palmitate), flavoring agents (fructose, mannitol, citric acid, malic acid, or xylitol), coating agents (carnauba wax, maltodextrin, or sodium citrate) , stabilizer (such as but not limited to carob), gelling agent, and/or emulsifying agents (such as but not limited to lecithin, beeswax). Those of skill in the art will understand that these excipients may be substituted for others depending on the specific function sought.

As has also been stated, although the instant compositions are synthesized with a variety of exchangeable cations ("A"), it is preferred to exchange the cation with secondary cations (A') which are more compatible with blood or do not adversely affect the blood. For this reason, preferred cations are sodium, calcium, hydronium and magnesium. Preferred compositions are those containing sodium and calcium, sodium and magnesium sodium, calcium and hydronium ions, sodium, magnesium, and hydronium ions, or sodium calcium, magnesium, and hydronium ions. The relative amount of sodium and calcium can vary considerably and depends on the microporous composition and the concentration of these ions in the blood. As discussed above, when sodium is the exchangeable cation, it is desirable to replace the sodium ions with hydronium ions thereby reducing the sodium content of the composition.

ZS crystals as described in related U.S. Application 13/371,080, which is incorporated by reference in its entirety, have increased cation exchange capacities or potassium exchange capacity. These increased capacity crystals may also be used in accordance with the present invention. The dosage utilized in formulating the pharmaceutical composition in accordance to the present invention will be adjusted according to the cation exchange capacities determined by those of skill in the art. Accordingly, the amount of crystals utilized in the formulation will vary based on this determination. Due to its higher cation exchange capacity, less dosage may be required to achieve the same effect.

The compositions of the present invention may be used in the treatment of diseases or conditions relating to elevated serum potassium levels. These diseases may include for example chronic or acute kidney disease, chronic, acute or sub-acute hyperkalemia. To those patients suffering from diseases or conditions with elevated serum potassium levels, the product of the present invention is administered at specific potassium reducing dosages. The administered dose may range from approximately 1.25-15 grams (~18-215 mg/Kg/day) of ZS, preferably 8-12 grams (~100-170 mg/Kg/day), more preferably 10 grams (~140 mg/Kg/day) three times a day. In another embodiment, the total administered dose of the composition may range from approximately 15-45 gram (~215-640 mg/Kg/day), preferably 24-36 grams (~350-520 mg/Kg/day), more preferably 30 grams (~400 mg/Kg/day). When administered to a subject, the composition of the present invention is capable of decreasing the serum potassium levels to near normal levels of approximately 3.5-5 mmol/L. The molecular sieves of the present product are capable of specifically removing potassium without affecting other electrolytes, (i.e., no hypomagnesemia or no hypocalcemia). The use of the present product or composition is accomplished without the aid of laxatives or other resins for the removal of excess serum potassium.

Acute hyperkalemia requires an immediate reduction of serum potassium levels to normal or near normal levels. Molecular sieves of the present invention which have a KEC in the range of approximately 1.3-2.5 meq/g would be capable of lowering the elevated levels of potassium to within normal range in a period of about 1-8 hours after administration. In one embodiment, the product of the present invention is capable of lowering the elevated levels in about at least 1, 2, 4, 6, 8, 10 hours after administration. The dose required to reduce the elevated potassium levels may be in the range of about 5-15 grams, preferably 8-12 grams, more preferably 10 grams. Molecular sieves having a higher KEC in the range of approximately 2.5-4.7 meq/g would be more efficient in absorbing potassium. As a result, the dose required to reduce the elevated potassium levels may be in the range of about 1.25-6 grams. The schedule of dose administration may be at least once daily, more preferably three times a day.

The treatment of chronic and sub-acute hyperkalemia will require maintenance dosing to keep potassium levels near or within normal serum potassium levels. As such, the administration of the product of the present invention will be lower than that prescribed to patients suffering from acute hyperkalemia. In one embodiment, compositions comprising molecular sieves having KEC in the range of approximately 2.5-4.7 meq/g will be scheduled for a dose in the range of approximately 1-5 grams, preferably 1.25-5 grams, preferably 2.5-5 grams, preferably 2-4 grams, more preferably 2.5 grams. Compositions comprising molecular sieves having a KEC in the range of approximately 2.5-4.7 meq/g will receive less and will be scheduled for a dose in the range of approximately 0.4-2.5 grams, preferably 0.8-1.6 grams, preferably 1.25-5 grams, preferably 2.5-5 grams, more preferably 1.25 grams. Compliance in this subset of patients is a major factor in maintaining normal potassium levels. As such, dosing schedule will therefore be an important consideration. In one embodiment, the dose will be given to patients at least three times a day, more preferably once a day.

One preferred aspect of the invention is its use of microporous zirconium silicate in the treatment of chronic kidney disease and/or chronic heart disease. The use of therapies comprising diuretics is common in the treatment of chronic kidney disease and/or chronic heart disease. Prior attempts to treat these conditions by using therapies comprising diuretics led to undesirable effects such as hyperkalemia. The inventors have observed that administration of microporous zirconium silicate to patients suffering from chronic kidney disease and being administered therapies that included diuretics, experienced significant reduction in potassium levels without the negative effects. These negative effects were observed when therapies comprising diuretics were used in connection with ACE inhibitors and ARB therapy. The inventors have also unexpectedly observed that systemic aldosterone reduction is achieved through administration of microporous zirconium silicate without the negative effects of the aldosterone blockers.

These observations demonstrate that zirconium silicate according to the present invention will be effective in treating patients suffering from chronic kidney disease. Administration of microporous zirconium silicate to these patients currently on therapies that include diuretics reduces the risk of developing hyperkalemia and also reduces aldosterone without inducing hyperkalemia. The zirconium silicate can be administered alone or in combination with existing treatments that include diuretics or diuretics and ACE inhibitors and/or ARB therapy. Given the separate mechanism of action of zirconium silicate and ACE/ARB therapy, the administration of microporous zirconium silicate in conjunction with these therapies is expected to improve the effects upon the renin-angiotensin-aldosterone system (RAAS) and further mitigate the negative effects of aldosterone on CKD and CVD. The different mechanisms and independent aldosterone-lowering ability of microporous zirconium silicate are expected to result in at least additive and possibly synergistic interaction between the combined therapies.

In another embodiment, the diuretics may include any diuretic selected from the three general classes of thiazine or thiazine-like, loop diuretics, or potassium sparing diuretics. In one preferred embodiment, the diuretic is potassium sparing diuretic, such as spironolactone, eplerenone, canrenone (e.g., canrenoate potassium), prorenone (e.g., prorenoate potassium), and mexrenone (mextreoate potassium), amiloride, triamterene, or benzamil. The following are examples of possible diuretics that can be used in combination with microporous zirconium silicate according to the invention: furosemide, bumetanide, torsemide, etacrynic acid, etozoline, muzolimine, piretanide, tienilic acid, bendroflumethiazide, chlorthiazide, hydrochlorthiazide, hydroflumethiazide, cyclopenthiazide, cyclothiazide, mebutizide, hydroflumethiazide, methyclothiazide, polythiazide, trichlormethiazide, chlorthalidone, indapamide, metolazone, quinethazone, clopamide, mufruside, clofenamide, meticrane, xipamide, clorexidone, fenquizone.

The following are examples of ACE inhibitors that can be used in combination with microporous zirconium silicate according to the invention: sulfhydryl-containing agents including captopril or zofenopril; dicarboxylate-containing agents including enalapril, ramipril, quinapril, perindopril, lisinopril, benazepril, imidapril, zofenopril, trandolapril; phosphate-containing agents including fosinopril; and naturally-occuring ACE inhibitors including casokinins and lactokinins. The following are examples of ARBs that can be used in combination with microporous zirconium silicate according to the present invention: valsartan, telmisartan, losartan, irbesartan, azilsartan, and olmesartan. Combinations of the above are particularly desirable. For example, a preferred method of treating CKD and/or CVD includes administration of microporous zirconium silicate, ramapril (ACE inhibitor) and telmisartan (ARB). For example, the invention may involve administration of microporous zirconium silicate in conjunction with combination therapy of ramapril/telmisartan to a patient diagnosed with chronic kidney disease. The ACE inhibitors and ARBs may be administered at their standard dose rates for the treatment of CKD, and in some instances at lower doses depending on the degree of synergy between the ACE inhibitor/ARBs in combination with microporous zirconium silicate.

Another approach to treating CKD and/or CVD involves administering microporous zirconium silicate with an aldosterone antagonist, i.e., an anti-mineralocorticoid. These agents are often used in adjunctive therapy for the treatment of chronic heart failure. Based on the observations of the inventor regarding the effects of microporous zirconium silicate on aldosterone, the combination of microporous zirconium silicate with an aldosterone antagonist may provide for additive and/or synergistic activity. Suitable aldosterone antagonists include spironolactone, eplerenone, canrenone (*e.g.,* canrenoate potassium), prorenone (*e.g.,* prorenoate potassium), and mexrenone (mextreoate potassium).

Another preferred embodiment relates to the co-administration of microporous zirconium silicates, preferably ZS-9, to patients who have undergone organ replacement or transplantation. Typically these patients will require the administration of an immunosuppressant to reduce the risk of organ rejection by the immune system. Unfortunately, these drugs also elevate levels of potassium in the patient, which increases the risk of developing hyperkalemia. Immunosuppressants may include either induction drugs or maintenance drugs (such as calcineurin inhibitors, antiproliferative agents, mTor inhibitors, or steroids). The inventors of the present invention have unexpected found that therapy using microporous ZS in combination with an immunosuppressant reduces the risk of developing hyperkalemia by lowering the serum potassium levels. Typical immunosuppressant may include tacrolimus, cyclosporine, mycophenolate mofetil, mycophenolate sodium, azathioprine, sirolimus, and/or prednisone.

The inventors have unexpectedly found that the administration of microporous ZS to diabetes patients, specifically diabetes mellitus patients, is able to reduce the serum levels of potassium. The inventors have also found that patients with diabetes may continue the renin-angiotensin aldosterone system inhibitors when combined with administration of ZS without the risk of increasing the serum potassium levels. Thus, in one embodiment of the invention is a method of treating diabetes patients who are being administered renin-angiotensin aldosterone system inhibitors a composition comprising microporous ZS. In yet another embodiment of the invention, a patient may be administered a combination of renin-angiotensin aldosterone system inhibitors and a microporous ZS, preferably ZS-9.

The composition or product of the present invention may be formulated in a manner that is convenient for administration. For example, the composition of the present invention may be formulated as a tablet, capsule, powder, granule, crystal, packet, or any other dose form that is generally known to one of skill in the art. The various forms can be formulated as individual dosages comprising between 5-15 grams, preferably 8-12 grams, or more preferably 10 grams for multiple administrations per day, week or month; or they may be formulated as a single dosage comprising between 15-45 grams, preferably 24-36 grams, or more preferably 30 grams. In an alternative embodiment, the individual dosage form can be at least greater than 1, 2, 3, 4, 5, 6, 7, 8, 9, 10, 20, 30, or 40 grams. If the dosage form is tablet, it may be formulated as a granule, granule-like, or as an extended release form. Capsules may be formulated for administration three times a day, as a sprinkle, an extended release sprinkle, or a dose pack. Powders may be formulated for reconstitution, contained in plastic bags or packets. Those of skill in the art will recognize that the above description of dosage forms is not limiting and that other dosage forms for solids may be used to administer the product or composition of the present invention.

Surprisingly, the administration of the composition of the present invention at the specifically described dosing of approximately 10 grams (~140 mg/Kg/day) three times a day (*i.e.,* 30 grams (~400 mg/Kg/day) total) is capable of reducing potassium levels in the serum for an extended duration of time. The inventors have found that when the product or composition of the present invention is administered at a dosage of approximately 10 grams three times a day, the effects of lowering serum potassium levels to within normal levels is sustained for 5 days after 2 days of acute therapy. It was expected, however, that the product of the present invention would be expelled in a relatively quick manner.

The ZS of the present invention may be modified and/or combined with other drugs or treatments if multiple conditions or diseases are present in a subject. For example, in one embodiment a subject may present with both hyperkalemia and chronic kidney disease, in which Na-ZS compositions may be used. In another embodiment, the ZS compositions used to treat chronic kidney disease may further comprise sodium bicarbonate in combination with protonated forms of the ZS. In another embodiment, subjects presenting with hyperkalemia and chronic heart failure may require the use of protonated ZS compositions. In another embodiment, the treatment of hyperkalemia and chronic heart disease will require no more than 10% sodium present in the ZS, more preferably less than 2% sodium.

In other embodiments of the invention, the ZS described herein may be further combined with activated carbon. The activated carbon has the effect of attracting organic molecules circulating within the system of a subject. *See,* e.g., HSGD Haemosorbents for Medical Device Applications, Nikolaev V.G. Presentation, London. As such, the combination of activated carbon with a ZS will act as a combination product having the ability to remove both excess potassium, and organic molecules. The activated carbon will comprise a multiplicity of adsorption pores of ranging from about 8 angstroms to about 800 angstroms in diameter, preferably at least about 50 angstroms in diameter. The ZS combined with activated carbon of the present invention will be useful in the treatment of many diseases and/or conditions requiring the removal of excess organic materials, such as but not limited to, lipids, proteins, and toxins. For example, the carbon containing ZS compositions of the present invention will be useful in the removal of pyrimidines, methylguanidines, guanidines, *o*-hydroxyhippuric acid, *p*-hydroxyhippuric acid, parathormone, purines, phenols, indols, pesticides, carcinogenic heterocyclic amines, conjugates of ascorbic acids, trihalomethanes, dimethylarginine, methylamines, organic chloramines, polyamines, or combinations thereof. The activated carbon combined with ZS will also be useful in adsorbing elevated levels of bile acids, albumin, ammonia, creatinine and bilirubin. To further improve the adsorption of activated carbon with coated ZS, the composition may be further coated with an albumin layer, a lipid layer, a DNA layer, a heparin layer, resulting in additional adsorption efficiencies ranging from about 12% to about 35%.

The activated carbon and ZS compositions will be useful in treating a subject presenting with multiple diseases or conditions, such as hyperkalemia, acute and chronic esogastritis, acute and chronic intestinal catarrhus, hyperacid gastritis, summer diarrhea, catarrhal jaundice, food related toxicoinfections, kidney disease, dysentery, choloera, typhoid, intestinal bacilli-carrier, heartburn, nausea, acute viral hepatitis, chronic active hepatitis and cirrhosis, concomitant hepatitis, mechanical jaundice, hepato-renal failure, hepatic coma, or combinations thereof.

In another embodiment, the ZS compositions described herein may be used in a variety of methods comprising administering to a subject in need thereof a composition described herein to remove excess levels of potassium. In another embodiment of the present invention, the method may include the administration of a combination of the ZS described herein and may further comprise additional compositions to aid in the removal of potassium while simultaneously removing other substances, such as but not limited to toxins, proteins, or ions, from the subject.

In order to more fully illustrate the invention, the following examples are set forth. It is to be understood that the examples are only by way of illustration and are not intended as an undue limitation on the broad scope of the invention as set forth in the appended claims.

## EXAMPLE 1

A solution was prepared by mixing 2058 g of colloidal silica (DuPont Corp. identified as Ludox™ AS-40), 2210 g of KOH in 7655 g $H_2O$. After several minutes of vigorous stirring 1471 g of a zirconium acetate solution (22.1 wt. % $ZrO_2$) were added. This mixture was stirred for an additional 3 minutes and the resulting gel was transferred to a stainless steel reactor and hydrothermally reacted for 36 hours at 200°C. The reactor was cooled to room temperature and the mixture was vacuum filtered to isolate solids which were washed with deionized water and dried in air.

The solid reaction product was analyzed and found to contain 21.2 wt. % Si, 21.5 wt. % Zr, K 20.9 wt. % K, loss on ignition (LOI) 12.8 wt. %, which gave a formula of $K_{2.3}ZrSi_{3.2}O_{9.5}*3.7H_2O$. This product was identified as sample A.

## EXAMPLE 2

A solution was prepared by mixing 121.5 g of colloidal silica (DuPont Corp. identified as Ludox® AS-40), 83.7 g of NaOH in 1051 g $H_2O$. After several minutes of vigorous stirring 66.9 g zirconium acetate solution (22.1 wt. % $ZrO_2$) was added. This was stirred for an additional 3 minutes and the resulting gel was transferred to a stainless steel reactor and hydrothermally reacted with stirring for 72 hours at 200°C. The reactor was cooled to room temperature and the mixture was vacuum filtered to isolate solids which were washed with deionized water and dried in air.

The solid reaction product was analyzed and found to contain 22.7 wt. % Si, 24.8 wt. % Zr, 12.8 wt. % Na, LOI 13.7 wt. %, which gives a formula $Na_{2.0}ZrSi_{3.0}O_{9.0}*3.5H_2O$. This product was identified as sample B.

## EXAMPLE 3

A solution (60.08 g) of colloidal silica (DuPont Corp. identified as Ludox® AS-40) was slowly added over a period of 15 minutes to a stirring solution of 64.52 g of KOH dissolved in 224 g deionized $H_2O$. This was followed by the addition of 45.61 g zirconium acetate (Aldrich 15-16 wt. % Zr, in dilute acetic acid). When this addition was complete, 4.75 g hydrous $Nb_2O_5$ (30 wt. % LOI) was added and stirred for an additional 5 minutes. The resulting gel was transferred to a stirred autoclave reactor and hydrothermally treated for 1 day at 200°C. After this time, the reactor was cooled to room temperature, the mixture was vacuum filtered, the solid washed with deionized water and dried in air.

The solid reaction product was analyzed and found to contain 20.3 wt. % Si, 15.6 wt. % Zr, 20.2 wt. % K, 6.60 wt. % Nb, LOI 9.32 wt. %, which give a formula of $K_{2.14}Zr_{0.71}Nb_{0.29}Si_3O_{9.2}*2.32H_2O$. Scanning Electron (SEM) of a portion of the sample, including EDAX of a crystal, indicated the presence of niobium, zirconium, and silicon framework elements. This product was identified as sample C.

## EXAMPLE 4

To a solution prepared by mixing 141.9 g of NaOH pellets in 774.5 g of water, there were added 303.8 g of sodium silicate with stirring. To this mixture there were added dropwise, 179.9 g of zirconium acetate (15% Zr in a 10% acetic acid solution). After thorough blending, the mixture was transferred to a Hastalloy™ reactor and heated to 200°C. under autogenous pressure with stirring for 72 hours. At the end of the reaction time, the mixture was cooled to room temperature, filtered and the solid product was washed with a 0.001 M NaOH solution and then dried at 100°C. for 16 hours. Analysis by x-ray powder diffraction showed that the product was pure ZS-11.

**EXAMPLE 5**

To a container there was added a solution of 37.6 g NaOH pellets dissolved in 848.5 g water and to this solution there were added 322.8 g of sodium silicate with mixing. To this mixture there were added dropwise 191.2 g of zirconium acetate (15% Zr in 10% acetic acid). After thorough blending, the mixture was transferred to a Hastalloy™ reactor and the reactor was heated to 200°C under autogenous conditions with stirring for 72 hours. Upon cooling, the product was filtered, washed with 0.001 M NaOH solution and then dried at 100°C. for 16 hours. X-ray powder diffraction analysis showed the product to be ZS-9 (i.e., a composition that is predominately ZS-9 crystalline form).

**EXAMPLE 6**

Approximately 57g (non-volatile-free basis, lot 0063-58-30) of Na-ZS-9 was suspended in about 25 mL of water. A solution of 0.1N HCl was added gradually, with gentle stirring, and pH monitored with a pH meter. A total of about 178 milliliters of 0.1 N HCl was added with stirring, the mixture filtered then further rinsed with additional 1.2 liters 0.1 N HCl washes. The material was filtered, dried and washed with DI water. The pH of the resulting material was 7.0. The H-ZS-9 powder resulting from this three batch-wise ion exchange with 0.1 N HCl has < 12% Na.
As illustrated in this example, batch-wise ion exchange with a dilute strong acid is capable of reducing the sodium content of a NA-ZS-9 composition to within a desired range.

**EXAMPLE 7**

Approximately 85 gram (non-volatile-free basis, lot 0063-59-26) of Na-ZS-9 was washed with approximately 31 Liters of DI water at 2 Liter increments over 3 days until the pH of the rinsate reached 7. The material was filtered, dried and washed with DI water. The pH of the resulting material was 7. The H-ZS-9 powder resulting from batch-wise ion exchange and water wash has < 12% Na.
As illustrated in this example, water washing is capable of reducing the sodium content of a NA-ZS-9 composition to within a desired range.

**EXAMPLE 8**

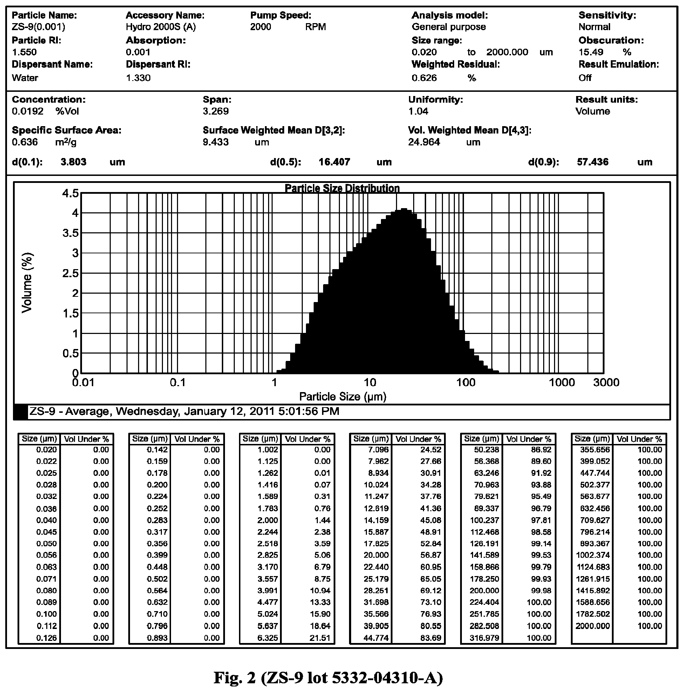

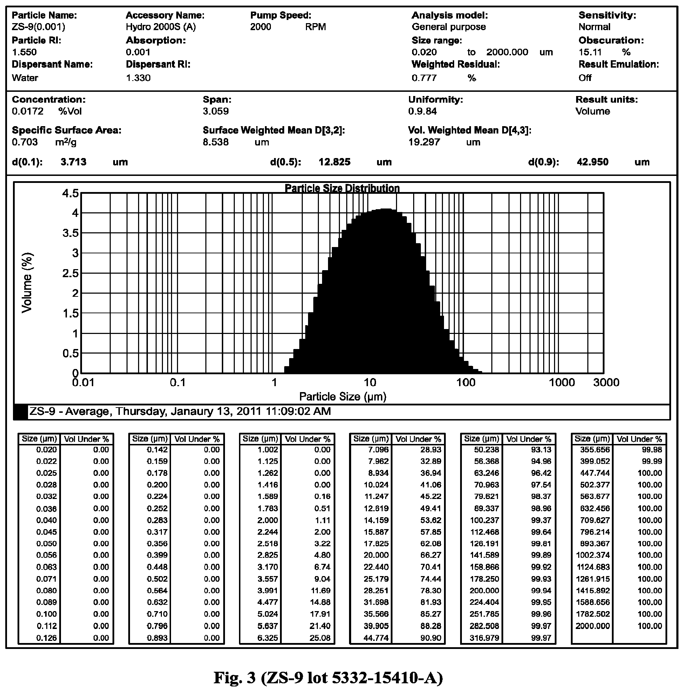

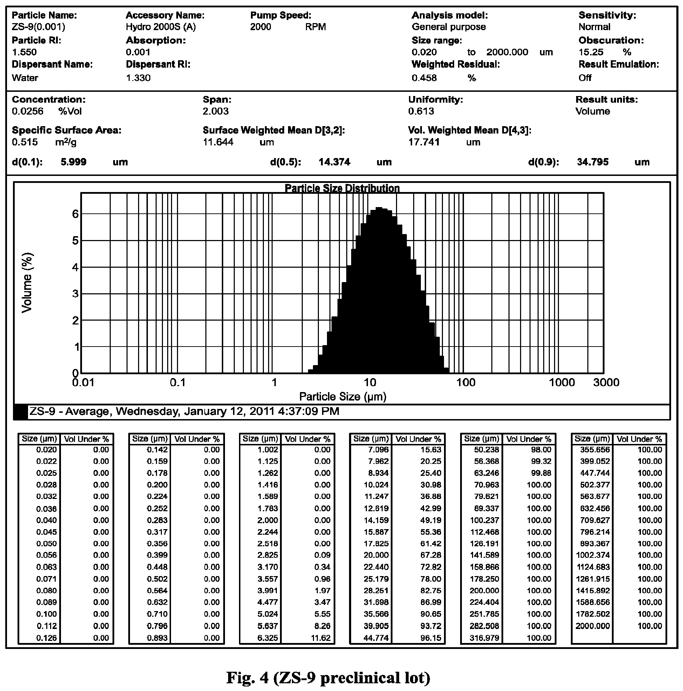

Separate batches of ZS-9 crystals were analyzed using light scatter diffraction techniques. The particle size distribution and other measured parameters are shown in Figs. 2-4. The d(0.1), d(0.5), and d(0.9) values represent the 10%, 50%, and 90% size values. The cumulative particle size distribution is shown in Fig. 4-6. As can be seen from the following figures, the cumulative volume of particles having a diameter below 3 microns ranges from approximately 0.3% to approximately 6%. In addition, different batches of ZS-9 have different particle size distributions with varying levels of particles having a diameter of less than 3 microns.

**EXAMPLE 9**

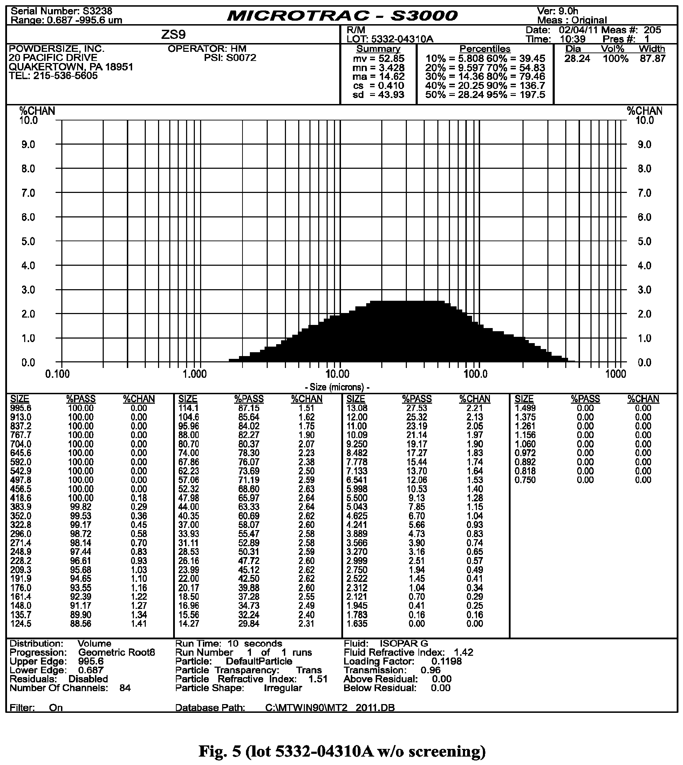

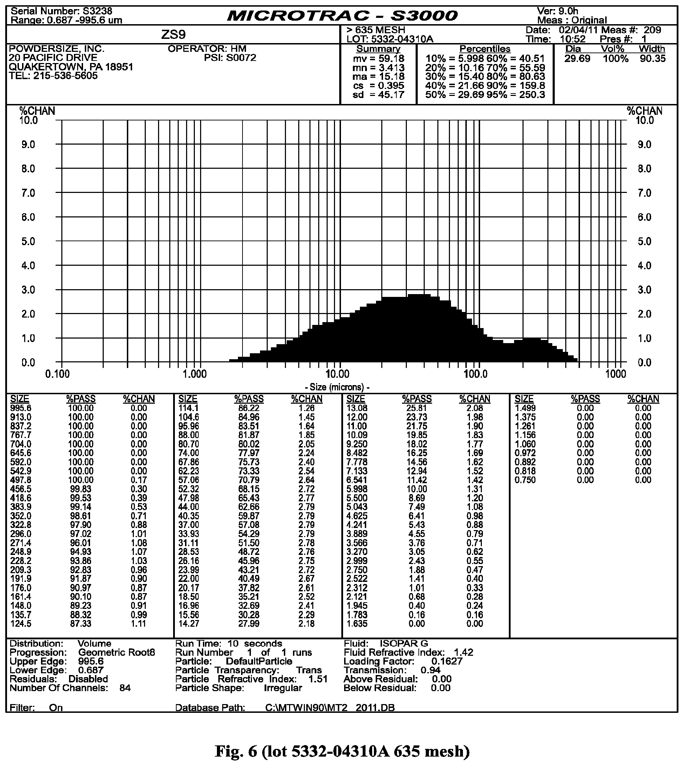

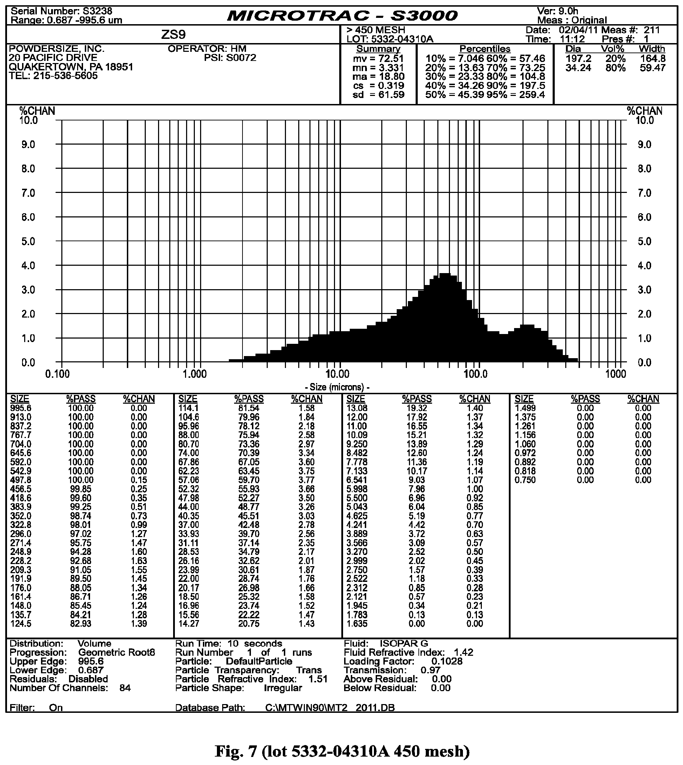

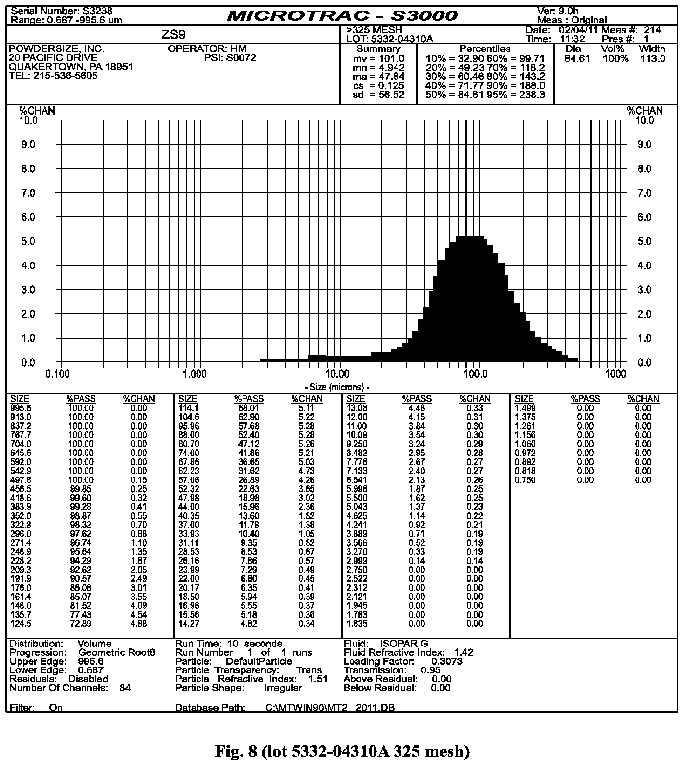

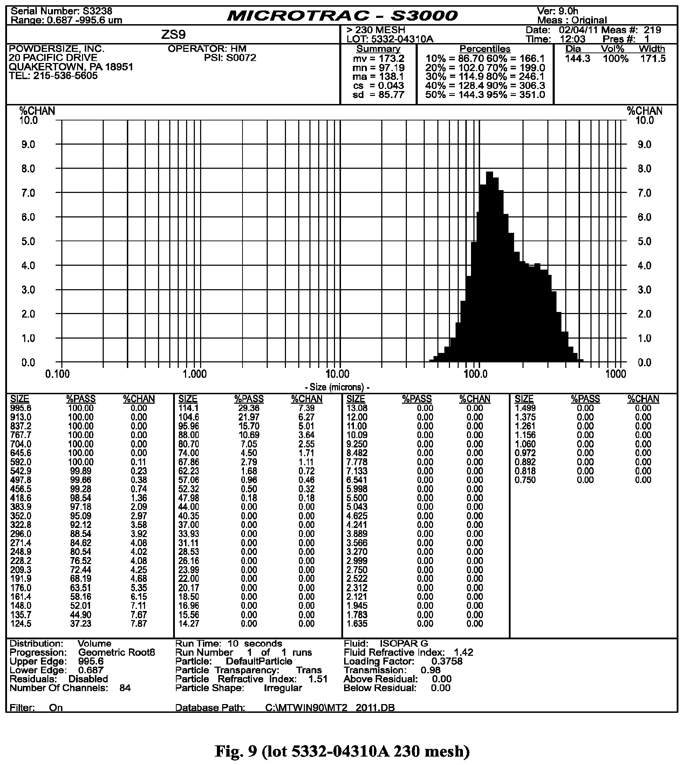

Crystals of ZS-9 were subject to screening to remove small diameter particles. The resulting particle size distribution of the ZS-9 crystals screened using different size screens was analyzed. As illustrated in the following figures, the fraction of particles having a diameter below 3 microns can be lowered and eliminated using an appropriate mesh size screen. Without screening, approximately 2.5% percent of the particles had a diameter of below 3 microns. *See* Fig. 5. Upon screening with a 635 mesh screen, the fraction of particles having a diameter below 3 microns was reduced to approximately 2.4%. *See* Fig. 6. Upon screening with a 450 mesh screen, the fraction of particles having a diameter below 3 microns was reduced further to approximately 2%. *See* Fig. 7. When a 325 mesh screen is used, the fraction of particles having a diameter below 3 microns is further reduced to approximately 0.14%. *See* Fig. 8. Finally, a 230 mesh screen reduces the fraction of particles below 3 microns to 0%. *See* Fig. 9.
The screening techniques presented in this example illustrate that particle size distributions may be obtained for ZS-9 that provide little or no particles below 3 microns. It will be appreciated that ZS-9 according to Example 5 or H-ZS-9 according to Examples 6 and 7 may be screened as taught in this example to provide a desired particle size distribution. Specifically, the preferred particle size distributions disclosed herein may be obtained using the techniques in this example for both ZS-9 and H-ZS-9.

**EXAMPLE 10**

A 14-Day repeat dose oral toxicity study in Beagle Dogs with Recovery was conducted. This GLP compliant oral toxicity study was performed in beagle dogs to evaluate the potential oral toxicity of ZS-9 when administered at 6 h intervals over a 12 h period, three times a day, in food, for at least 14 consecutive days. In the Main Study ZS-9 was administered to 3/dogs/sex/dose at dosages of 0 (control), 325, 650 or 1300 mg/kg/dose. An additional 2 dogs/sex/dose, assigned to the Recovery Study, received 0 or 1300 mg/kg/dose concurrently with the Main study animals and were retained off treatment for an additional 10 days. A correction factor of 1.1274 was used to correct ZS-9 for water content. Dose records were used to confirm the accuracy of dose administration.

During the acclimation period (Day -7 to Day -1) dogs were trained to eat 3 portions of wet dog chow at 6 h intervals. During treatment the requisite amount of test article (based on the most recently recorded body weight) was mixed with ~100g of wet dog food and offered to the dogs at 6 h intervals. Additional dry food was offered following consumption of the last daily dose. Each dog received the same amount of wet dog feed. Body weights were recorded at arrival and on Days -2, -1, 6, 13 and 20. Clinical observations were performed twice daily during the acclimation, treatment and recovery periods. Wet and dry food consumption was measured daily during the treatment period. Blood and urine samples for analysis of serum chemistry, hematology, coagulation and urinalysis parameters were collected pretest (Day -1) and Day 13. Ophthalmologic examinations were performed pretest (Day -6/7) and on Day 7 (females) or 8 (males). Electrocardiographic assessments were performed pretest (Day -1) and on Day 11. At study termination (Day 14- Main Study and Day 24- Recovery Study), necropsy examinations were performed, protocol specified organ weights were weighed, and selected tissues were microscopically examined.

Oral administration of 325, 650 and 1300 mg ZS-9/kg/dose with food, three times a day at 6 h intervals over a 12-hour period for 14 days was well tolerated. Clinical signs were limited to the observation of white material, presumed to be test article, in the feces of some dogs at the 325 mg/kg/dose and in all animals receiving $\geq$ 650 mg/kg/dose during the second week of treatment. There were no adverse effects on body weight, body weight change, food consumption, hematology and coagulation parameters or ophthalmoscopic and ECG evaluations.

There were no macroscopic findings associated with administration of ZS-9. Microscopically, minimal to mild focal and/or multifocal inflammation was observed in the kidneys of treated animals but not in Control animals. The lesions had similar incidence and severity at 650 and 1300 mg/kg and were less frequent and severe at 325 mg/kg. In some dogs the inflammation was unilateral rather than bilateral and in some cases was associated with inflammation in the urinary bladder and origin of the ureter. Taken together these observations suggest that factors other than direct renal injury, such as alterations in urine composition of ZS-9-treated dogs may have resulted in increased susceptibility to subclinical urinary tract infections, even though no microorganisms were observed in these tissues. In recovery animals the inflammation was completely resolved in females and partly resolved in males suggesting that whatever the cause of the inflammation it was reversible following cessation of dosing.

The increased incidence of mixed leukocyte inflammation observed in Beagle dogs treated with ZS-9 is summarized below.

| Summary of Inflammation in Kidneys | | | | | | | | | |
|---|---|---|---|---|---|---|---|---|---|
| Terminal Necropsy (TN): Day 14 | | | | | | | | | |
| Dose | | 0 mg/kg | | 325 mg/kg | | 650 mg/kg | | 1,300 mg/kg | |
| Sex | | M | F | M | F | M | F | M | F |
| Number of Animals | | 3 | 3 | 3 | 3 | 3 | 3 | 3 | 3 |
| Left Kidney | Incidence | 0/3 | 0/3 | 0/3 | 2/3 | 2/3 | 3/3 | 3/3 | 3/3 |
| | minimal | 0/3 | 0/3 | 0/3 | 2/3 | 2/3 | 2/3 | 3/3 | 1/3 |
| | mild | 0/3 | 0/3 | 0/3 | 0/3 | 0/3 | 1/3 | 0/3 | 2/3 |
| | | | | | | | | | |
| Right Kidney | Incidence | 0/3 | 0/3 | 1/3 | 1/3 | 2/3 | 3/3 | 2/3 | 2/3 |
| | minimal | 0/3 | 0/3 | 1/3 | 1/3 | 2/3 | 1/3 | 2/3 | 0/3 |
| | mild | 0/3 | 0/3 | 0/3 | 0/3 | 0/3 | 2/3 | 0/3 | 2/3 |
| | | | | | | | | | |
| Both Kidneys | Incidence | 0/6 | 0/6 | 1/6 | 3/6 | 4/6 | 6/6 | 5/6 | 5/6 |
| | minimal | 0/6 | 0/6 | 1/6 | 3/6 | 4/6 | 3/6 | 5/6 | 1/6 |
| | mild | 0/6 | 0/6 | 0/6 | 0/6 | 0/6 | 3/6 | 0/6 | 4/6 |

(continued)

| Sum of Severity Scores | 0 | 0 | 2 | 3 | 4 | 9 | 5 | 9 |
| --- | --- | --- | --- | --- | --- | --- | --- | --- |
| | 0 | | 5 | | 13 | | 14 | |
| Mean Group Severity Scores | 0.00 | | 0.83 | | 2.17 | | 2.33 | |

Minimal acute urinary bladder inflammation and unidentified crystals were also observed in the renal pelvis and urine of females dosed at 650 mg/kg/dose as summarized below

| Summary of Crystals observed at the 650 mg/kg/dose | | | |
| --- | --- | --- | --- |
| Animal No | 4420 | 4421 | 4422 |
| Unidentified crystals in urine | + | - | + |
| Crystals in renal pelvis | - | + | - |
| Urinary bladder acute inflammation | + | + | - |

Crystals were not identified in group 2 or 4 females or in any ZS-9 treated males.

In both studies it was noted that urinary pH was elevated compared to control and it was postulated that the change in urinary pH and/or urinary composition affected urine solute solubility resulting in crystal formation that caused urinary tract irritation and/or increased susceptibility to urinary tract infections (UTIs).

The description of the urinary crystals (long thin spiky clusters) coupled with the particle size profile and insolubility of test article make it very unlikely that these crystals are ZS-9.

**EXAMPLE 11**

Crystals of ZS-9 are prepared and designated "ZS-9 Unscreened." Screening in accordance with the procedures of Example 10 is conducted on a sample of ZS-9 crystals and the screened sample is designated "ZS-9 >5µm." Another sample of Crystals of ZS-9 undergo an ion exchange in accordance with the procedures of Example 6 above and are then screened in accordance with the procedures of Example 10. The resulting H-ZS-9 crystals are designated "ZS-9 + >5µm."

The following 14-day study is designed to show the effect of particle size and particle form on the urinary pH and presence of crystals in the urine. The compounds above are administered to beagles orally by mixing with wet dog food. The regimen is administered 3 times a day at 6 hour intervals over a 12 hour period in the following manner:

**STUDY DESIGN**

| Group | mg/kg/dose* | Female |
| --- | --- | --- |
| Control | 0 | 3 |
| ZS-9 Unscreened | 600 | 3 |
| ZS-9 >5µm | 600 | 3 |
| ZS-9 + >5µm | 600 | 3 |
| ZS-9 Unscreened | 100 | 3 |
| ZS-9 >5µm | 100 | 3 |
| ZS-9 + >5µm | 100 | 3 |
| $NaHCO_3$ | 50 | 3 |
| * uncorrected for water ZS-9+ = pH neutral crystal | | |

| Total number of dogs | 24 females |
| --- | --- |

(continued)

| Age | 5 months of age on arrival |
| --- | --- |
| Acclimation | $\geq$ 10 days |
| Test Article Formulation | Mixed with wet dog food |
| Test article administration | Within 30 minutes of administration |
| Dose Formulation Analysis | Dose records will be used to confirm dosing. Weight of any remaining wet food will be recorded. |

The following table outlines the observations, toxicokinetic evaluation, laboratory investigation (hematology, urinalysis), and terminal procedures.

| OBSERVATIONS | |
| --- | --- |
| Mortality & Signs of ill health or reaction to treatment | Twice daily (after treatment and evening) including feces assessment |
| Detailed Exam | During acclimation, weekly on study |
| Body Weights | Arrival, Day -1, Day 7 and 14 |
| Food Consumption | Daily (Wet and Dry food) |
| Ophthalmoloscopy | None |

| TOXICOKINETIC (FOR POTENTIAL ZR ANALYSIS) | |
| --- | --- |
| 3 X 1 ml whole blood/sample with sample weights recorded | Day -1: Pre-dose |
| | Day 13: Pre-dose and 4 h post 2nd dose |

| LABORATORY INVESTIGATIONS | |
| --- | --- |
| **Hematology/Clinical chemistry** (see list) | Pretreatment and during Weeks 1 and 2 on study |
| **Urinalysis** (see list) | Pretreatment and during Weeks 1 and 2 on study (Metabolic cage, urine sample to be kept cool) |
| | Remaining urine aliquoted and retained frozen for possible future Zr analysis |

| Terminal Procedures | |
| --- | --- |
| Necropsy | All animals regardless of mode of death. All tissues collected into NBF (see list) |
| Histopathology | Urinary tract only (Kidney and bladder) |

During this study in female dogs, the test articles, ZS-9 unscreened, ZS-9>5$\mu$m, and ZS-9 + >5$\mu$m, were administered three times daily at 6 hour intervals over a 12-hour period for 14 consecutive days via dietary consumption utilizing a wet food vehicle. The dose levels were 100 or 600 mg/kg/dose.

All animals survived the 14-day administration period. There were no test article-related changes in mortality, body weight, body weight gain, organ weights, macroscopic findings, or on clinical chemistry or blood gas parameters. ZS-9 related findings were limited to an increase in the fractional excretion of sodium and an increase in urinary pH in animals receiving screened or unscreened ZS-9 at a dose of 6000 mg/kg/dose, and deceases in the fractional excretion of potassium and the urinary urea nitrogen/creatinine ratio in animals dosed at 600 mg/kg/dose ZS-9 unscreened, ZS-9>5µm, and ZS-9 + >5µm.

Statistically significant increases in urinary pH compared to Control in animals treated with 600 mg/kg/dose of ZS-9 unscreened and ZS-9>5µm, that was not observed at the 100 mg/kg/dose or in animals treated with 600 mg/kg/dose of ZS-9 + >5µm. Mean urinary pH in these animals increased from 5.33 to ~7.67 on Day 7 and from 5.83 to 7.733 on Day 13. The lack of effect on urinary pH in animals treated with 600 mg/kg/dose of protonated ZS-9 (ZS-9 + >5µm) suggests that the increase in the urinary pH in animals treated with the higher dose of sodium loaded ZS-9 (ZS-9 unscreened and ZS-9>5µm) was a result of gastrointestinal hydrogen absorption.

All differences found in urine volume and specific gravity were considered within an acceptable range for normal biological and/or procedure-related variability. There were some variations between treatment groups among biochemical (protein, ketones, etc.) and microscopic (crystals, blood cells, etc.) urinary components that were also considered within an acceptable range for biological and/or procedure-related variability. Triple phosphate crystals (magnesium ammonium phosphate) were observed in most animals at all study intervals, rarely calcium oxalate dihydrate crystals were also observed in a few animals. Both of these crystal types are considered a normal finding in dogs. No patterns were observed to suggest that any of the crystals observed were treatment or test article-related in any animal. No unidentified crystals were observed in the urinary sediment of any animal.

On Days 7 and 13 the fractional excretion of sodium was increased relative to predose intervals in all groups including controls. Animals receiving 600 mg/kg/dose ZS-9 unscreened, ZS-9>5µm, and ZS-9 + >5µm tended to have increases that were slighter greater (up to 116% relative to controls) than those seen in other treatment groups or among the control animals. The increases observed in these three groups occasionally reached magnitudes that were considered above expected ranges and were attributed to the test article. No discernible differences between the changes observed in these three groups could be identified. There was no difference in the fractional excretion of sodium in animals treated with 600 mg/kg/dose of the protonated ZS-9. These changes were attributed to the test article and were not considered toxicologically adverse.

Significant decreases in the fractional excretion of potassium, relative to Control, were observed in animals treated with 600 mg/kg/dose ZS-9 unscreened, ZS-9>5µm, and ZS-9 + >5µm, and 100 mg/kg/dose ZS-9>5µm on Days 7 and 13. Most of these values reached statistical significance relative to controls on Days 7 and 13. These decreases were attributed to the pharmacological effect of the test article.

On Days 7 and 13 urea nitrogen/creatinine ratio was mildly increased relative to predose intervals in all groups including controls. There were mild decreases in urea nitrogen/creatinine ratios on Days 7 and 13 in animals receiving 600 mg/kg/dose ZS-9 unscreened, ZS-9>5µm, and ZS-9 + >5µm relative to controls (up to 26%). Most of the changes observed in these four groups reached statistical significance compared to controls for Days 7 and 13 although group mean values did not differ appreciably when compared to their respective pretest values. These findings were considered test article-related.

Although there were occasional statistically significant differences among other endpoints, no test article-related effects on creatinine clearance, calcium/creatinine ratio, magnesium/creatinine ratio, or urine osmolality were identified in any treatment group.

Test article related microscopic findings in the kidney were observed at the 600 mg/kg/dose. The most common findings were minimal to mild mixed leukocyte infiltrates (lymphocytes, plasma cells, macrophages and/or neutrophils), and minimal to mild renal tubular regeneration (slightly dilated tubules lined by attenuated epithelial cells, epithelial cells with plump nucleus and basophilic cytoplasm). Minimal pyelitis (infiltration of neutrophils, lymphocytes and plasma cells in the submucosa of the renal pelvis) and minimal renal tubular degeneration/necrosis (tubules lined by hypereosinophilic cells with either pyknotic or karyorrhectic nucleus and containing sloughed epithelial cells and/or inflammatory cells in the lumen) were observed in 1/3 dogs receiving 600 mg/kg/dose ZS-9 unscreened and 1/3 dogs receiving 600 mg/kg/dose ZS-9>5µm. Minimal pyelitis and mixed leukocyte infiltration in the urethra or ureter were also present in some dogs given ZS-9>5µm.

The changes in the kidney were mostly present in the cortex and occasionally in the medulla with a random, focal to multifocal (up to 4 foci) distribution. These foci were variably sized, mostly irregular, occasionally linear (extending from the outer cortex to the medulla), and involved less than 5% of the kidney parenchyma in a given section. Most of these foci consisted of minimal to mild infiltration of mixed leukocytes with minimal to mild tubular regeneration, some foci had only minimal to mild tubular regeneration without the mixed leukocyte infiltrate. A few of these foci (two dogs given 600 mg/kg/dose ZS-9 unscreened and one dog given 600 mg/kg/dose ZS-9>5µm) contained a small number of tubules with degeneration/necrosis. Pyelitis was present in four dogs (one given ZS-9 unscreened 600 mg/kg/dose and three dogs given ZS-9>5µm at 600 mg/kg/dose).

The infiltration of mixed leukocytes was also present in the submucosa of both ureters in dogs given 600 mg/kg/dose ZS-9>5µm and the submucosa of the urethra in animals given 600 mg/kg/dose ZS-9 unscreened, 600 mg/kg/dose ZS-9>5µm. The incidence and/or severity of mixed leukocyte infiltrates in the kidney parenchyma were higher in dogs with pyelitis compared to the dogs without pyelitis. The presence of pyelitis and/or the mixed leukocyte infiltrates in the urethra and ureters in some dogs and the multifocal, random distribution of kidney findings with inflammatory infiltrates are reminiscent of an ascending urinary tract infection and suggest that the kidney findings at the 600 mg/kg/dose are likely an indirect effect of the test article.

In dogs given ZS-9 unscreened at 600 mg/kg/dose, kidneys in two of the three dogs were affected with one or more of the aforementioned findings. All three dogs given ZS-9>5$\mu$m at 600 mg/kg/dose had kidney lesions including pyelitis and mixed leukocyte infiltrates in the submucosa of urethra or ureters. Dogs given ZS-9 + >5$\mu$m at 600 mg/kg/dose, minimal mixed leukocyte infiltrate with tubular regeneration was present in only the left kidney in one dog while another dog had a few foci of minimal tubular regeneration.

Test article-related findings (direct or indirect) were not present in female dogs given ZS-9 unscreened at 100 mg/kg/dose (ZS-9, ZS-9>5$\mu$m, ZS-9 +>5$\mu$m). An occasional focus or two of minimal tubular regeneration were present in three of the animals without an evidence of mixed leukocyte infiltrate or tubular degeneration/necrosis. Similar focus/foci of tubular regeneration were also present in a control female dog. The foci of tubular regeneration observed in female dogs given lower doses of ZS-9 unscreened were slightly smaller and were not associated with either mixed leukocyte infiltrates or tubular degeneration/necrosis. There was no evidence of crystals in any of the sections examined. Tubular mineralization in the papilla and glomerular lipidosis are background findings in beagle dogs and were not considered test article-related.

ZS-9 unscreened, ZS-9>5$\mu$m, and ZS-9 + >5$\mu$m at the 600 mg/kg/dose had minimal to mild mixed leukocyte infiltrates in the kidney sometimes associated with minimal to mild renal tubular regeneration, and occasionally minimal renal tubular degeneration/necrosis, minimal mixed leukocyte infiltrates in ureter and /or urethra and minimal pyelitis in dogs dosed with ZS-9 unscreened and ZS-9>5$\mu$m.

The lack of increased urinary pH in dogs treated with 600 mg/kg/dose ZS-9 + >5$\mu$m coupled with the reduced incidence of microscopic findings in these dogs and dogs treated with 600 mg/kg/dose ZS-9 unscreened supplemented with potassium suggest that elevated urinary pH and/or removal of potassium due to the pharmacological action of the test article, may have increased susceptibility to the background insult from urinary crystals and bacteria.

Based on these results, the no-observable-effect-level (NOEL) was 100 mg/kg/dose ZS-9 unscreened, ZS-9>5$\mu$m, and ZS-9 + >5$\mu$m. The no-observable-adverse-effect-level (NOAEL) was established for ZS-9 unscreened at 600 mg/kg/dose, screened ZS-9 (ZS-9>5$\mu$m) at 600 mg/kg/dose, and screened and protonated ZS-9 (ZS-9 + >5$\mu$m) at 600 mg/kg/dose.

## EXAMPLE 12

ZS-9 crystals were prepared by reaction in a standard 5-G crystallization vessel.

The reactants were prepared as follows. A 22-L Morton flask was equipped with an overhead stirrer, thermocouple, and an equilibrated addition funnel. The flask was charged with deionized water (3.25 L). Stirring was initiated at approximately 100 rpm and sodium hydroxide (1091 g NaOH) was added to the flask. The flask contents exothermed as the sodium hydroxide dissolved. The solution was stirred and cooled to less than 34 °C. Sodium silicate solution (5672.7 g) was added. To this solution was added zirconium acetate solution (3309.5 g) over 43 minutes. The resulting suspension was stirred for another 22 minutes. Seed crystals of ZS-9 (223.8 g) were added to the reaction vessel and stirred for approximately 17 minutes.

The mixture was transferred to a 5-G Parr pressure vessel with the aid of deionized water (0.5 L). The vessel had smooth walls and a standard agitator. The reactor did not have a cooling coil present. The vessel was sealed and the reaction mixture was stirred at approximately 275-325 rpm and heated to 185 +/- 10 °C over 4 hours, then held at 184-186 °C and soaked for 72 hours. Finally, the reactants were then cooled to 80 °C over 12.6 hours. The resulting white solid was filtered with the aid of deionized water (18L). The solids were washed with deionized water (125 L) until the pH of the eluting filtrate was less than 11 (9.73). The wet cake was dried *in vacuo* (25 inches Hg) for 48 hours at 95-105 °C to give 2577.9 g (107.1%) of ZS-9 as a white solid.

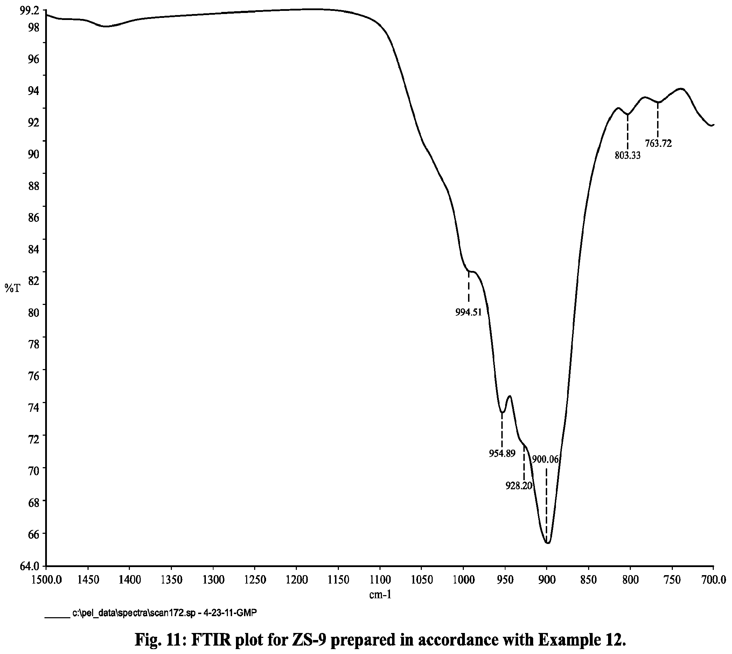

The XRD plot of the ZS-9 obtained in this example is shown in Fig. 10. The FTIR plot of this material is shown in Fig. 11. These XRD and FTIR spectra are characterized by the presence of absorption peaks typically associated with the ZS-11 crystalline form. In addition, the peaks that are associated with ZS-9 exhibit significant spreading due to crystal impurities (*e.g.* the presence of ZS-11 crystals in a ZS-9 composition). For example, the FTIR spectra shows significant absorption around 764 and 955 cm$^{-1}$. The XRD plot for this example exhibits significant noise and poorly defined peaks at 2-theta values of 7.5, 32, and 42.5.

## EXAMPLE 13

In this example ZS-9 crystals were protonated.

To a 100 L reaction vessel deionized water is charged (15.1 L) with vacuum and agitation (60-100 rpm). ZS-9 crystals (2.7 kg) were added to the 100 L vessel containing deionized water and allowed to reaction for a period of 5-10 minutes. Initial pH readings were recorded.

In a separate 50 L carboy, a hydrochloric acid solution is prepared comprising the steps of charging the carboy with deionized water (48 L) followed by hydrochloric acid (600 ml). To the 100 L reaction vessel, the hydrochloric acid solution is charged over a period of 1.5-2 hours. Hydrochloric acid solution was added to the reaction mixture until the pH reached a range of approximately 4.45-4.55. The reaction mixture was continually mixed for an additional period of 30-45 minutes. If the pH was greater than 4.7, additional hydrochloride solution was added until the pH was in the range of approximately 4.45-4.55. The reaction was allowed to stir for an additional 15-30 minutes.

The protonated ZS-9 crystals were filtered through Buchner funnel fitted with a 2 micron stainless steel mesh screen of approximately 18 inches in diameter. The filter cake formed was rinsed three times with approximately 6 L of deionized water to remove any excess hydrochloric acid. The filter cake containing the protonated crystals were dried in an vacuum oven at approximately 95-105 °C for a period of 12-24 hours. Drying was continued until the percent difference in net weight loss is less than 2% over greater than a 2 hour period. Once the product achieved appropriate dryness, the crystals were samples for quality.

## EXAMPLE 14

High capacity ZS-9 crystals were prepared in accordance with the following representative example.

The reactants were prepared as follows. A 22-L Morton flask was equipped with an overhead stirrer, thermocouple, and an equilibrated addition funnel. The flask was charged with deionized water (8,600 g, 477.37 moles). Stirring was initiated at approximately 145-150 rpm and sodium hydroxide (661.0 g, 16.53 moles NaOH, 8.26 moles Na20) was added to the flask. The flask contents exothermed from 24 °C to 40 °C over a period of 3 minutes as the sodium hydroxide dissolved. The solution was stirred for an hour to allow the initial exotherm to subside. Sodium silicate solution (5,017 g, 22.53 mole SO2, 8.67 moles Na20) was added. To this solution, by means of the addition funnel, was added zirconium acetate solution (2,080 g, 3.76 moles Zr02) over 30 min. The resulting suspension was stirred for an additional 30 min.

The mixture was transferred to a 5-G Parr pressure vessel Model 4555 with the aid of deionized water (500g, 27.75 moles). The reactor was fitted with a cooling coil having a serpentine configuration to provide a baffle-like structure within the reactor adjacent the agitator. The cooling coil was not charged with heat exchange fluid as it was being used in this reaction merely to provide a baffle-like structure adjacent the agitator.

The vessel was sealed and the reaction mixture was stirred at approximately 230-235 rpm and heated from 21 °C to 140-145 °Cover 7.5 hours and held at 140-145 °C for 10.5 hours, then heated to 210-215 °C over 6.5 hours where the maximum pressure of 295-300 psi was obtained, then held at 210-215 °C for 4 1.5 hours. Subsequently, the reactor was cooled to 45 °C over a period of 4.5 hours. The resulting white solid was filtered with the aid of deionized water (1.0 KG). The solids were washed with deionized water (40 L) until the pH of the eluting filtrate was less than 11 (10.54). A representative portion of the wet cake was dried in vacuo (25 inches Hg) overnight at 100 °C to give 1,376 g (87.1%) of ZS-9 as a white solid.

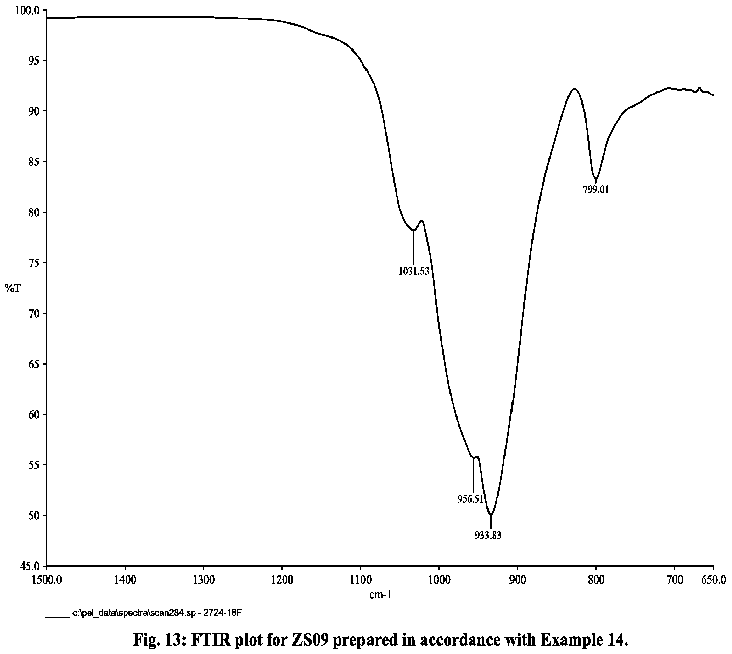

The XRD plot of the ZS-9 obtained is shown in Fig. 12. The FTIR plot of this material is shown in Fig. 13. These XRD and FTIR spectra, when compared to those for Example 12 (Figs. 10-11), exhibited well-delineated peaks without spreading and the absence of peaks associated with crystalline forms other than ZS-9 (e.g., ZS-11 peaks). This example illustrates how the presence of a baffle-like structure within the reactor drastically and unexpectedly improves the quality of the thus obtained crystals. Although not wishing to be bound by theory, the inventors understand that baffles provide added turbulence which lifts the solids (i.e., crystals) and results in a more even suspension of crystals within the reaction vessel while the reaction is ongoing. This improved suspension allows for more complete reaction to the desired crystalline form and reduces the presence of unwanted crystalline forms of ZS in the end product.

## EXAMPLE 15

The KEC of ZS (ZS-9) was determined according to the following protocol.

This test method used a HPLC capable of gradient solvent introduction and cation exchange detection. The column was an IonPac CS12A, Analytical (2 x 250 mm). The flow rate was 0.5 mL/minute with a run time of approximately 8 minutes. The column temperature was set to 35 °C. The injection volume was 10 $\mu$L and the needle wash was 250 $\mu$L. The pump was operated in Isocratic mode and the solvent was DI water.

A stock standard was prepared by accurately weighing and recording the weight of about 383 mg of potassium chloride (ACS grade), which was transferred into a 100-mL plastic volumetric flask. The material was dissolved and diluted to volume with diluent followed by mixing. The stock standard had a $K^+$ concentration of 2000 ppm (2mg/mL). Samples were prepared by accurately weighing, recording, and transferring about 112 mg of ZS-9 into a 20 mL plastic vial. 20.0 mL of the 2000 ppm potassium stock standard solution was pipetted into the vial and the container was closed. The sample vials were placed onto a wrist action shaker and were shook for at least 2 hours but not more than 4 hours. The sample preparation solution was filtered through a 0.45 pm PTFE filter into a plastic container. 750 pL of the sample solution was transferred into a 100-mL plastic volumetric flask. The sample was diluted to volume with DI water and mixed. The initial K$^+$ concentration was 15 ppm (1 SpgImL).

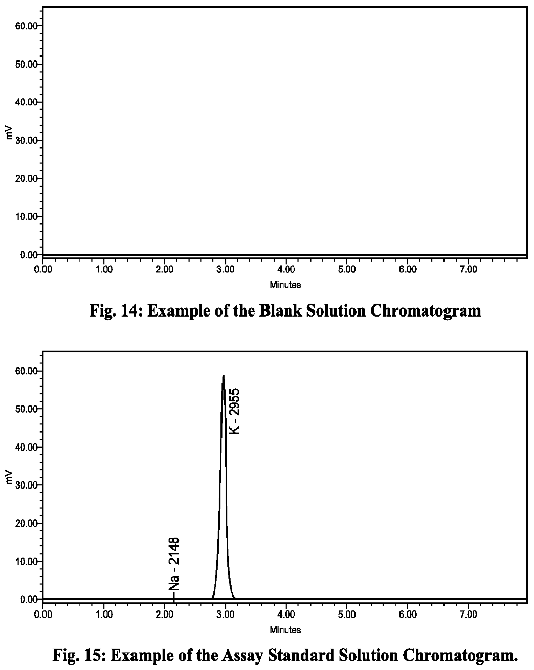

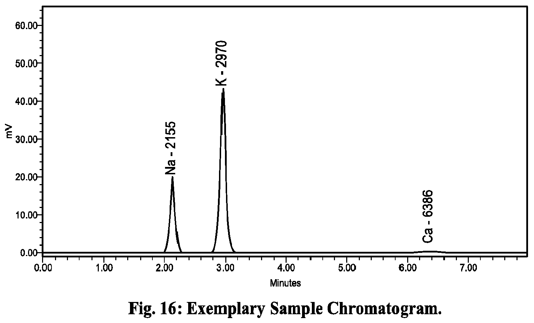

The samples were injected into the HPLC. Fig. 14 shows an example of the blank solution chromatogram. Fig. 15 shows an example of the assay standard solution chromatogram. Fig. 16 shows an exemplary sample chromatogram. The potassium exchange capacity was calculated using the following formula:

$$KEC = \frac{\frac{(IC - FC) \times V}{Eq\,wt.}}{Wt_{spl} \times \frac{(100\% - \%Water)}{100\%} \times \frac{1g}{1000mg}}$$

KEC is the potassium exchange capacity in mEq/g. The initial concentration of potassium (ppm) is IC. The final concentration of potassium (ppm) is FC. The equivalent weight (atomic weight/valence) is Eq wt. The volume (L) of standard in sample preparation is V. The weight of ZS-9 (mg) used for sample preparation is $Wt_{spl}$. The percent (%) of water content (LOD) is % water.

Three samples of ZS-9 produced in accordance with the procedures of Example 12, i.e., in a reactor without baffles (e.g., internal cooling coil structure), were tested for potassium exchange capacity (KEC) in accordance with the above-referenced procedure. Likewise, three samples of ZS-9 produced in accordance with Example 14 in a reactor having cooling coils serving as baffles were tested in accordance with this procedure. The results in Table 3 below show that the procedure of Example 14 and the presence of baffles within the crystallization vessel resulted in a dramatic increase in the potassium exchange capacity.

| Table 3 Potassium Exchange Capacity (KEC) | | | |
|---|---|---|---|
| Example 12 (Without baffles) | | Example 14 (With baffles) | |
| Lot 5368-10311A | 2.3 meq/gm | Lot 2724-9A | 3.9 meq/gm |
| Lot 5368-12211A | 1.7 meq/gm | Lot 2724-13D | 3.8 meq/gm |
| Lot 5368-13811A | 1.8 meq/gm | Lot 2724-18F | 3.8 meq/gm |

The high capacity ZS prepared in accordance with Example 14 will, upon protonation using the techniques of Example 13, have a slightly lower potassium exchange capacity. The protonated ZS prepared in this way has been found to have a potassium exchange capacity of about 3.2 meq/g. Accordingly, the high capacity ZS has been found to increase the capacity of the protonated form prepared using this process. This demonstrates that protonated ZS can be prepared having a potassium exchange capacity within the range of 2.8 to 3.5 meq/g, more preferably within the range of 3.05 and 3.35 meq/g, and most preferably about 3.2 meq/g.

**EXAMPLE 16**

The use of an internal cooling coil to provide a baffle-like structure within the reactor is only feasible for small reactors on the order of 5-gallons because larger reactors cannot be easily fitted with, and typically do not utilized, cooling coils.

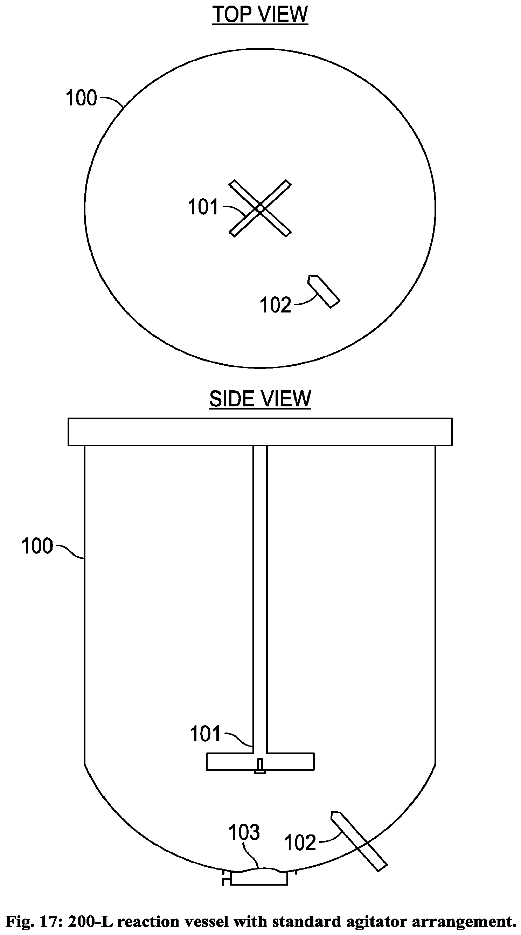

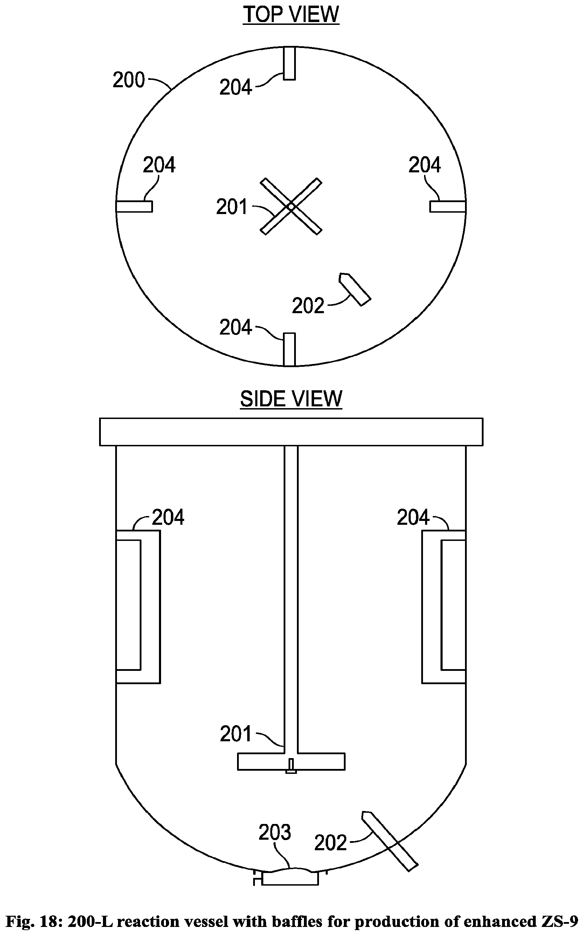

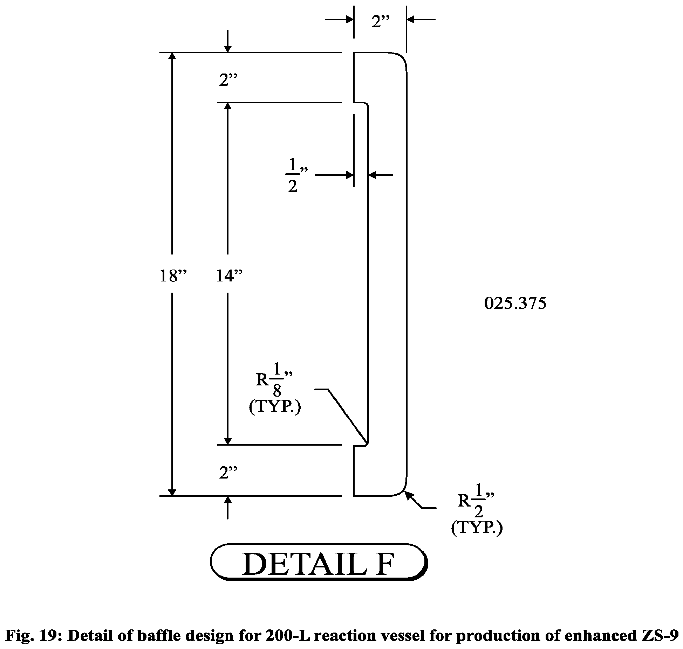

The inventors have designed a reactor for larger-scale production of high purity, high-KEC ZS-9 crystals. Large-scale reactors typically utilize a jacket for achieving heat transfer to the reaction chamber rather than coils suspended within the reaction chamber. A conventional 200-L reactor 100 is shown in Fig. 17. The reactor 100 has smooth walls and an agitator 101 extending into the center of the reaction chamber. The reactor 100 also has a thermowell 102 and a bottom outlet valve 103. The inventors have designed an improved reactor 200, Fig. 18, which also has an agitator 201, thermowell 202, and bottom outlet valve 203. The improved reactor 200 has baffle structures 204 on its sidewalls, which in combination with the agitator 201 provide significant lift and suspension of the crystals during reaction and the creation of high purity, high KEC ZS-9 crystals. The improved reactor can also include a cooling or heating jacket for controlling the reaction temperature during crystallization in addition to the baffle structures 204. The details of an exemplary and non-limiting baffle design is shown in Fig. 19. Preferably the reactor has a volume of at least 20-L, more preferably 200-L or more, or within the range of 200-L to 30,000-L. In an alternative embodiment, the baffle design may be configured to extend the

**EXAMPLE 17**

The several dosages of ZS-9 were studied in the treatment of human subjects suffering from hyperkalemia. A total of 90 subjects were enrolled in the study. The study involved three stages with dose escalation of the ZS in each stage. The ZS-9 used in these studies was prepared in accordance with Example 12. The ZS-9 crystals of an appropriate size distribution were obtained by air fractionation to have a distribution of crystals where greater than or equal to 97% are larger than 3 microns. The screening is such that the ZS crystals exhibit a median particle size of greater than 3 microns and less than 7% of the particles in the composition have a diameter less than 3 microns. The ZS-9 crystals were determined to have a KEC of approximately 2.3 meq/g. The protonation is such that the ZS crystals exhibit a sodium content below 12% by weight. The study utilized 3g silicified microcrystaline cellulose, which are indistinguishable from ZS as the placebo.

Each patient in the study received either a 3 g dose of either the placebo or ZS three times daily with meals. Both ZS and Placebo were administered as a powder in water suspension that was consumed during meals. Each stage of the study had a 2:1 ratio between the number of subjects in the ZS cohort and placebo. In stage I, 18 patients were randomized to receive three daily doses of 0.3 g ZS or placebo with meals. In Stage II, 36 patients were randomized to receive three daily doses of 3 g ZS or placebo with meals. In Stage III, 36 patients were randomized to receive three daily doses of 10 g ZS placebo with meals. Altogether there were 30 patients that received placebo and 60 patients that received various doses of ZS. Diet was essentially unrestricted, and patients were allowed to choose which food items they wished from a variety of local restaurants or the standard in-house diet of the clinic.

The screening value for potassium ("K") was established on day 0 by measuring serum K three times at 30-minute intervals and calculating the mean (time 0, 30 and 60 minutes). The baseline K level was calculated as the mean of these values and the serum K on day one just before ingestion of the first dose. If the screening K value was less than 5.0 meq/l the subject was not included in the study.

On study Days 1-2, all subjects received the study drug 3 times daily in conjunction with meals starting at breakfast (there was a delay of the first meal until 1.5 hours after the first dose on Day 1). Serum K levels were evaluated 4 hours after each dose for 48 hours following the initiation of treatment. If K levels became normal, the subject was discharged from the clinic at 48 hours without further study drug treatment. If K levels were still elevated (K > 5.0 meq/l), subjects received another 24 hours of study drug treatment and then were re-assessed and discharged at 72 hours or 96 hours. All subjects received a minimum of 48 hours of study drug treatment, but a few received up to 96 hours of study drug treatment. The primary efficacy endpoint of the study was the difference in the rate of change in potassium levels during the initial 48 hours of study drug treatment between the placebo treated subjects and the ZS treated subjects. Table 4 provides the p-values of the various cohorts at the 24 and 48 hour endpoints. Patients receiving 300 mg of the ZS three times daily had no statistical difference relative to placebo at either of the 24 and 48 hour endpoints. Patients receiving 3 grams of ZS demonstrated a statistical difference at only the 48 hour time period, suggesting that this particular dosing was relatively effective at lowering serum potassium levels. Unexpectedly, those patients receiving 10 grams of ZS three times daily demonstrated the greatest reduction in potassium levels in both concentration and in rate. The decrease in potassium was considerable in magnitude, with an approximate 0.5 meq/g reduction at the 3 gram dose and approximately 0.5-1 meq/g reduction at the 10 gram dosing.

| Table 4: Primary endpoint: Serum potassium (mmol/l) exponential rate of change from 24 hours and 48 hours Intent-to-Treat Population (Primary endpoint at 48 hours) | | | |
|---|---|---|---|
| | Cohort 1 300 mg tid *p*-value | Cohort 2 3 g tid *p*-value | Cohort 3 10 g tid *p*-value |
| **24 hours** | 0.7668 | 0.0737 | 0.1301 |
| **48 hours** | 0.4203 | 0.0480 | <0.0001 |

Subjects were then followed for a total of 7 days (168 hours) with K measurements performed daily. 24 hour urine collections were performed on the day before the study (day 0) in all patients, and for as long as the patients ingested the test product. Table 5 provides the difference in the rate of change in serum potassium levels over 7 days of study between placebo treated subjects and the various cohorts. Patients receiving 300 mg of the drug had no statistically significant reduction in potassium levels relative to the placebo over the 7 day period. Patients receiving 3 grams of the drug had no statistically significant reductions in potassium levels after the initial 24 hour period . Patients receiving 3 grams of the drug had the most statistically significant reduction in serum potassium levels over the 7 day time course. These data suggests that when given at least 10 grams of ZS, an extended reduction of potassium is achieved, and that a single (*i.e.*, 1 day) dose is suitable for significant reduction in potassium levels. It is also possible that dosages of 3,4, or 5 grams may be effective at reducing the potassium levels when given once daily.

| Table 5: Serum Potassium (mmol/l) over time in intent-to-treat population | | | |
|---|---|---|---|
| | Cohort 1 300 mg tid Unpaired t-test p-value | Cohort 2 3 gm tid Unpaired t-test p-value | Cohort 3 10 gm tid Unpaired t-test p-value |
| Baseline | | | |
| Day 1-30 Min Post 1st | 0.566 | 0.604 | 0.356 |
| Day 1-1 Hr Post 1st | 0.875 | 0.125 | 0.022 |
| Day 1-2 Hr Post 1st (Fed Breakfast) | 0.231 | 0.688 | 0.160 |
| Day 1-4 Hr Post 1st (Fed Lunch) | 0.640 | 0.774 | 0.232 |
| Day 1-4 Hr Post 2nd | 0.219 | 0.415 | 0.072 |
| Day 1-4 Hr Post 3rd | 0.603 | 0.365 | 0.025 |
| Day 2-0 Hr | 0.700 | 0.026 | 0.092 |
| Day 2- 4 Hr Post 1st | 0.675 | 0.136 | <0.001 |
| Day 2- 4 Hr Post 2nd | 0.891 | 0.044 | <0.001 |
| Day 2- 4Hr Post 3rd | 0.783 | 0.064 | <0.001 |
| Day 2-20Hr Post 1st | 0.822 | 0.157 | <0.001 |
| Day 3-0 Hr | 0.914 | 0.074 | <0.001 |
| Day 4-0 Hr | 0.756 | 0.775 | <0.001 |
| Day 5-0 Hr | 0.404 | 0.595 | 0.001 |
| Day 6-0 Hr | 0.717 | 0.321 | 0.016 |

| Day 7-0 Hr | 0.217 | 0.476 | 0.065 |

Comparison of treatment groups demonstrated no significant difference in any parameters including: age, sex, weight, serum creatinine level, estimated Glomerular filtration rate ("GFR"), potassium levels, and cause of Chronic Kidney Disease ("CKD").

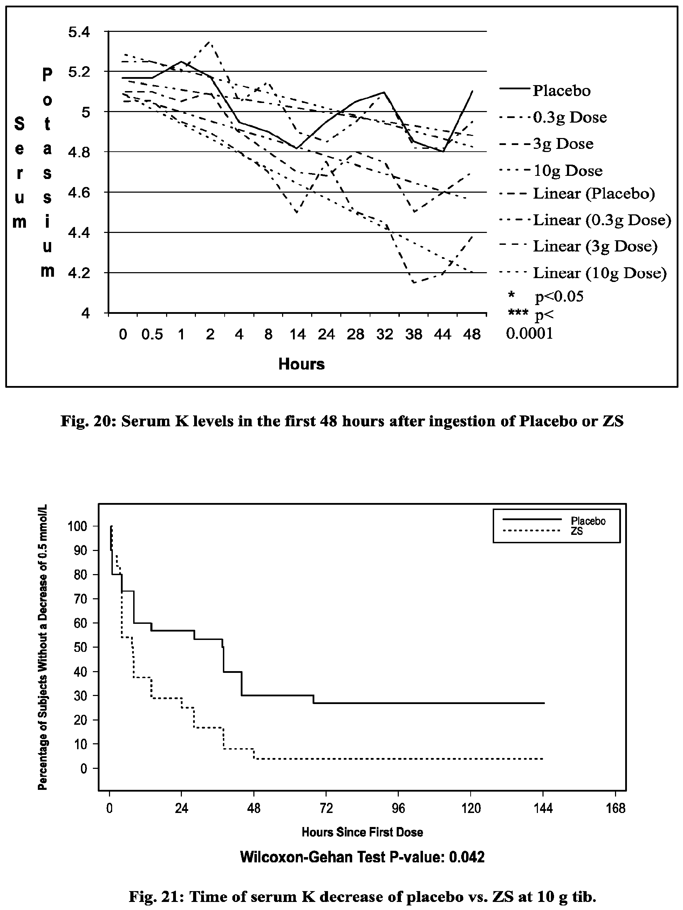

Figure 20 shows changes in serum K in the first 48 hours after ingestion of the placebo, ZS at 0.3 g per dose (Cohort 1), ZS at 3 g per dose (Cohort 2) and ZS at 10 g per dose (Cohort 3). Slopes of K versus time for the patients administered ZS were significantly different from the placebo for Cohort 2 (0.5 meq/L/48 hours, P<0.05) and Cohort 3 (1 meq/L/48 hours P<0.0001).

The time to normalization of serum K was significantly less in Cohort 3 versus the placebo group (P=0.040). Results for the other Cohort groups were not significantly different from placebo. Figure 21 compares the time to decrease of serum K by 0.5 meq/L for subjects administered ZS at the 10 g doses versus placebo. Time to decrease in serum K was significantly shorter in ZS administered subjects than in placebo (P=0.042).

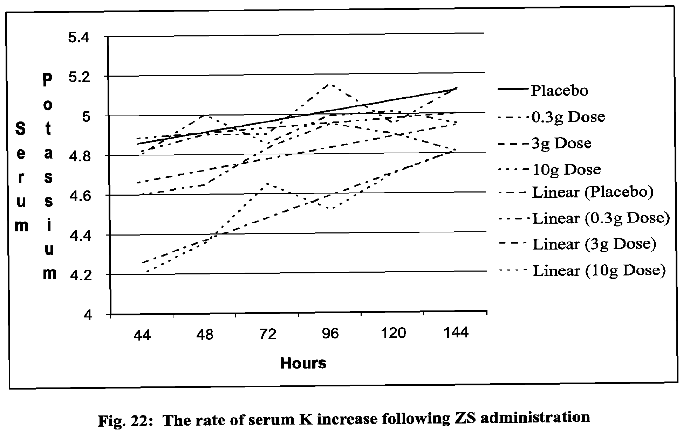

The increase in serum K from 48 hours to 144 hours of the study was also examined after discontinuing the administration of the study drug. The rate of increase in serum K was roughly proportional to the rate of decrease in serum K during ingestion of the drug, as shown in Figure 22.

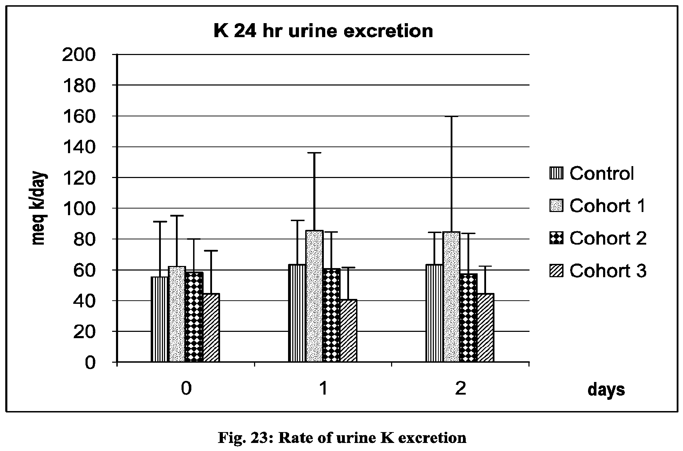

Analysis of 24 hour urine K excretion demonstrated that there was a significant (P<0.002) decrease of approximately 20 meq/day in urinary K excretion for ZS at the 10 g dose, while excretion remained the same or increased in all other groups as shown in Figure 23.

Analysis of the K/creatinine ratio in daily urine samples confirmed the same trends as in 24 hour urine K excretion. Cohort 3 had a downward trend in urinary K/creatinine ratio while the other Cohorts remained constant or increased. Separate analysis indicated no change in creatinine clearance or daily creatinine excretion in any of the groups during the study.

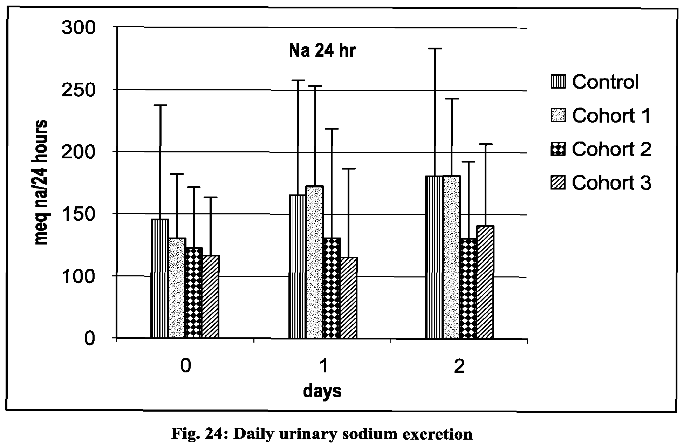

Analysis of the 24 hour urine samples also allowed calculation of the urinary daily sodium excretion. As shown in Figure 24, sodium excretion was generally stable in all of the groups. Urinary sodium excretion appeared to rise more in Cohort 1 and Control patients than in Cohort 3 though there were no significant changes in any group.

Blood Urea Nitrogen ("BUN") was tested as a measure of the effect of ZS to bind ammonium which is generated by bacterial urease in the gut. There was a dose-related and statistically significant reduction in BUN from Study Day 2 to Study Day 7, mirroring that of serum K (p-values between 0.035 [Study Day 2] and <0.001 [Study Days 5-7]). This was also accompanied by a reduction in urine excretion of urea.

There was a statistically significant decrease in serum calcium that remained within the normal range (from 9.5mg/dL to 9.05mg/dL) at the 10 g three times daily dose of ZS (p-values from 0.047 to 0.001 on Study Days 2-6, but no subjects developed hypocalcemia; there were no significant changes in serum magnesium, serum sodium, serum bicarbonate or any other electrolytes at any dose level of ZS. There was a trend towards a reduction in serum creatinine, which became statistically significant on Study Day 6 (p=0.048). There were no dose-related changes in any other evaluated kidney parameters, including urinary sediment, estimated Glomerular filtration rate ("GFR") or the renal biomarkers NGAL and KIM-1.

This clinical trial, which was randomized and double-blind, demonstrates that ingestion of moderate amounts of ZS significantly decreases serum K levels in patients with Stage 3 CKD. No laxative agents were given with the ZS, so the removal of K was solely due to the binding of K in the gut by ZS, rather than due to effects of diarrhea.

Oral sodium polystyrene sulfonate ("SPS") therapy invariably causes sodium load to the patient. Sodium is released in 1:1 ratio of the binding of all cations (K, hydrogen, calcium, magnesium, etc.). ZS is loaded partly with sodium and partly with hydrogen, to produce a near physiologic pH (7 to 8). At this starting pH, there is little release of sodium and a some absorption of hydrogen during binding of K. Urinary excretion of sodium does not increase during ingestion of ZS and thus ZS use should not contribute to sodium excess in patients.

The rapidity of action of ZS on serum K and the effectiveness in diminishing K excretion in the urine is surprising at the maximum dose of about 10 g three times daily (about 30 g daily or about 0.4 g/kg/day). This also resulted in a fall in urinary K by the second day of about 40% from the baseline level. It thus appears that ZS is at least as effective in diminishing body K stores in humans as in animals, and possibly more so due to the high K concentration in human stool.

## EXAMPLE 18

High capacity ZS (ZS-9) is prepared in accordance with Example 14. The material is protonated in accordance with the techniques described in Example 13. The material has been screened such that the ZS crystals exhibit a median particle size of greater than 3 microns and less than 7% of the particles in the composition have a diameter less than 3 microns. The ZS crystals exhibit a sodium content below 12% by weight. The dosage form is prepared for administration to patients at a level of 5g, 10g, and 15g per meal. The ZS in this example has an increased potassium exchange capacity of greater than 2.8. In a preferred aspect, the potassium exchange capacity is within the range of 2.8 to 3.5 meq/g, more preferably within the range of 3.05 and 3.35 meq/g, and most preferably about 3.2 meq/g. A potassium exchange capacity target of about 3.2 meq/g includes minor fluctuations in measured potassium exchange capacity that are expected between different batches of ZS crystals.

The ZS-9, when administered according to the protocol established in Example 17, will provide for a similar reduction in potassium serum levels. Because ZS-9 has an improved KEC, the dosing administered to the subject in need thereof will be lowered to account for the increased cation exchange capacity. Thus, to patients suffering from potassium levels elevated above the normal range, approximately 1.25, 2.5, 5, and 10 grams of the ZS-9 will be administered three times daily.

## EXAMPLE 19

ZS (ZS-2) is prepared in accordance with known techniques of U.S. Patent Nos. 6,814,871, 5,891,417, and 5,888,472, discussed above. The x-ray diffraction pattern for the ZS-2 has the following characteristics d-spacing ranges and intensities:

| Table 6 - ZS-2 | |
|---|---|
| d(Å) | I |
| 5.8-6.6 | m |
| 4.2-5.0 | w |
| 3.9-4.6 | m |
| 2.9-3.7 | m |
| 2.5-3.3 | vs |
| 2.3-3.0 | s |

In one aspect of this example, the ZS-2 crystals are prepared using the reactor with baffles described in Example 14. The material is protonated in accordance with the techniques described in Example 13. The material has been screened such that the ZS crystals exhibit a median particle size of greater than 3 microns and less than 7% of the particles in the composition have a diameter less than 3 microns. The ZS crystals exhibit a sodium content below 12% by weight. The dosage form is prepared for administration to patients at a level of 5g, 10g, and 15g per meal. The ZS-2 crystals prepared in accordance with this example are beneficial for reducing serum potassium and can be manufactured using the alternative techniques for making ZS-2. These alternative manufacturing techniques may provide advantages under certain circumstances.

## EXAMPLE 20

Several batches of protonated ZS crystals were prepared using the reactor described in Example 16.
The batches of the ZS crystals were generally prepared in accordance with the following representative example.
The reactants were prepared as follows. To a 200-L reactor, as shown in Fig. 17, sodium silicate (56.15 kg) was added and charged with deionized water (101.18 kg). Sodium hydroxide (7.36 kg) was added to the reactor and allowed to dissolve in the reactor in the presence of rapid stirring over a period of greater than 10 minutes until there was complete dissolution of the sodium hydroxide. Zirconium acetate (23 kg) was added to the reactor in the presence of continuous stirring and allowed to stir over a period of 30 minutes. The reactants were mixed at a rate 150 rpm with the reactor set to 210°C $\pm$ 5°C for a period of $\geq$ 60 hours.
After the reaction period, the reactor was cooled to 60 °C -80 °C and the slurry of reactants were filtered, washed and dried over a period of $\geq$ 4 hours at a temperature of approximately 100 °C. To prepare the dried crystals for protonation, deionized water (46 L) was charged to re-slurry the crystals. A solution of 15% HCl (approximately 5 to 7 kg of the 15% HCl solution) was mixed with the slurry for a period of 25 to 35 minutes. Following the protonation reaction, the reactants were once again filter dried and washed with approximately $\geq$ 75 L of deionized water.
Exemplary details of several protonated ZS crystal batches produced utilizing the above described procedure are presented in Table 7:

| Table 7 | | | | |
|---|---|---|---|---|
| Lot Number | 5602-26812-A | 5602-28312-A | 5602-29112-A | 5602-29812-A |
| Yield (kg) | 16.60 | 16.65 | 16.61 | 16.14 |
| % Theoretical Yield | 95 | 94.5 | 94.7 | 92.2 |
| IP KEC | 3.35 | 2.9 | 2.46 | 2.92 |
| XRD highest | 28.9 | 28.9 | 28.9 | 28.9 |
| XRD 2nd highest | 15.5 | 15.5 | 15.5 | 15.5 |
| XRD 3rd highest | 26.2 : 13.9 | 26.1 : 13.9 | 26.2 :26.2 | 26.2 : 26.2 |
| pH | 8.3 | 8.7 | 8.6 | 8.9 |
| % < 3um (2.50) | 0.4 | 1.27 | 1.52 | 3.08 |
| % < 3um (3.00) | 1.69 | 2.77 | 2.8 | 6.37 |
| Mean D(4,3) | 10.6 | 12.5 | 12.8 | 10.1 |
| KEC | 3.1 | 3.0 | 2.94 | 3.04 |

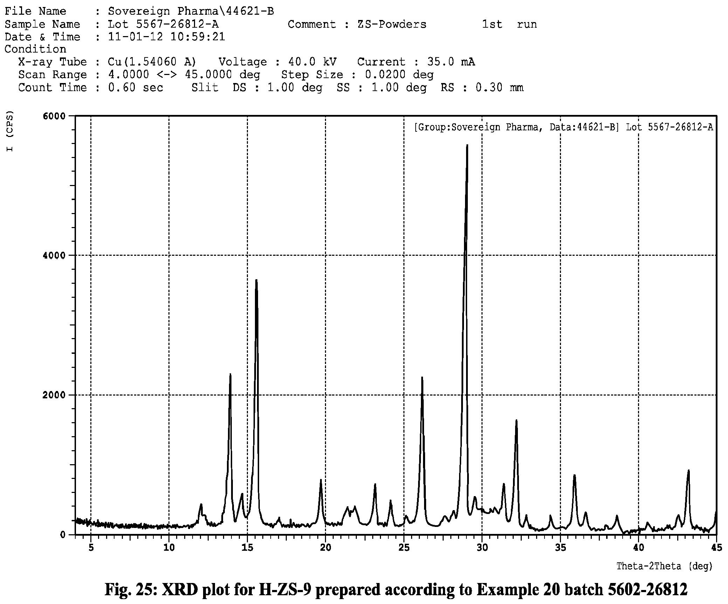

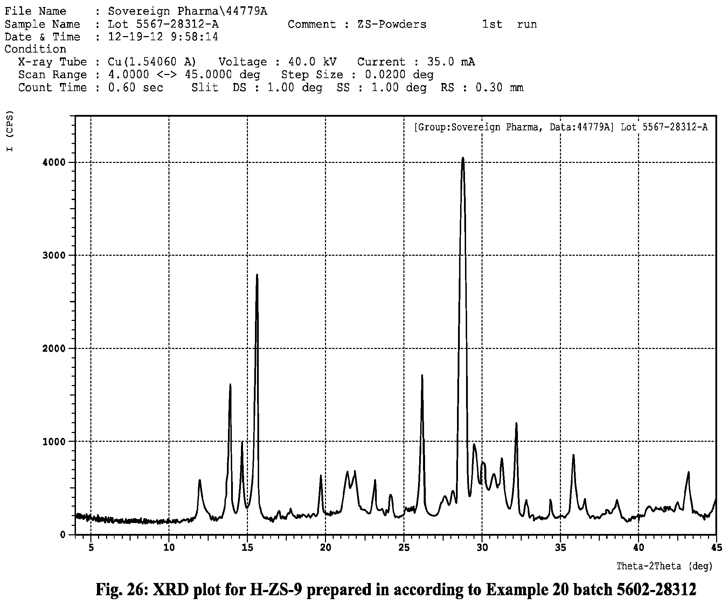

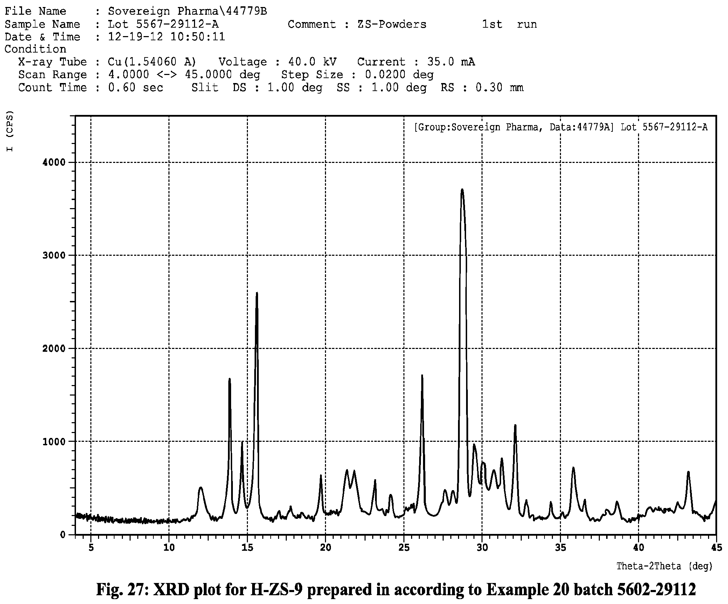

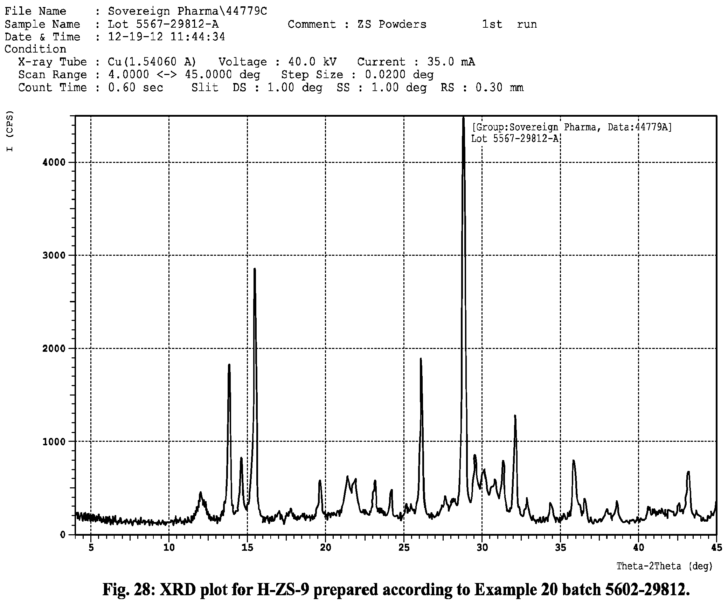

The XRD plot of the H-ZS-9 obtained above are provided in Figs. 25-28. The XRD plots demonstrate that H-ZS-9 can be manufactured in commercially significant batch quantities having desired potassium exchange capacity. Lot 5602-26812-A attained the most uniform crystalline distribution. It was found that when crystallization conditions result in a highly uniform particle size distribution, the subsequent protonation step reduced the cation exchange capacity from 3.4 to 3.1 meq/g. In contrast, Lots 5602-28312-A, 5602-29112-A, and 5602-29812-A exhibited a less uniform particle size distribution. The less uniform particle size distribution resulted from increasing the fill ratio of the reactor. When fill ratios reached 80-90%, the particle size distributions became less uniform. Unexpectedly, however, the subsequent protonation of these lots resulted in a significant increase in the potassium exchange capacity. Because the reaction according to the invention can be run in a manner that increases potassium exchange capacity upon protonation, it is expected that higher capacity ZS-9 can be obtained in commercially significant quantities than otherwise would have been thought possible.

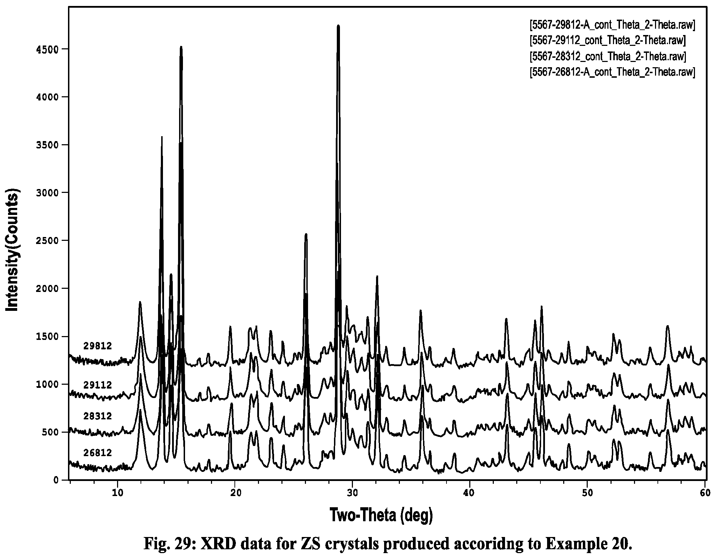

Phase quantification to determine the diffraction pattern of the various batches of protonated ZS crystal samples were also performed using the Rietveld method in a Rigaku MiniFlex600. Manufacturing procedures using the 200-L reactor produced the phase composition described in Table 8 and XRD data described in Figs. 25-29.

| Table 8 Phase Composition (wt%) via Reitveld Analysis | | | | |
|---|---|---|---|---|
| Lot Number | ZS-9 | ZS-7 | ZS-8 | Amorphous Crystals |
| 5567-26812-A | 61.6 | 16.0 | | 22.3 |
| 5567-28312-A | 55.7 | 21.8 | | 22.5 |
| 5567-29112-A | 55.7 | 25.7 | | 18.6 |
| 5567-29812-A | 66.6 | 19.1 | | 14.3 |

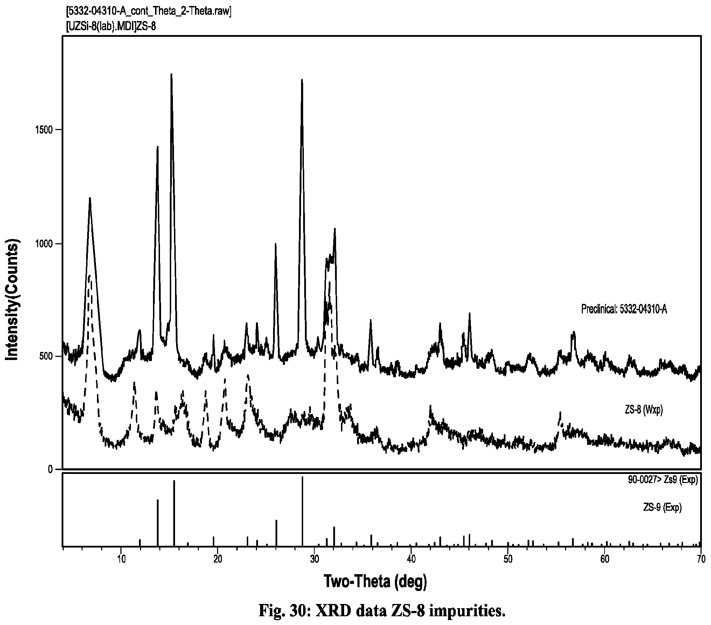

The diffraction patterns for the batches produced provided a mixture of ZS-9 and ZS-7 crystals in additional to a series of amorphous crystals. It was found that ZS crystals made in the larger 200 L reactor according to the above processes resulted in no detectable levels of ZS-8 crystals and lower levels of amorphous material than previously produced. The absence of ZS-8 crystals is highly desirable due to the undesirably higher solubility of ZS-8 crystals and their attendant contribution to elevated levels of zirconium in urine. Specifically, levels of zirconium in the urine are typically around 1 ppb. Administration of zirconium silicate containing ZS-8 impurities has led to zirconium levels in the urine between 5 to 50 ppb. The presence of ZS-8 can be confirmed by XRD as shown in Fig. 30. The ZS-9 crystals according to this embodiment are expected to lower levels of zirconium in the urine by eliminating impurities of soluble ZS-8 and minimizing the amorphous content.

**EXAMPLE 21**

ZS-9 was dried and ground in an agate mortar, then placed into a powder diffractometer. Data were collected at room temperature with monochomated Cu $\alpha_1$ radiation ($\lambda$=1.5406 Å). Rietveld least squares structural refinements were performed, and the interatomic distances were calculated from the resulting atom positions. The size of the pore opening was calculated by subtracting twice the atomic radius of oxygen (van der Waals radius, r=1.52 Å) from center-center interatomic distances. For the thermodynamic stability modeling, the predicted energies for different cation forms of ZS-9 (ie, Na-ZS-9, K-ZS-9, Ca-ZS-9 and Mg-ZS-9) and alkali and alkaline earth oxides from models were used to estimate the cation exchange energies in ZS-9. All energies were computed relative to the $Na^+$ form of ZS-9, defined as the reference state.

The structure of ZS-9 consists of units of octahedrally and tetrahedrally coordinated zirconium and silicon atoms with oxygen atoms acting as bridges between the units, forming an ordered cubic lattice structure. The framework is negatively charged due to the octahedral $[ZrO_6]^{-2}$ units. The pore opening of ZS-9 is composed of an asymmetrical seven-member ring (Figure 34) with an average size of ~3 Å. Thermodynamically, ZS-9 with $K^+$ was calculated to be more stable than ZS-9 with $Na^+$, $Ca^{2+}$, or $Mg^{2+}$. For example, the $K^+$ form of ZS-9 was 20 kcal/mol more stable than the $Na^+$ form.

**EXAMPLE 22**

The batches of protonated zirconium crystals described in Example 20 were used in studies to treat human subjects suffering from hyperkalemia. The ZS compositions were generally characterized as having a mixture of ZS-9 and ZS-7, where the ZS-9 was present at approximately 70% and the ZS-7 was present at approximately 28% (hereafter ZS-9/ZS-7). All of the characterized ZS-9/ZS-7 crystals lack detectable quantities of ZS-8 crystals. Subjects were administered the ZS-9/ZS-7 composition according the method described in Example 17. A summary of the results are provided in Table 9.

| Table 9: Kidney Function Test using the ZS-9/ZS-7 composition | | | | | | | | | |
|---|---|---|---|---|---|---|---|---|---|
| Subject ID | Lab Test | Day 0 | Day 3 | Day 4 | Day 5 | Day 6 | Day 9 | Day 15 | Day 21 |
| 009-006 L-D | BUN | 64.6 | 71.3 | 77.2 | 80.7 | 82.5 | 78.1 | 64.4 | 63.7 |
| | Creat | 2.37 | 2.38 | NA | NA | NA | 2.37 | 2.34 | 2.40 |
| 009-011 CHR | BUN | 28.5 | 27.9 | 31.7 | 28.1 | 28.1 | 22.2 | 32.6 | 36.9 |
| | Creat | 2.31 | 2.27 | NA | NA | NA | 2.21 | 2.32 | 2.54 |
| 009-014 RWR | BUN | 18.6 | 15.6 | 16.1 | 15.6 | 14.4 | 15.6 | 18.5 | 18.9 |
| | Creat | 1.11 | 1.13 | NA | NA | NA | 1.23 | 1.13 | 1.16 |
| 009-017 SMK | BUN | 60.3 | 61.7 | 67.1 | 75.3 | 75.2 | 75.9 | 71.3 | 74.4 |
| | Creat | 2.37 | 2.31 | NA | NA | NA | 2.31 | 2.29 | |
| 009-019 GLS | BUN | 51.4 | 41.9 | 44.8 | ND | 41.4 | 37.7 | 46.6 | |
| | Creat | 3.14 | 2.71 | NA | ND | NA | 2.33 | 2.85 | |
| 009-022 JHR | BUN | 87.3 | 103.3 | 101.6 | ND | 94.6 | 85.3 | 76.4 | 97.8 |
| | Creat | 2.40 | 2.40 | NA | ND | NA | 2.50 | 1.93 | |
| 009-023 EEF | BUN | 42.3 | 39.5 | 36.3 | 39.9 | 36.5 | 37.9 | 37.4 | 33.5 |
| | Creat | 2.50 | 2.48 | NA | NA | NA | 2.22 | 2.44 | 2.39 |
| 009-025 DHK | BUN | 42.4 | 43.1 | 37.9 | ND | 28.2 | 25.9 | 31.3 | |
| | Creat | 2.35 | 2.09 | NA | ND | NA | 1.82 | 2.05 | |
| 009-026 ABL | BUN | 24.3 | 25.5 | 28.5 | ND | 27.1 | 29.1 | 35.4 | |
| | Creat | 2.02 | 2.04 | NA | ND | NA | 1.99 | 1.94 | |
| 009-028 GMS | BUN | 46.9 | 55 | | | | | | |
| | Creat | 4.51 | 4.61 | NA | NA | NA | | | |

Surprisingly, the glomerular filtration rate (GFR) for subjects administered the ZS-9/ZS-7 composition were unexpectedly higher relative to the patient's baseline. Without being bound to any particular theory, the inventors posit that the improved GFRs and lowered creatinine levels (see Table 9 above) are due to absence of the ZS-8 impurities in the ZS-9/ZS-7 composition. As is generally known in the prior art, ZS-8 crystals have been characterized as having a higher solubility and therefore is able to circulate systemically. This, the inventors believe, may be the causes of elevated BUN and creatinine levels upon administration of zirconium crystals described in the prior art.

**EXAMPLE 23**

ZS crystals having greater than or equal to 95% ZS-9 forms were produced in accordance to the following representative example.

The reactants were prepared as follows. To a reactor having a similar design to the one described in Example 16, but having a capacity of 500-L, sodium hydroxide (98.3 kg) was added along with deionized water (85.9 kg). Before the addition of the sodium silicate (110.6 kg) and additional water (10.8 kg), the reactor was agitated at a rate of 150 RPM. The rate of the agitation was increased to 200 RPM and agitated for a period of about 20 minutes, followed by a decrease in agitation to 100 RPM. An additional amount of deionized water (53.7 kg) was added followed by an increase to 200 RPM for a period of 5 minutes followed by a decrease to 150 RPM. Zirconium Silicate (81.0 kg) and deionized water (63.9 kg) was charged to the reactants and allowed to agitate at 150 RPM for a period of 30-40 minutes. Following the 30-40 minute agitation period, the reactants were heated to 210 °C for a period of greater than or equal to 36 hours.

After the reaction period, the reactor was cooled to 60 °C-80 °C and the slurry of the reactants were filtered, washed and dried over a period of greater than or equal to 4 hours at a temperature of approximately 100 °C. To prepare the dried crystals for protonation, deionized water (170 kg) was charged to re-slurry the crystals. A solution of 15% HCl was mixed with the slurry for a period of 25 to 35 minutes. Following the protonation reaction, the reactants were once again filter dried and washed with approximately ≥ 170 kg of deionized water.

The ZS crystals produced using the described procedure resulted in crystals having the following properties:

| Appearance | White free flowing powder essentially free of debris and particles |
|---|---|
| FTIR | Bands at approximately 799 and 927 $cm^{-1}$ |
| XRD | Two highest peaks occurring at 15.5 and 28.9 with the highest peak occurring at 28.9 |
| pH | ~9 pH |
| Particle size distribution | 1% less than 3 microns |
| Potassium exchange capacity | 3.5 mEq/g |

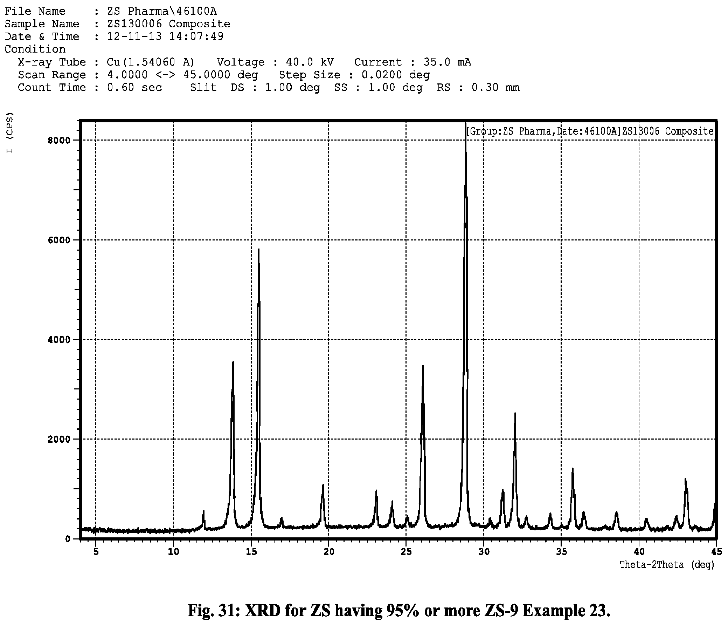

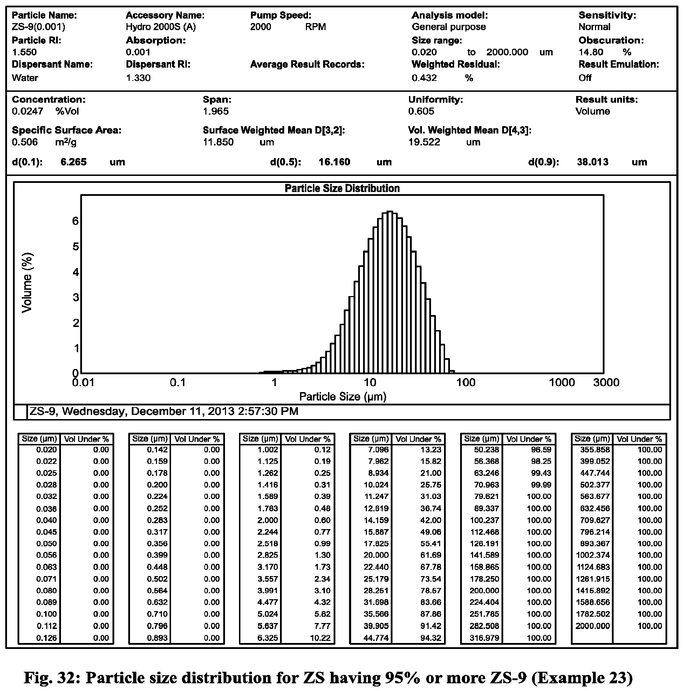

The XRD was performed using a Shimazdu, Lab X, XRD-6000 and operated from 4-45 degree two-theta. The results of the XRD are found in Fig. 31. The XRD spectrums demonstrate that ZS-9 can be manufactured at high purity levels, with improved potassium exchange capacities of greater than 3.2 mEq/g. The results of the particle size distribution were performed and are found in Fig. 32.

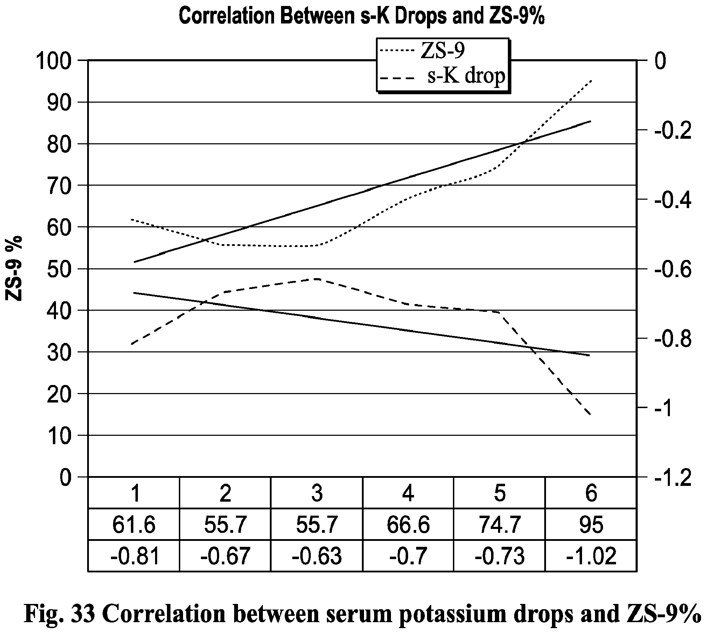

The *in vitro* binding capacity of the ZS made according to this example was compared to that of the material made in accordance with Example 16 above. *See* Fig. 33. Specifically, materials 1-4 in Fig. 33 were identical to materials described in Table 8 above. The data show a significant increase in binding capacity as the percentage of ZS-9 material reached 95%.

The percentage of ZS-9 was evaluated using powder x-ray diffraction. Quantification of ZS9, ZS8, ZS7 and non-crystalline, or amorphous, material was done through Rietveld full pattern refinement using the crystal structure model of ZS9, cubic cell, by Ferreira et al., Inor Chim Acta 2003, 356, 19 (without the occluded NaCl). The model for ZS-7 was the structure of the mineral Petarasite (monoclinic cell). Because there is no structural model for ZS-8, a layered material, exists, it was modeled as non-crystalline or amorphous material. The Rietveld subroutines included in Jade 9.5 software from MDI, Inc. were used for the full pattern refinements. Electron density and variables that could not be exactly modeled are reported as percent Residials (R/E in the reports)

## EXAMPLE 24

This clinical trial demonstrates that ingestion of moderate amounts of ZS-9/ZS-7 surprisingly and unexpectedly decreases creatinine levels in patients.

A total of 750 subjects with mild to moderate hyperkalemia (i-STAT potassium levels between 5.0-6.5 mmol/l, inclusive) will be enrolled in the study where they, in a double-blind fashion, will be randomized 1:1:1:1:1 to receive one of four (4) doses of ZS (1.25g, 2.5g, 5g, and 10g) or placebo control, administered 3 times daily (tid) with meals for the initial 48 hours (Acute Phase), followed by a Subacute Phase (randomized withdrawal) during which subjects treated with active doses in the Acute Phase, who achieve normokalemia ( i-STAT potassium values 3.5 to 4.9 mmol/l, inclusive) will be randomized to 12 days of subacute, once a day (qd) dosing. There will be a one-time randomization to assign the Acute Phase treatment and the Subacute Phase treatment. The Subacute Phase will include subjects who became normokalemic on active drug and those who became normokalemic on placebo. The former will be randomized in a 1:1 ratio between the same dose of ZS they received during the acute phase but only administered once a day (qd) or placebo, qd.

Subjects on placebo during the Acute Phase who are normokalemic in the morning of Study Day 3, will be randomized to receive either 1.25 or 2.5 g ZS, qd as Subacute Phase treatment. Safety and tolerability will be assessed on an ongoing basis by an Independent Data Monitoring Committee (iDMC). Each active dose group will consist of 150 subjects per treatment group including the placebo control group for a total of 750 subjects; the 1:1:1:1:1 allocation helps to optimize the multiple active dose comparisons to the respective placebo controls for the Subacute Phase.

Endpoints:

Acute Phase: The primary efficacy endpoint will be the difference in the exponential rate of change in serum potassium (S-K) levels during the initial 48 hours of study drug treatment between the placebo-treated and ZS-treated subjects. Secondary endpoints will include S-K at all time points, time to normalization of S-K (as defined by S-K levels of 3.5 - 5.0 mmol/l), time to a decrease of 0.5 mmol/l in S-K levels, proportion of subjects who achieve normalization in S-K levels after 48 hours of treatment with ZS or placebo control as well as the type, incidence, timing, severity, relationship, and resolution of all treatment-emergent adverse events.

Subacute Phase (randomized withdrawal): The primary efficacy endpoint in the Subacute Phase will be the difference in the exponential rate of change in S-K levels over the 12 day treatment interval. In addition, the time subjects remain normokalemic (3.5 - 5.0 mmol/l), time to relapse (return to hyperkalemia), and the cumulative number of days between Study Days 3-14 where subjects are normokalemic will also be determined. Another secondary efficacy endpoint will be the proportion of subjects who are normokalemic at the end of the 12-day Subacute Phase (as defined by S-K between 3.5-5.0 mmol/l). Other secondary endpoints will include safety and tolerability as well as other electrolytes, incidence of hospitalization, and need for additional treatments to control S-K levels.

Acute Phase Measurements: Potassium levels will be evaluated prior to the first dose on Study Days 1 and 2, 1, 2, and 4 hours after the first dose on Study Day 1, 1 and 4 hours after the first dose on Study Day 2 and prior to breakfast on Study Day 3, after 48 hours of treatment. The primary efficacy comparison will include all S-K outcomes through the initial 48 hours of assessment.

Subjects who have potassium levels > 6.5 mmol/l (as determined by i-STAT) on Study Day 1 at the 4 hour post Dose 1 timepoint will be withdrawn from the study and will receive standard of care. If potassium is between 6.1 and 6.5 mmol/l (as determined by i-STAT) at the 4 hour post Dose 1 blood draw, subjects will be kept in the clinic for another 90 minutes post Dose 2 and another blood draw will be taken and an ECG will be performed.

If the i-STAT potassium level is ≥ 6.2 mmol/l at this timepoint the subject will be discontinued from the study and standard of care will be instituted. If the i-STAT potassium level is < 6.2 mmol/l, and the ECG does not show any of the ECG withdrawal criteria (see below), the subject will continue in the study. Subjects who achieve potassium levels in the morning of Study Day 3 between 3.5 - 4.9 mmol/l inclusive (as determined by i- STAT) will enter the Subacute Phase where they will receive one of 4 doses of ZS (1.25g, 2.5g, 5.0g, 10.0g) or placebo, as determined by their randomization schedule, administered qd for another 12 days of subacute treatment. Subjects who are either hyperkalemic (i-STAT potassium ≥ 5.0 mmol/l) or hypokalemic (i-STAT potassium <3.5 mmol/l) in the morning of Study Day 3 (including placebo subjects) will be deemed treatment failures, discontinue from the study, and receive standard of care at the discretion and the direction of their own physician. Such subjects will return to the clinic on Study Day 9 (7 days after last dose of ZS) for a final safety follow-up.

Subacute Phase Measurements: For subjects who continue into the Subacute Phase, potassium levels will be evaluated in the morning of Study Days 4-6, 9 and 15. If, at the end of the Subacute Phase, potassium is still elevated (≥5.0 mmol/l, as determined by i-STAT), the subject will be referred to his/her own physician for standard of care treatment.

Number of subjects and number of sites

A total of 750 subjects with mild to moderate hyperkalemia at screening (i-STAT potassium values between 5.0 and 6.5 mmol/l, inclusive) will be enrolled in the study at up to 100 investigational sites throughout the North America, Europe and Australia.

Inclusion criteria

1. Provision of written informed consent.
2. Over 18 years of age.
3. Mean i-STAT potassium values between 5.0 - 6.5 mmol/l inclusive, at screening (Study Day 0).
4. Ability to have repeated blood draws or effective venous catheterization.
5. Women of childbearing potential must be using two forms of medically acceptable contraception (at least one barrier method) and have a negative pregnancy test at screening. Women who are surgically sterile or those who are postmenopausal for at least 2 years are not considered to be of child-bearing potential

Exclusion criteria

1. Pseudohyperkalemia signs and symptoms, such as excessive fist clinching hemolyzed blood specimen, severe leukocytosis or thrombocytosis.
2. Subjects treated with lactulose, xifaxan or other nonabsorbed antibiotics for hyperammonemia within the last 7 days.
3. Subjects treated with resins (such as Sevelamer acetate or Sodium polystyrene sulfonate [SPS; e.g. Kayexalate®]), calcium acetate, calcium carbonate, or lanthanum carbonate, within the last 7 days.
4. Subjects with a life expectancy of less than 3 months.

5. Subjects who are HIV positive.

6. Subjects who are severely physically or mentally incapacitated and who in the opinion of investigator are unable to perform the subjects' tasks associated with the protocol.

7. Women who are pregnant, lactating, or planning to become pregnant.

8. Subjects with diabetic Ketoacidosis.

9. Presence of any condition which, in the opinion of the investigator, places the subject at undue risk or potentially jeopardizes the quality of the data to be generated.

10. Known hypersensitivity or previous anaphylaxis to ZS or to components thereof.

11. Previous treatment with ZS.

12. Treatment with a drug or device within the last 30 days that has not received regulatory approval at the time of study entry.

13. Subjects with cardiac arrhythmias that require immediate treatment.

14. Subjects on insulin where a stable dose has not yet been established*

15. Subjects on dialysis.

* Subjects on stable insulin or insulin analogues can be enrolled. Whenever possible, all blood draws collected prior to meals should be collected prior to insulin/insulin analogue treatment.

Drug, dose and mode of administration

Microporous, Fractionated, Protonated Zirconium Silicate (ZS, particle size $\geq 3\ \mu$m) administered orally as a slurry/suspension in purified water. Acute Phase: ZS will be administered three times daily (tid) in conjunction with meals (1.25g, 2.5g, 5g and 10g tid) or matching placebo for 48 hours for a total of 6 doses over Study Days 1 and 2.

Subacute Phase: ZS (1.25g, 2.5g, 5g and 10g tid) or matching placebo will be administered once daily (qd) in conjunction with breakfast on Study Days 3 - 14 for a total of 12 days of dosing (see study design above).

Study Duration

The treatment duration is 14 days per subject post-randomization with a subsequent final follow up visit 7 days later after the last study treatment administration for all subjects; the study will be performed on an outpatient basis. For subjects who do not enter the Subacute Phase, the last study visit will be on Study Day 3 with a subsequent final follow up visit 7 days later after the last study treatment (Study Day 9).

Reference therapy and mode of administration

Oral placebo powder (PROSOLV SMCC®90; silicified microcrystalline cellulose) with the exact same appearance, taste, odor, and mode of administration as ZS.

Criteria for evaluation

Efficacy - S-K at regular intervals

Pharmacodynamic/safety parameters

- Serum-creatinine (S-Cr) at regular intervals
- Other electrolytes (serum-sodium (S-Na), serum magnesium (S-Mg), serum calcium (S-Ca))
- Adverse Events (AEs), Serious Adverse Events (SAEs) Suspected Adverse Reactions (SARs) and Serious Unexpected Suspected Adverse Reactions (SUSARs)
- Incidence of clinically significant cardiac arrhythmias
- Laboratory safety data, vital signs, temperature, at regular intervals

Stopping rules

If a subject develops i-Stat potassium values > 7.0 or <3.0 mmol/l, or a clinically significant cardiac arrhythmia (see below), the subject should immediately receive appropriate medical treatment and be discontinued from study drug.

Acute Phase: If a subject develops i-STAT potassium values between 3.0 - 3.4 mmol/l, the next dose of study drug will not be administered. The subject will still be eligible for enrolment onto the Subacute Phase if the i-STAT potassium level is within the normal range (3.5 - 4.9 mmol/l, inclusive) on the morning of Study Day 3.

Subacute Phase: If a subject develops i-STAT potassium values <3.4 mmol/l the subject will be discontinued from the study but should return on Study Day 21 for an end of study visit. Any of the following cardiac events will result in immediate discontinuation from the study (independent of whether it is in the Acute or Subacute Phase):

- Serious cardiac arrhythmias (ventricular tachycardia or ventricular fibrillation, new atrial fibrillation or atrial flutter, paroxysmal supraventricular tachycardia [other than sinus tachycardia], 2nd or 3rd degree AV block or significant bradycardia [HR < 40 bpm])
- Acute congestive heart failure
- Significant increase in PR interval (to more than 0.25 s in the absence of pre-existing atrioventricular block), widening of the QRS complex (to more than 0.14s in the absence of pre-existing bundle branch block) or peaked Twave

Study Hypothesis

Acute Phase: It is hypothesized that ZS is more effective than placebo control (alternative hypothesis) in lowering S-K levels in subjects with S-K between 5.1 - 6.5 mmol/l versus no difference between ZS and placebo control (null hypothesis)

Subacute Phase (randomized withdrawal): It is hypothesized that ZS once daily is more effective than placebo control (alternative hypotheses) in maintaining normokalemic levels (3.5 - 5.0 mmol/l) among subjects completing the Acute Phase versus no difference between each ZS dose and respective placebo controls (null hypotheses).

Study Results

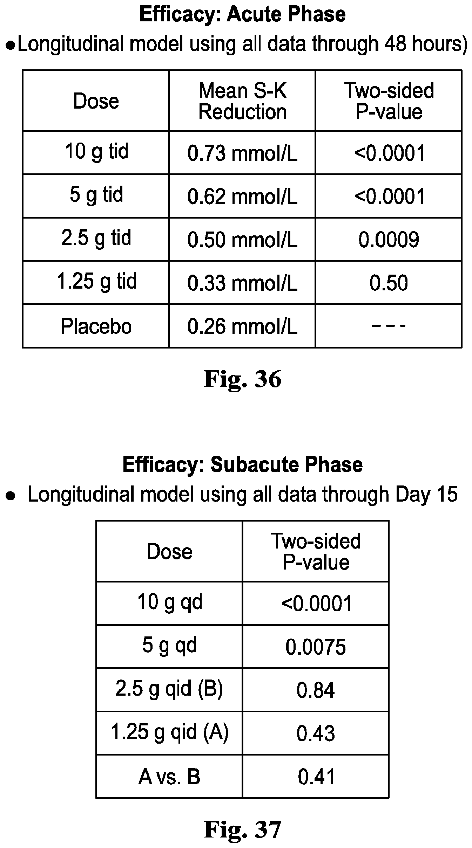

The results of the trial show significant decline in serum potassium for acute dosing as shown in Fig. 35. The statistical significance of these results is shown in Fig. 36. Statistically significant reductions in serum potassium were observed for treatment of acute hyperkalemia with doses of 2.5, 5 and 10 g administered three times daily (tid). Doses of greater than 1.25 g tid are preferred, and doses of 2.5 - 10 g tid are more preferred for treatment of acute hyperkalemia.

Statistical significance was observed for the subacute phase as shown in Fig. 37. Statistically significant reductions in serum potassium were observed for treatment of subacute or chronic hyperkalemia with doses of 5 and 10 g administered once daily (qd). Doses of greater than 2.5 g qd are preferred, with 5 - 10 g qd are more preferred for treatment of subacute hyperkalemia.

Serum Potassium Dependent Dosing Regimens Serum potassium levels exceeding 5.0 meq/l are considered hyperkalemic. Patients exhibiting a serum potassium level of 3.5 meq/l or below are considered hypokalemic. The goal of this dosing regimen is to maintain patients within the normal serum potassium range of 3.5 to 4.9 meq/l.

During the initial induction phase of this dosing regimen, patients having elevated serum potassium levels of 5.3 meq/g (corresponding to plasma levels by iStat of 5.4 meq/l) are preferably administered 10 g tid for two days. The dose could range from 2.5 to 30 grams per day total dose until serum potassium falls below 5.0.

Where serum potassium is in the sub-acute range of 4.0 to 4.9, the patients are administered total doses of 5 to 20 grams per day, using preferably 5.0, 7.5 and 10.0 grams bid,, until serum potassium is brought below 4.0 meq/g, at which point qd dosing will ensue.

Where serum potassium is in the chronic range of below 4.0, dosing of 5.0, 7.5, and 10.0 grams qd are used. This could also be 1.25 to 10 g tid dosing.

## EXAMPLE 25

Hyperkalaemia is a risk factor for mortality in patients with cardiovascular disease and chronic kidney disease (CKD) (Goyal, 2012; Torlen, 2012) and limits use of renin-angiotensin-aldosterone system inhibitors (RAASi) in these patients. Sodium (or calcium) polystyrene sulfonate (SPS/CPS) has uncertain efficacy and has been associated with substantial adverse events, as well as poor gastrointestinal tolerability, and hence is suboptimal for acute use and unsuitable for chronic use (Harel, 2013; Sterns, 2010). Therefore, there is a need for a hyperkalaemia treatment that rapidly reduces serum potassium (K+) and is safe and well tolerated in these patients. ZS-9, a nonabsorbed cation exchanger designed to specifically entrap excess K+, significantly reduced K+ vs placebo over 48 hr with excellent tolerability in patients with CKD (Ash, 2013). We report acute-phase efficacy in a Phase 3 trial of ZS-9 in patients with hyperkalemia.

Patients (N=753) with serum K+ 5-6.5 mmol/L were randomised (1:1:1:1:1) to ZS-9 (1.25g, 2.5g, 5g or 10g) or placebo given three times daily (TID) with meals for 48 hr (acute phase), after which those with K+ ≤4.9 mmol/L (n=542) were re-randomised to ZS-9 or placebo once daily for Day 3-15. Serum K+ was measured at baseline and at predefined intervals, including 1, 4, 24, and 48 hr after the first dose. The acute-phase primary efficacy endpoint was the rate of $K^+$ change over the first 48 hr, using longitudinal modeling to account for all post-baseline data.

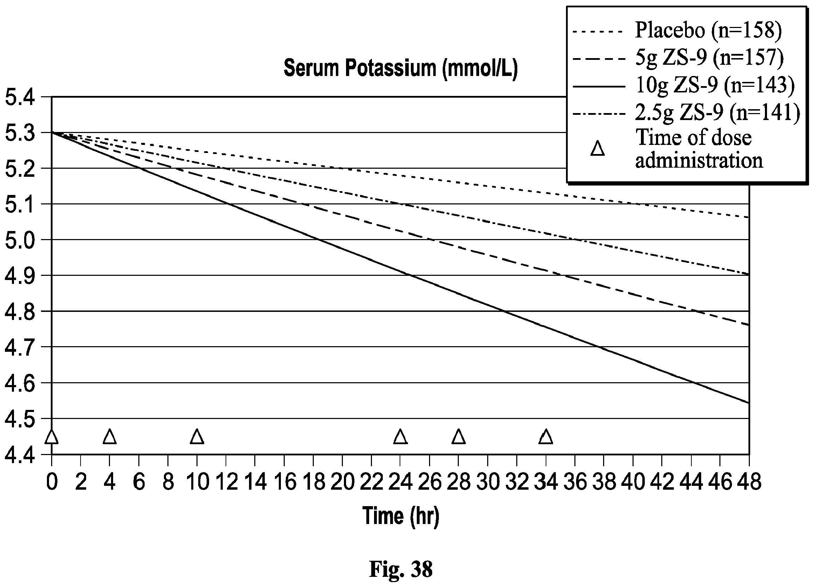

Mean $K^+$ at baseline was 5.3 mmol/L. Substantial percentages of patients had CKD (60%), a history of heart failure (40%), or diabetes (60%) or were on RAASi therapy (67%). ZS-9 demonstrated significant dose-dependent reductions in $K^+$; the acute-phase primary efficacy endpoint was met for ZS-9 2.5g (p=0.0009), 5g (p<0.0001) and 10g TID (p<0.0001; Fig. 38).

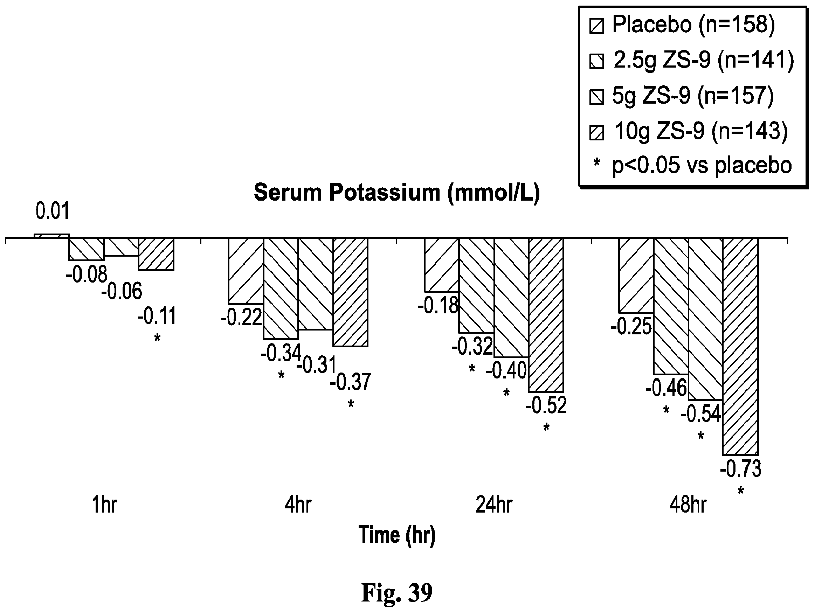

There was a significant decrease in $K^+$ by -0.11 mmol/L with ZS-9 10g vs an increase of +0.01 mmol/L with placebo (p=0.009) 1 hr after the first dose (Fig. 39). Reductions in $K^+$ were significant at 4 hr for the 2.5g and 10g doses and at 24 and 48 hr for the 2.5g, 5g, and 10g doses vs placebo.

Rates of all adverse events (AEs) and gastrointestinal AEs were not significantly different in the ZS-9 and placebo groups.

ZS-9 produced significant dose-dependent reductions in $K^+$ when given TID for 48 hr, with an AE profile similar to placebo. The significant reduction in serum $K^+$ 1 hr after the first ZS-9 10g dose further suggests that ZS-9 is effective in removing $K^+$ from the small intestine fluid, where it is in equilibrium with blood levels. ZS-9 may address an important unmet clinical need by rapidly correcting hyperkalaemia in high-risk patients, many of whom require RAASi for end-organ protection.

## EXAMPLE 26

The use of RAAS inhibitors (RAASi) are limited by hyperkalemia (HK, where the serum K+ is >5.0 mEq/L), and is a mortality risk factor in patients with heart failure (HF) and chronic kidney disease (CKD). The use of ZS-9 was well tolerated and acutely reduced and maintained $K^+$ in hyperkalemia patients in the Phase 3 study (see Example 22). This example describes the acute phase efficacy of ZS-9 vs placebo (PBO) across pre-specified subgroups of patients baseline (BL) K+, eGFR, history of heart failure, CKD, diabetes mellitus (DM), and RAASi use.

Patients (n=753) with serum potassium levels of 5.0-6.5 mEq/L were randomized (1:1:1:1:1) to ZS-9 (1.25, 2.5, 5 or 10g) or placebo orally 3X/day for 48 hr, after which patients with potassium less than 4.9 mEq/L (n=542) were switched to ZS-9 or placebo 1x/day on Days 3-14. RAASi was kept constant. Mean serum K+ (95% CIs) was calculated at baseline and 48 hr. Differences between groups were compared using unpaired t-test.

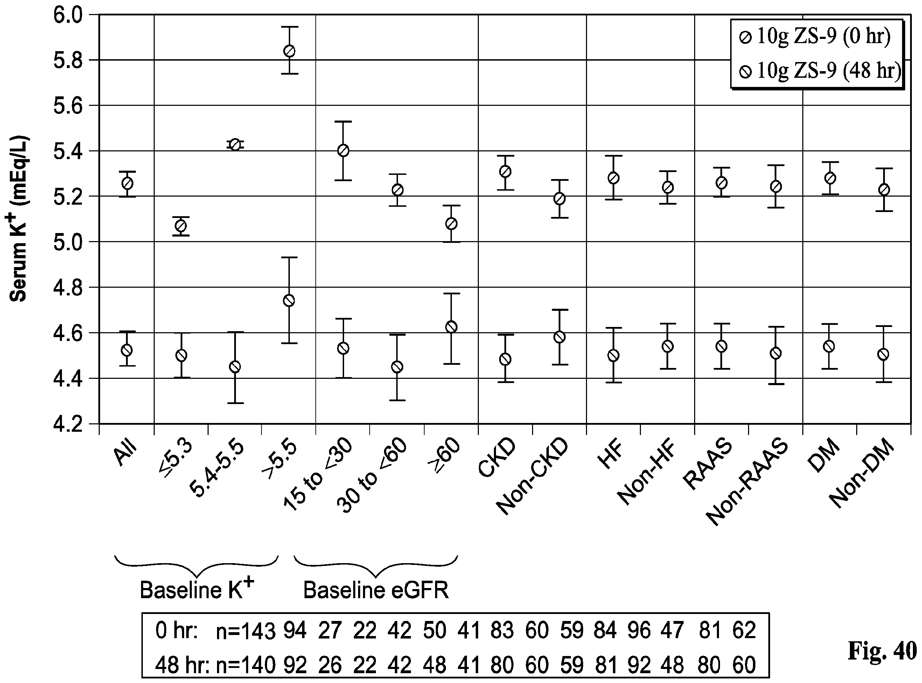

The prevalence of the subgroups of patients were classified as having CKD (60%), heart failure (41%), and diabetes mellitus (58%); and 2/3 of the patients were on RAASi. ZS-9 10g (n=158) vs placebo (n=143) groups are presented. Mean baseline potassium was 5.3 mEq/L in both ZS-9 and placebo groups. Mean change in potassium at 48 hr was -0.73 mEq/L and -0.25 mEq/L in ZS-9 10g and placebo groups, respectively (p<0.001). At 48 hr, normokalaemia was achieved in the overall 10g ZS-9 group and in all subgroups. Fig. 40.

Patients with starting K+ >5.5 mEq/L had the greatest decrease in K+ with 10g ZS-9 (-1.1 mEq/L vs -0.4 mEq/L PBO; p<0.001). Little difference was observed in the adverse events in the acute phase between the groups (12% ZS-9 vs 11% PBO; p=0.86).

This demonstrates that ZS-9 is well tolerated and achieved normokalemia in all pre-specified subgroups of hyperkalemia patients with CKD, heart failure, diabetes mellitus and on RAASi and may potentially permit optimal cardiorenal protection by life-saving RAASi.

## EXAMPLE 27

Hyperkalaemia (potassium [$K^+$] >5.0 mmol/L) is a common disorder in patients with chronic kidney disease (CKD), diabetes, and in those on renin-angiotensin-aldosterone inhibitor therapy. Polystyrene sulfonate (sodium or calcium) has limited efficacy and has been associated with substantial adverse events (AEs) and poor gastrointestinal (GI) tolerability. There is a need for a safe, fast-acting, effective treatment for sustained reduction of serum $K^+$ in patients with hyperkalaemia, independent of its severity. ZS-9, a nonabsorbed cation exchanger designed to specifically entrap excess $K^+$ in the GI tract, was shown to significantly reduce $K^+$ (vs placebo) over 48 hr with excellent tolerability in patients with CKD and $K^+$ 5-6 mmol/L. Here we report acute-phase efficacy stratified by baseline $K^+$ in a large Phase 3 trial of ZS-9 in patients with relatively more severe, asymptomatic hyperkalaemia.

Patients (N=753) with $K^+$ 5.0-6.5 mmol/L were randomised (1:1:1:1:1) to ZS-9 (1.25g, 2.5g, 5g or 10g) or placebo given three times daily (TID) with meals for 48 hr (acute phase), after which those with $K^+$ ≤4.9 mmol/L (n=542) were re-randomized to ZS-9 or placebo once daily for Days 3-15. Changes in serum $K^+$ over 48 hr stratified by starting $K^+$ (≤5.3, 5.4-5.5, and >5.5 mmol/L) for ZS-9 5g and 10g vs placebo were compared by unpaired t-test.

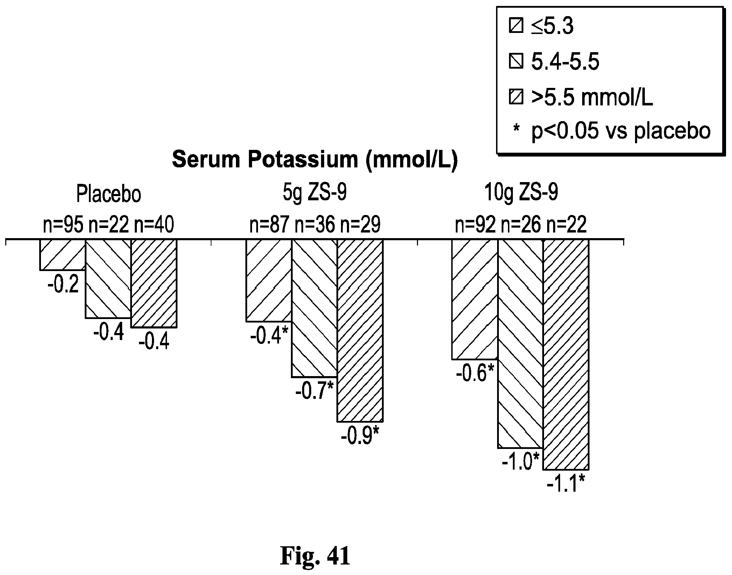

Baseline $K^+$ was ≤5.3 mmol/L in 427 (56.7%), 5.4-5.5 mmol/L in 152 (20.2%) and >5.5 in 174 (23.1%). Within each of these subgroups, mean $K^+$ levels were similar across treatment groups at baseline (Table). At 48 hr, patients on ZS-9 5g or 10g TID had significantly greater decreases in $K^+$ than did those on placebo, regardless of baseline $K^+$ (Table, Figure 41). For those with starting $K^+$ >5.5 mmol/L, the ZS-9 10g dose group achieved a mean $K^+$ reduction of 1.1 mmol/L at 48 hr, 14 hr after the last dose of ZS-9. Mean $K^+$ levels for ZS-9 5g and 10g TID were within the normokalaemic range (3.5-4.9 mmol/L) at the end of the acute phase (Table 10), and there was no severe hypokalaemia (<3.0 mmol/L) during the study. In the overall population, rates of AEs were not significantly different in the ZS-9 5g, 10g, and placebo groups.

| Table 10: Mean (SD) Acute Efficacy Phase $K^+$ Values (mmol/L) | | | | | | | |
|---|---|---|---|---|---|---|---|
| **Acute** | | N | ≤5.3 | N | 5.4-5.5 | N | >5.5 |
| **Placebo** | Acute Phase Baseline | 95 | 5.1 (0.20) | 22 | 5.5 (0.05) | 41 | 5.8 (0.18) |
| | 48 Hour | 95 | 4.9 (0.45) | 22 | 4.9 (0.43) | 40 | 5.4 (0.46) |
| | Δ **baseline** | **95** | **-0.2 (0.41)** | **22** | **0.6 (0.41)** | **40** | **-0.4 (0.41)** |
| **5g ZS-9** | Acute Phase Baseline | 90 | 5.1 (0.18) | 36 | 5.5 (0.05) | 31 | 5.7 (0.19) |
| | 48 Hour | 87 | 4.7 (0.41) | 36 | 4.8 (0.43) | 29 | 5.0 (0.48) |
| | Δ **baseline** P-value (vs placebo) | **87** | **-0.4 (0.40)** <0.001 | **36** | **-0.7 (0.44)** 0.010 | **29** | **-0.9 (0.46)** <0.001 |
| **10g ZS-9** | Acute Phase Baseline | 94 | 5.1 (0.46) | 27 | 5.4 (0.05) | 22 | 5.8 (0.24) |
| | 48 Hour | 92 | 4.5 (0.48) | 26 | 4.5 (0.38) | 22 | 4.7 (0.43) |
| | Δ **baseline** P-value (vs placebo) | **92** | **-0.6 (0.46)** <0.001 | **26** | **-1.0 (0.39)** <0.001 | **22** | **-1.1 (0.47)** <0.001 |

Results of this subgroup analysis indicate that ZS-9 TID is effective in reducing $K^+$ over 48 hr, regardless of baseline $K^+$ concentration. Importantly, $K^+$ reductions were largest in patients with the highest baseline $K^+$ levels, suggesting that ZS-9 TID promotes a return to normokalaemia regardless of starting $K^+$, with a low risk (0.3%) of mild hypokalaemia (3.0-3.5 mmol/L). ZS-9 is a novel therapy designed to specifically entrap excess $K^+$ and may address an important unmet medical need by rapidly correcting various levels of hyperkalaemia.

**EXAMPLE 28**

Metabolic acidosis is a common finding in patients with chronic kidney disease (CKD) and hyperkalaemia. Treatment of hyperkalaemia with sodium (or calcium) polystyrene sulfonate has uncertain efficacy and has been associated with poor tolerability and rare intestinal necrosis. ZS-9 is a selective cation exchanger designed to entrap excess potassium ($K^+$) in exchange for sodium and hydrogen. ZS-9 absorbs ammonium as well as $K^+$. In a multicenter, randomised, double-blind, controlled study, ZS-9 5g and 10g was shown to significantly reduce $K^+$ vs placebo over 48 hr with excellent tolerability in patients with CKD. Here we report relevant acid-base related laboratory values with ZS-9 10g and placebo during this Phase 2 trial.

Patients (glomerular filtration rate, 30-60 mL/min/1.73 $m^2$; $K^+$, 5-6 mmol/L) were randomized 2:1 to ZS-9 (n=60; 0.3g [n=12], 3g [n=24], or 10g [n=24]) or placebo (n=30) given orally three times daily for 2 days (and up to 2 more days if $K^+$ ≥5.0 mmol/L; only 2 days needed for ZS-9 10g) with regular meals as in-patients. Serum and urine samples were collected through Day 7. RAAS inhibitors were continued during the study. Differences between groups were compared by unpaired t-test.

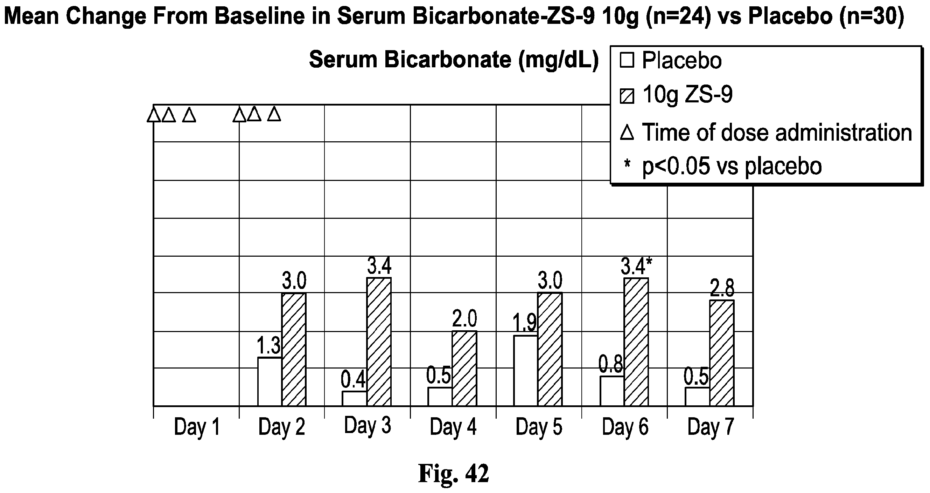

At baseline mean bicarbonate (28.1 mg/dL and 27.4 mg/dL) and urinary pH (5.8 and 5.7) were similar between ZS-9 10g and placebo, respectively. Bicarbonate increased more with ZS-9 10g than with placebo from Day 2-7. By Day 3 (14 hr after the last dose of ZS-9 10g) bicarbonate increased by +3.4 mg/dL with ZS-9 10g vs +0.4 mg/dL with placebo; at Day 6 the difference between groups was significant (p<0.05; Fig. 42).

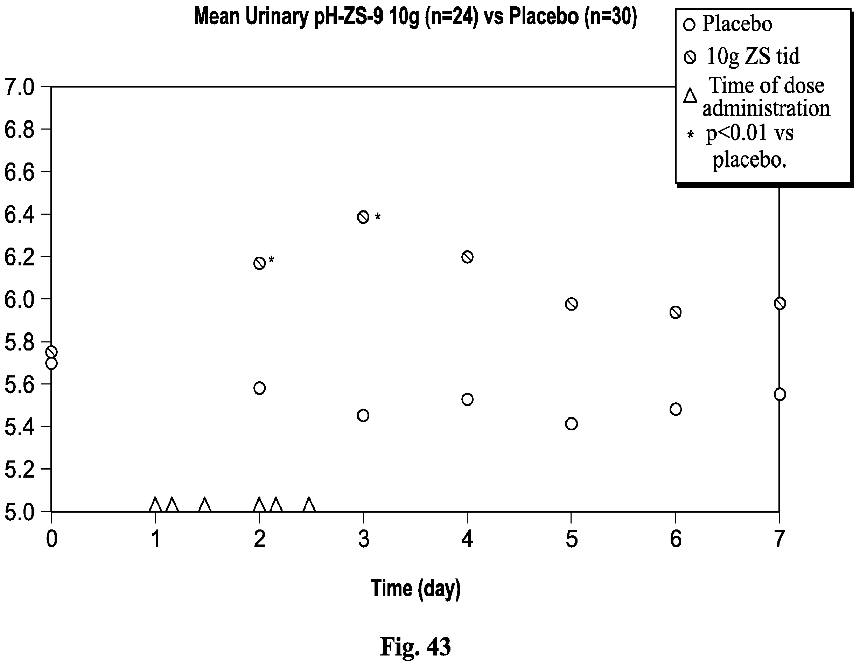

Mean urinary pH increased with ZS-9 10g to 6.2 at Day 2 and 6.4 at Day 3 and remained higher than placebo through Day 7 (Fig. 43). In contrast, urine pH fell in the placebo group to 5.6 at Day 2 and 5.5 at Day 3, resulting in significant (p<0.01) differences between groups at both time points. Mean blood urea nitrogen (BUN) decreased from baseline with ZS-9 10g vs placebo (p<0.05 for all evaluations between Day 2-7). There were no cases of significant hypocalcemia (≤8 mg/dL), hypomagnesemia (≤1.2 mmol/L), or hypokalaemia (≤3.0 mmol/L).

Serum bicarbonate increased by approximately 12% from baseline with ZS-9 10g after 48 hr. Increases in urinary pH were also observed, suggesting that ZS-9 may improve acid-base balance in CKD patients with hyperkalaemia. The improvement in metabolic acidosis can be explained by removal of ammonium by ZS-9, as illustrated by the significant reduction in BUN. A two-stage Phase 3 trial that has just completed (N=753) will provide a larger dataset with which to evaluate ZS-9's effects in patients with hyperkalaemia and the impact on acid-base balance.

**EXAMPLE 29**

Hyperkalaemia predicts mortality in patients with cardiovascular disease and chronic kidney disease (CKD), and limits use of life-saving renin-angiotensin-aldosterone system inhibitors (RAASi). Sodium (or calcium) polystyrene sulfonate (SPS, CPS) has unreliable efficacy and has been associated with potentially serious adverse events. Due to poor gastrointestinal tolerability, SPS or CPS is not suitable for chronic use. ZS-9, a nonabsorbed cation exchanger designed to specifically entrap excess potassium ($K^+$), significantly reduced serum $K^+$ vs placebo over 48 hr with excellent tolerability in patients with hyperkalaemia and CKD. Here we report the efficacy of ZS-9 during extended maintenance treatment in a Phase 3 trial in hyperkalaemic patients.

Patients (N=753) with serum $K^+$ 5.0-6.5 mmol/L were randomised (1:1:1:1:1) to ZS-9 (1.25g, 2.5g, 5g or 10g) or placebo three times daily for 48 hr (acute phase), after which those with $K^+$ ≤4.9 mmol/L were re-randomised 1:1 to the same dose of ZS-9 given during the acute phase or placebo once daily (QD) for Day 3-15 (extended phase). Serum $K^+$ was measured at baseline and at predefined intervals, including on Days 4-6, 9, 15 and 21 (7 days after the last dose of study drug). The primary efficacy endpoint for this phase was the rate of $K^+$ change over Day 3-15, using longitudinal modeling to account for all post-baseline data.

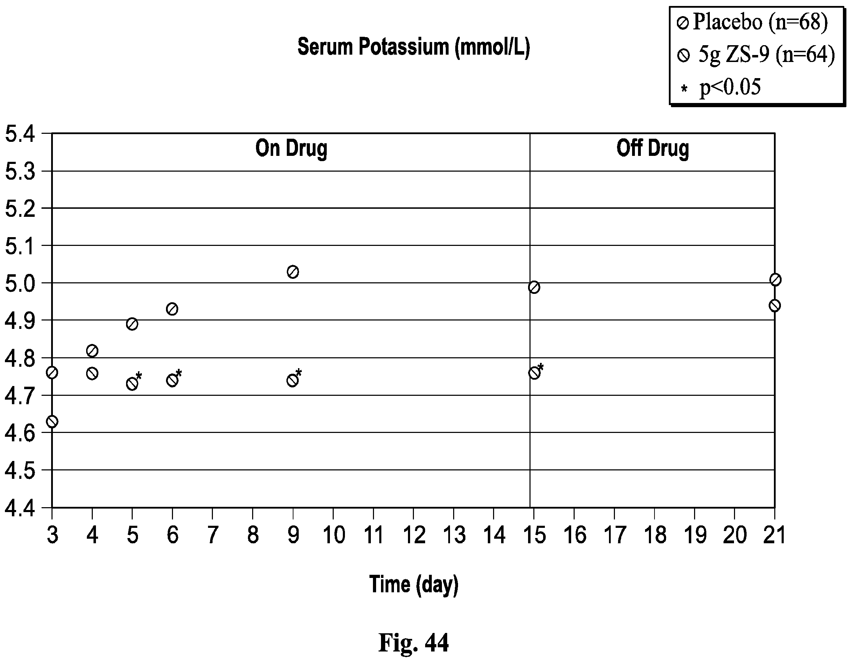

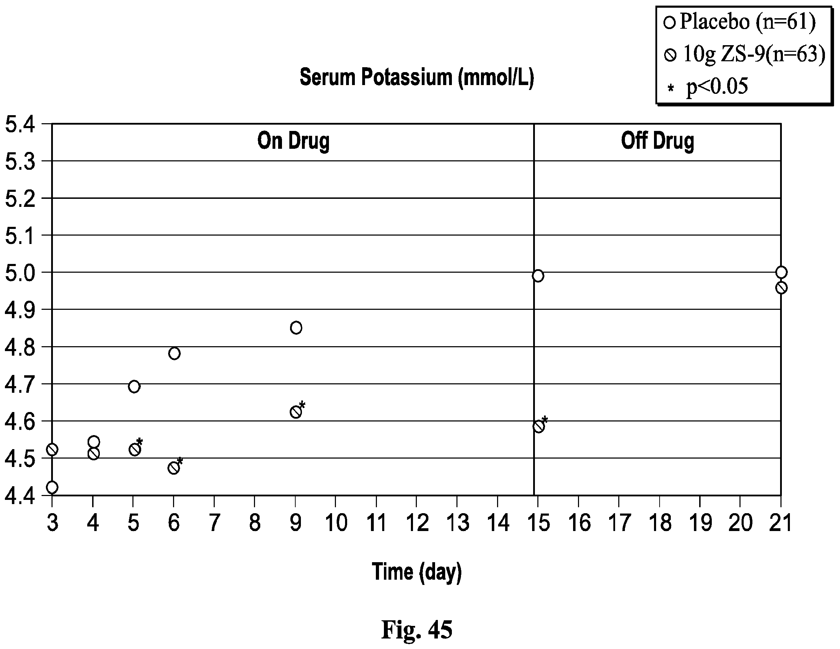

Mean $K^+$ at baseline was 5.3 mmol/L; the prevalence of CKD, heart failure, or diabetes was 60%, 40% and 60% respectively. Two-thirds of the patients were on concomitant RAASi. Overall, 542 (72%) patients entered the extended phase. The primary efficacy endpoint was met for ZS-9 5g (p<0.008) and 10g QD (p<0.0001). Between Day 3-15, mean $K^+$ was maintained between 4.6 and 4.8 mmol/L in the ZS-9 5g group (Figure 44) and 4.5 to 4.6 mmol/L in the ZS-9 10g group (Figure 45), indicating that normokalaemia was maintained. The placebo groups experienced a rise in mean $K^+$ starting on Day 5, reaching 5.0 mmol/L by Day 15. At each evaluation point between Day 5-15, mean $K^+$ was lower for both 5g and 10g QD vs placebo (p<0.05). After the last ZS-9 dose on Day 15, mean $K^+$ increased to levels similar to those in the placebo groups by Day 21.

Rates of adverse events were not significantly different for ZS-9 groups vs placebo during the extended-treatment phase.

In this Phase 3 trial, ZS-9 5g and 10g QD maintained normokalaemia for 12 days compared with placebo. This effect was more pronounced with ZS-9 10g, with a relatively lower and narrower range of mean serum $K^+$. ZS-9 once daily may fulfil an important unmet need by safely and effectively maintaining normokalaemia in high-risk patients, including those requiring treatment with RAASi.

**EXAMPLE 30**

Using the study criteria and data described in Example 22, a subgroup of patients with diabetes mellitus was examined for outcomes relating to treatment with placebo or ZS-9. The subgroup of patients having diabetes mellitus was examined for multiple acute (3 times daily, TID) and extended (once daily, QD) treatment regimens of ZS-9 according to figure 39. The acute phase was determined to be the primary efficacy endpoint and was measured as the rate of potassium change from baseline over a 48 hour period. The Extended phase was determined to be the secondary efficacy endpoint and was measured as the rate of potassium change over a period of 3-15 days. Patients receiving ZS-9 who achieved normokalemia (K+ 3.5-5.0 mEq/L) in the acute phase were re-randomized to either the same dose of ZS-9 or placebo (QD dosing) for the extended phase. Adverse events (AEs) and serious AEs were recorded through study end.

An analysis of a subgroup of patients with DM from the acute treatment phase of a Phase 3 trial of ZS-9 (5g and 10g) and placebo with TID dosing for the treatment of hyperkalemia showed:

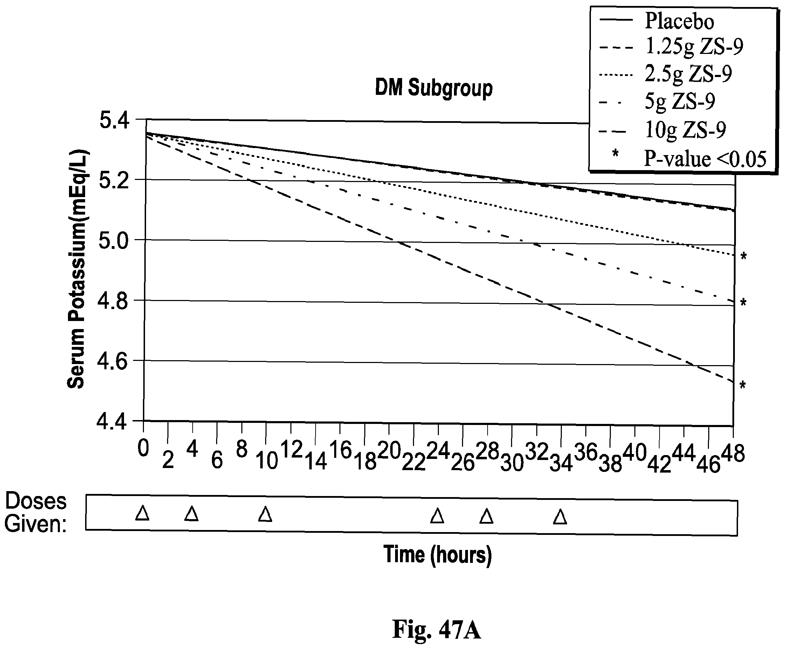

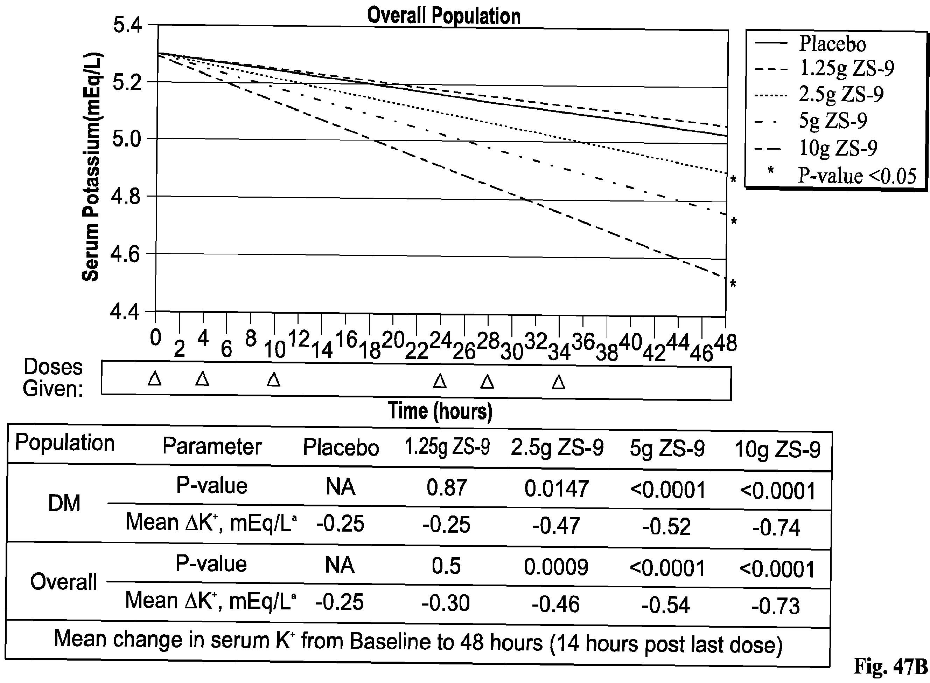

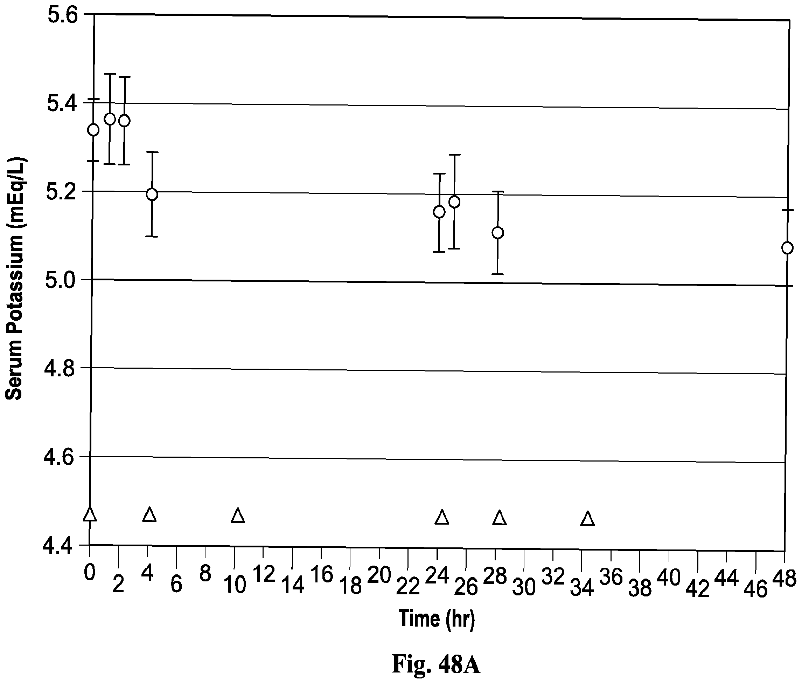

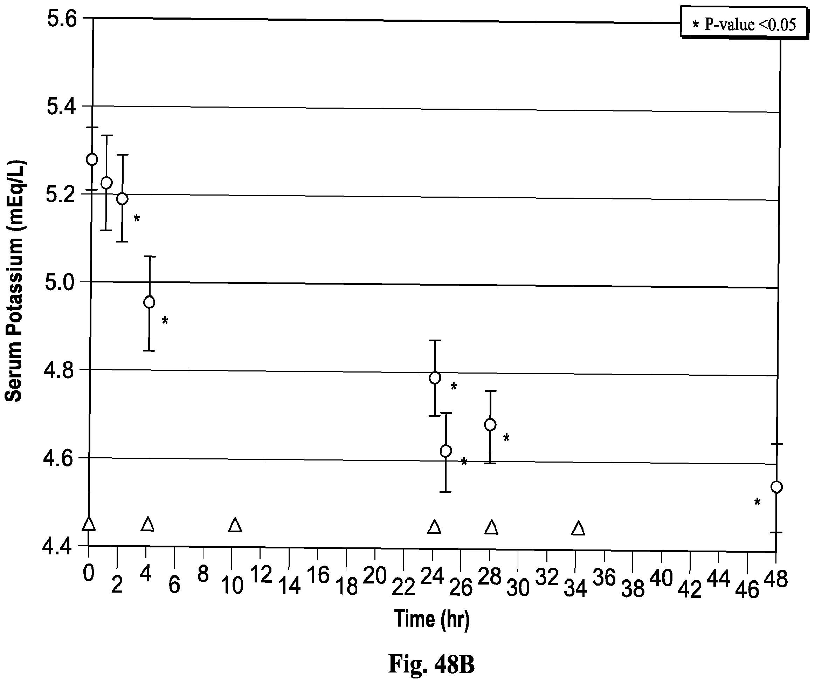

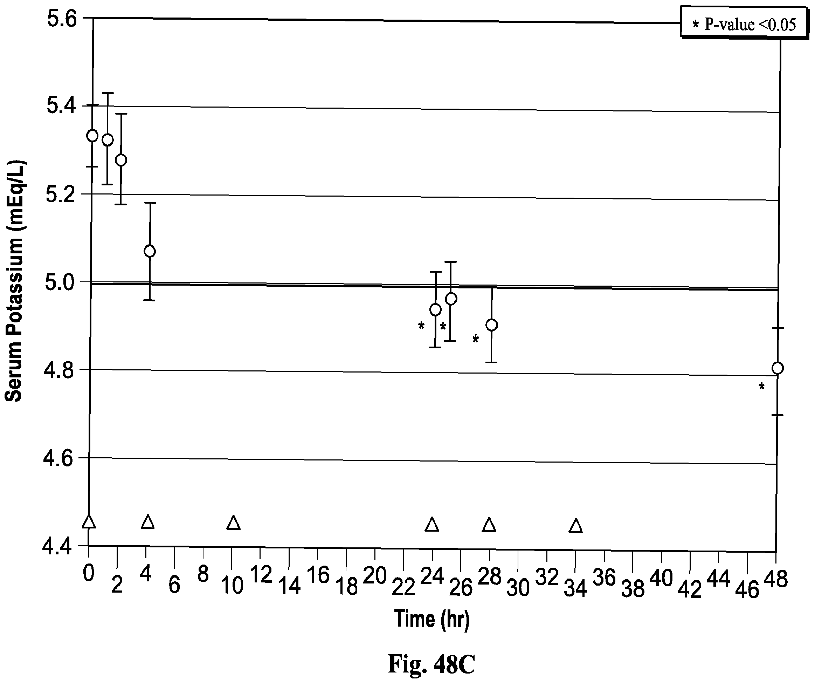

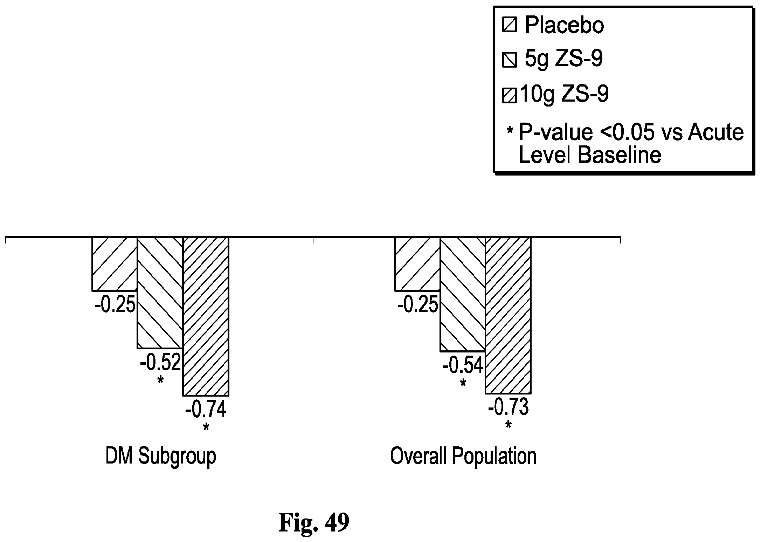

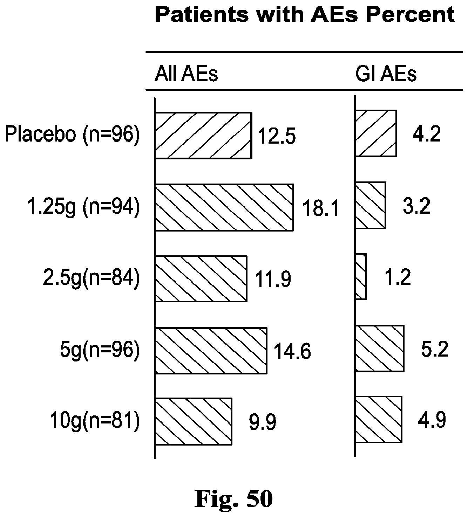

- ZS-9 led to a dose-dependent reduction in serum potassium in the first 48 hours with TID dosing (Figure 47).
- The mean change in potassium was significantly greater in the 2.5g, 5g, and 10g ZS-9 dose groups, compared with placebo (Figure 47).
- Significant reduction in mean potassium was achieved by 4 hours in the ZS-9 10g dose group (Figure 48).
- Changes in K+ were not related to changes in blood sugar.
- There were no apparent differences in magnitude of K+ reduction between the diabetes mellitus subgroup and the overall population (Figure 49).
- Rates of adverse events were similar between ZS-9-treated patients and placebo-treated patients (Figure 50).

The study showed that ZS-9 at 5g and 10g restored normokalemia with a low incidence of adverse events in hyperkalemic patients with diabetes mellitus. These results are promising for patients with DM who are more susceptible to HK and potentially more difficult to treat than the overall population. This demonstrates that ZS-9 represents a therapeutic opportunity to treat hyperkalemia in patients with diabetes mellitus.

An analysis of a subgroup of patients with diabetes mellitus from the extended treatment portion of the Phase 3 trial of ZS-9(10g) and placebo with QD dosing for treatment of HK showed:

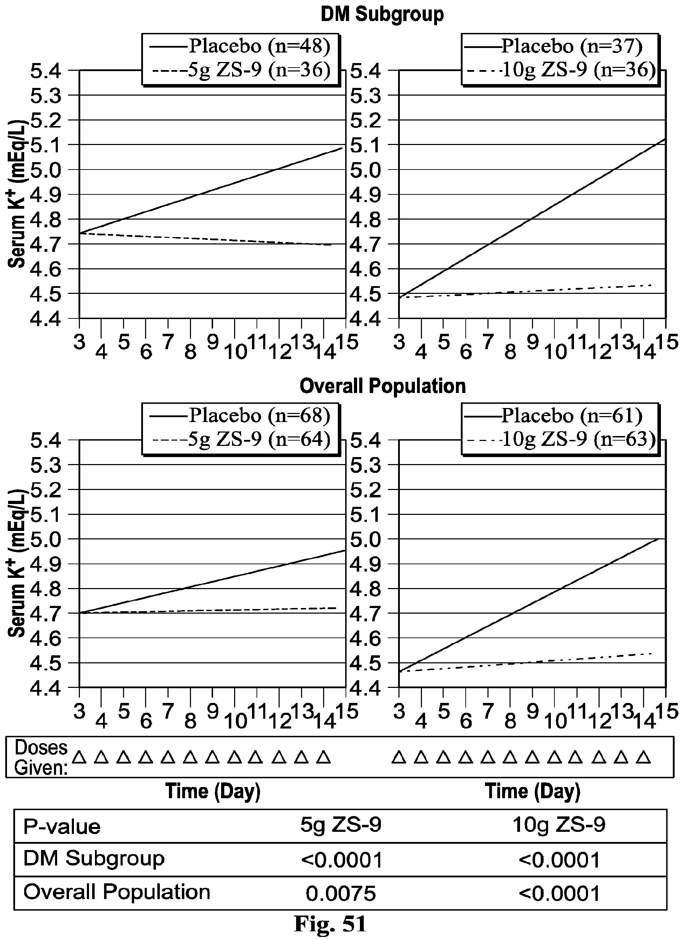

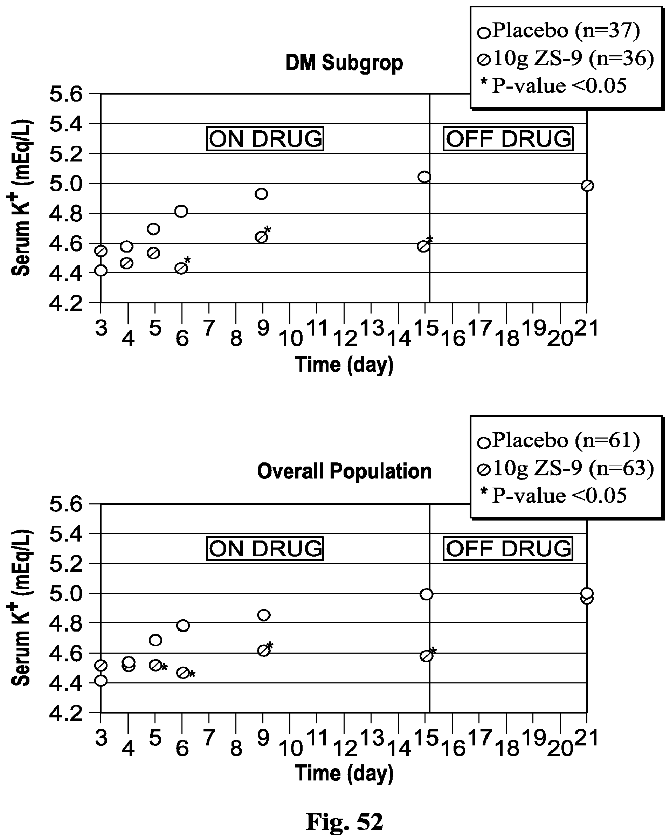

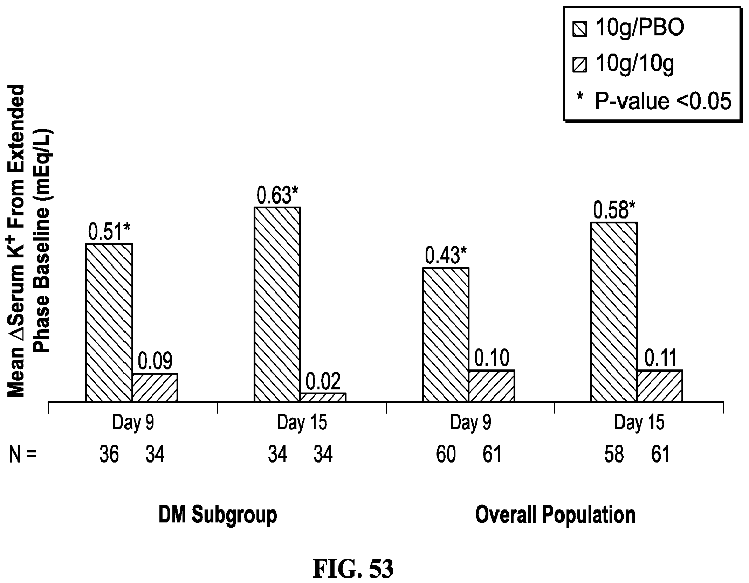

- 5g and 10g ZS-9 maintained normokalemia with QD dosing after achieving normokalemia with TID dosing (Figures 51 and52).
- In patients who switched to placebo after restoring normokalemia, serum K+ returned to baseline hyperkalemic levels (Figures 51 and 52).
- Changes in potassium were not related to changes in blood sugar.
- Rates of AEs and GI AEs were similar between ZS-9 and placebo groups in both the acute phase and the extended phase (Figure 53).

These findings are promising for the subgroup of patients with diabetes mellitus who are more susceptible to hyperkalemia and face greater challenges in obtaining effective therapies. This demonstrates that ZS-9 is an important therapy for restoring and maintaining normokalemia, particularly by facilitating the optimization of RAAS therapies and other medications in patients with diabetes mellitus.

## EXAMPLE 31

In another phase 3 study the effect of ZS compositions having greater than about 95% ZS-9 were measured in an acute phase and maintenance phase at three different daily dose levels of 5g, 10g, and 15g. Administration of the ZS composition achieved the following mean serum potassium levels (mEq/L) during the maintenance phase, days 8-29:

| Mean Serum K+ from Dav 8-29 | | | | |
|---|---|---|---|---|
| | Placebo | 5g (n=45) | 10g (n=50) | 15g (n=54) |
| Mean K+ (mEq/L) | 5.06 | 4.75 | 4.51 | 4.37 |

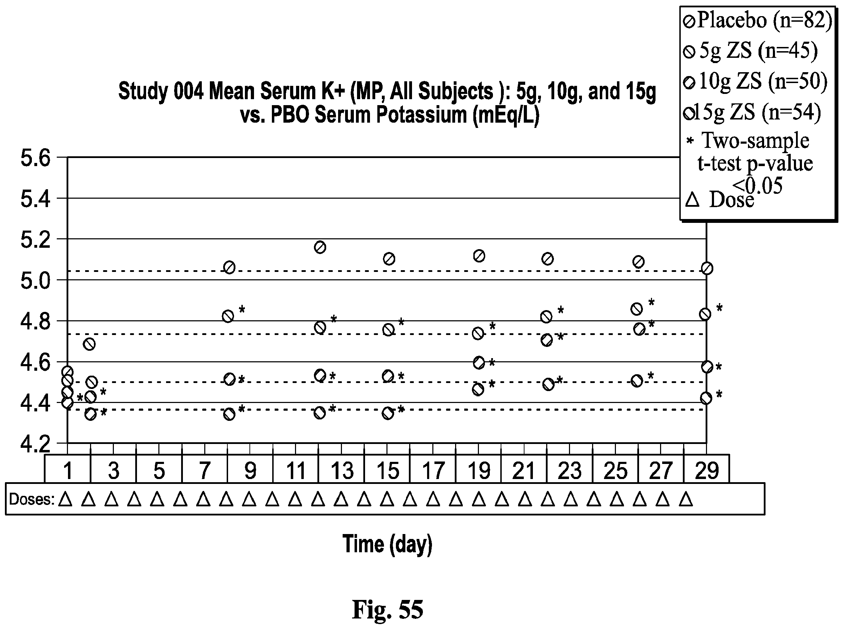

These data show that the level of serum potassium *in vivo* after administering the ZS compositions is dose dependent and can be titrated as needed. As shown in Fig. 55, the mean serum potassium is controlled within close ranges for each of the dose levels.

As shown in the table below, the ZS administration resulted in between 13 and 17 days of normal serum potassium levels within days 8-29 of treatment.

| Number of Normalkalemic Days Between MP Days 8-29 | | | | |
|---|---|---|---|---|
| | Placebo | 5g (n=45) | 10g (n=50) | 15g (n=54) |
| Mean Number of Normalkalemic Days | 7.4/22 34% | 13.4/22 61% | 13.9/22 63% | 16.8/22 76% |

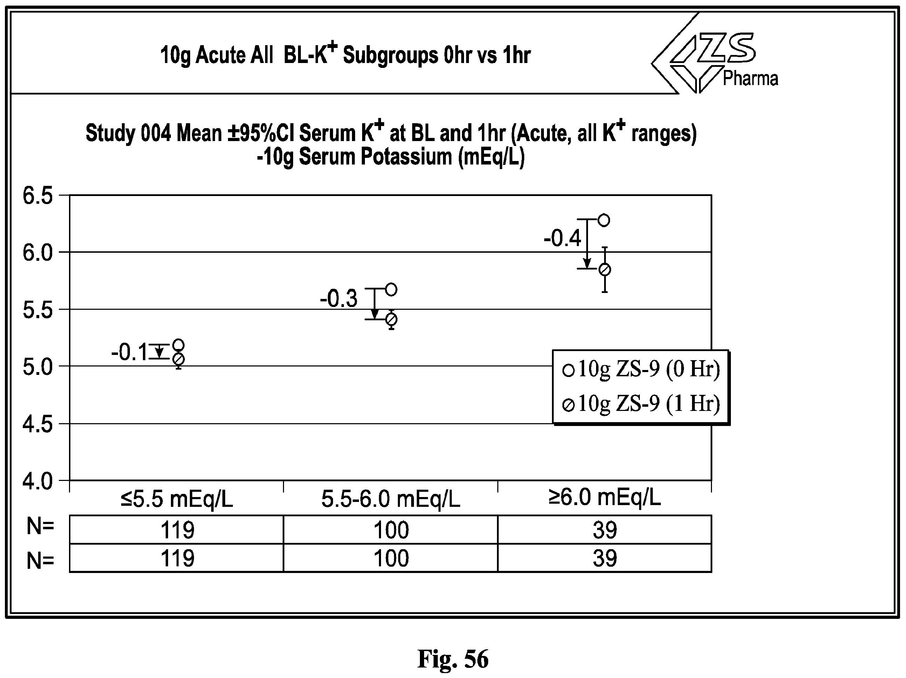

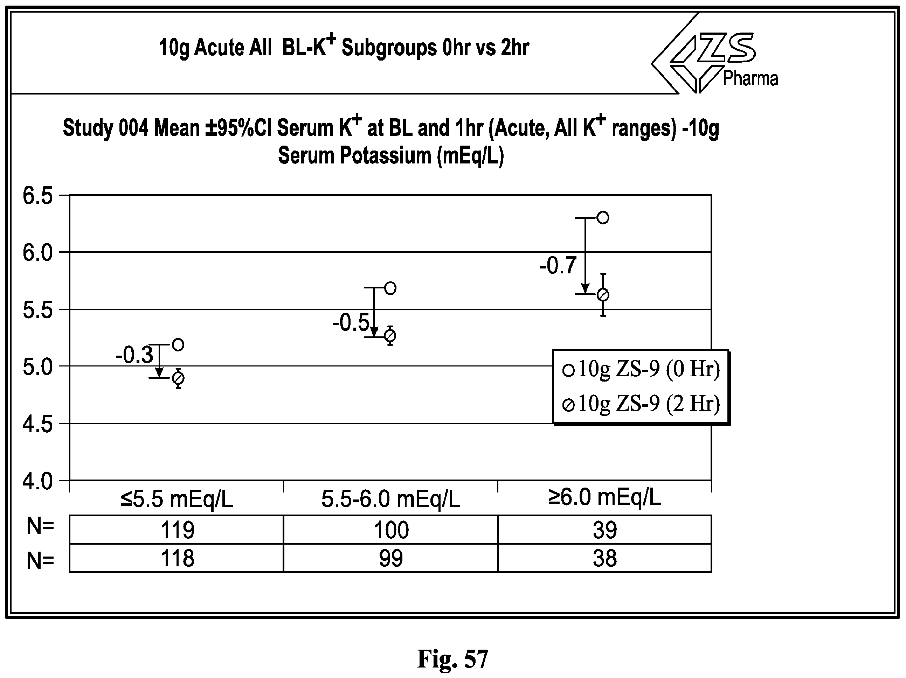

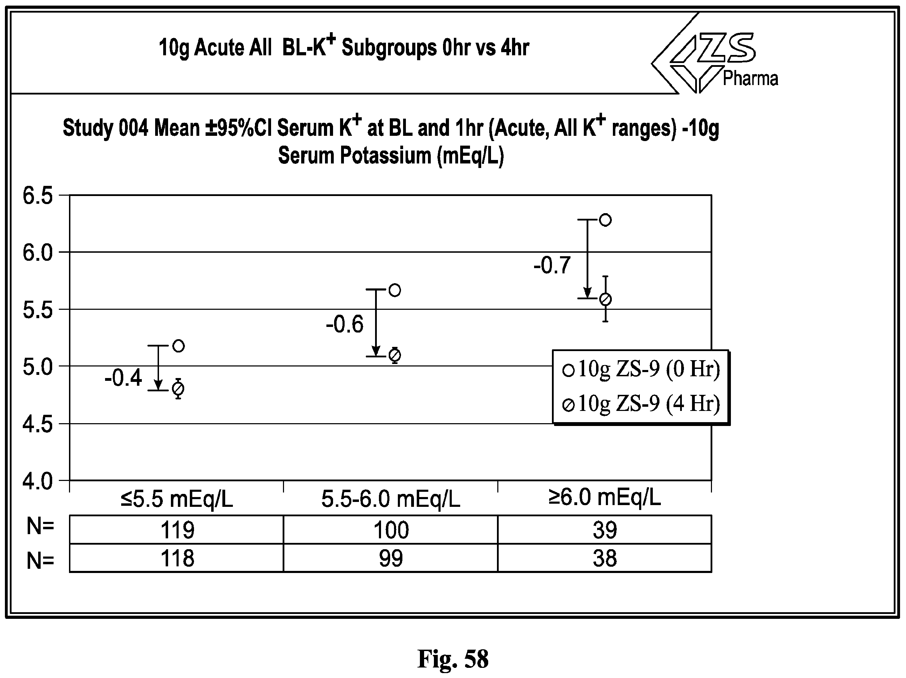

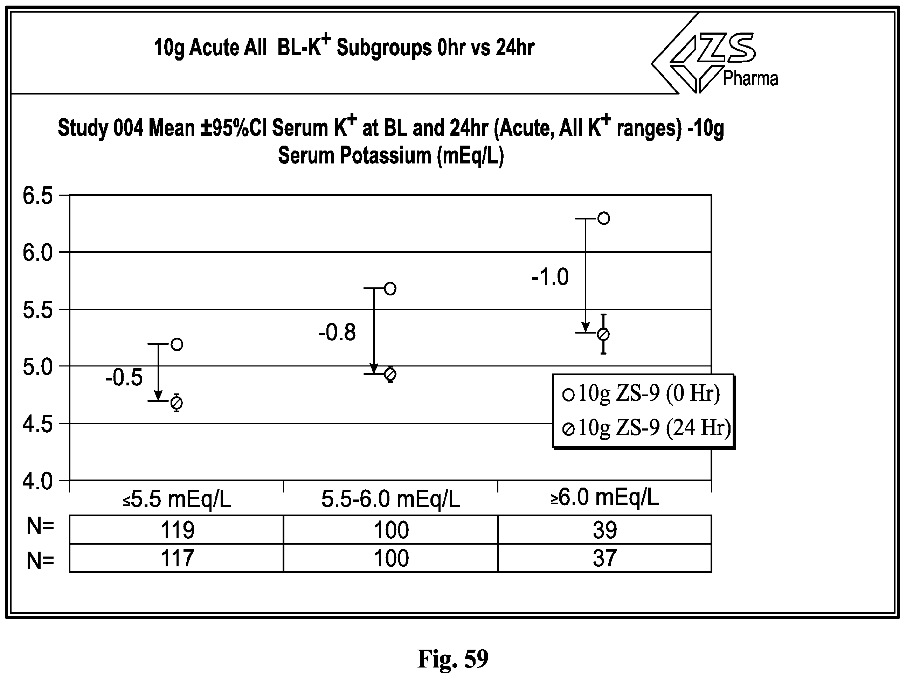

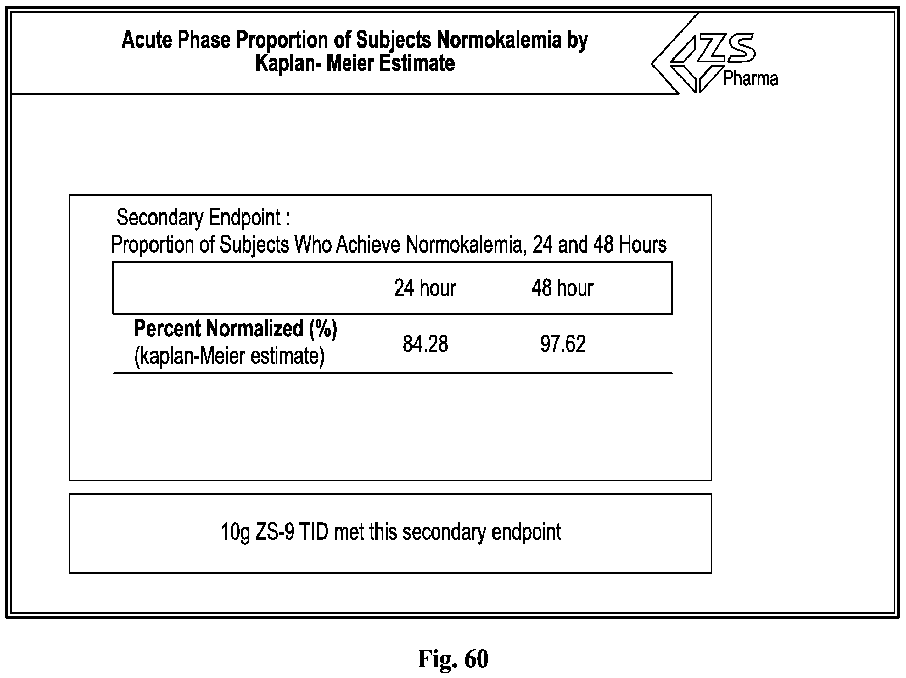

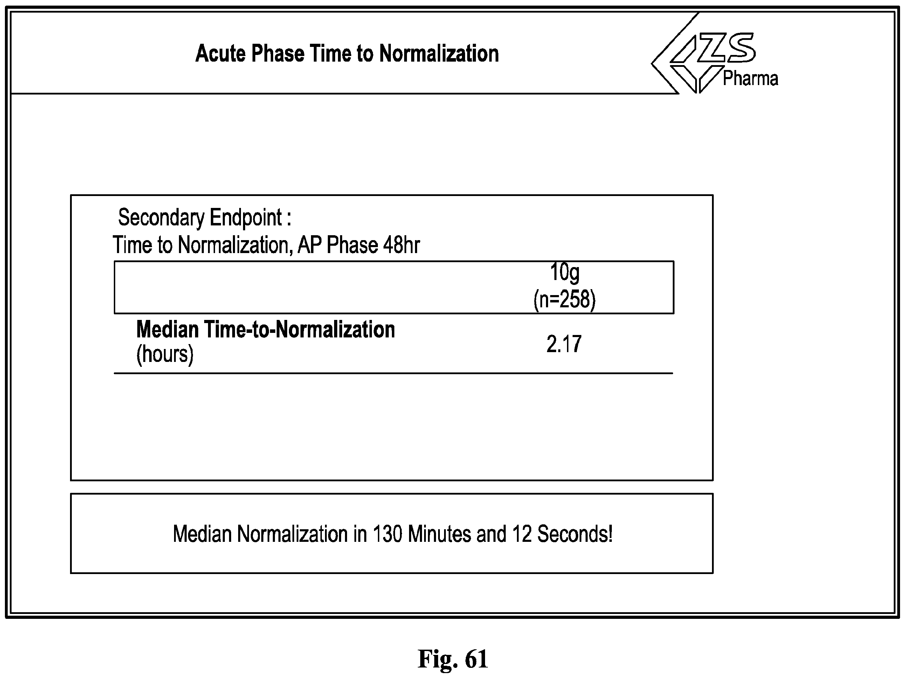

The 95% or greater ZS prepared in accordance with Example 23 when administered at the 10g level exhibited a significant and rapid reduction in serum potassium. Fig. 56 shows the reduction in serum potassium after 1 hour. Fig. 57 shows the reduction in serum potassium after 2 hours. Fig. 58 shows the reduction in serum potassium after 4 hours. Fig. 59 shows the reduction in serum potassium after 24 hours. Fig. 60 shows that 84% of patients achieved normalization of serum potassium within 24 hours, and 98% achieved normalization within 48 hours. The median time to normalization shown in Fig. 61 is 2.17 hours.

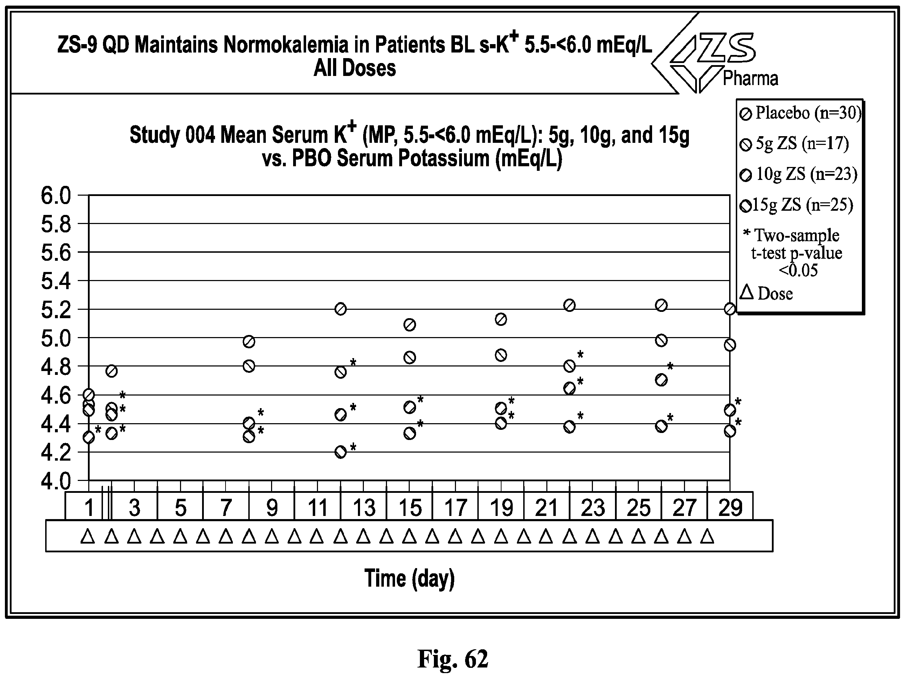

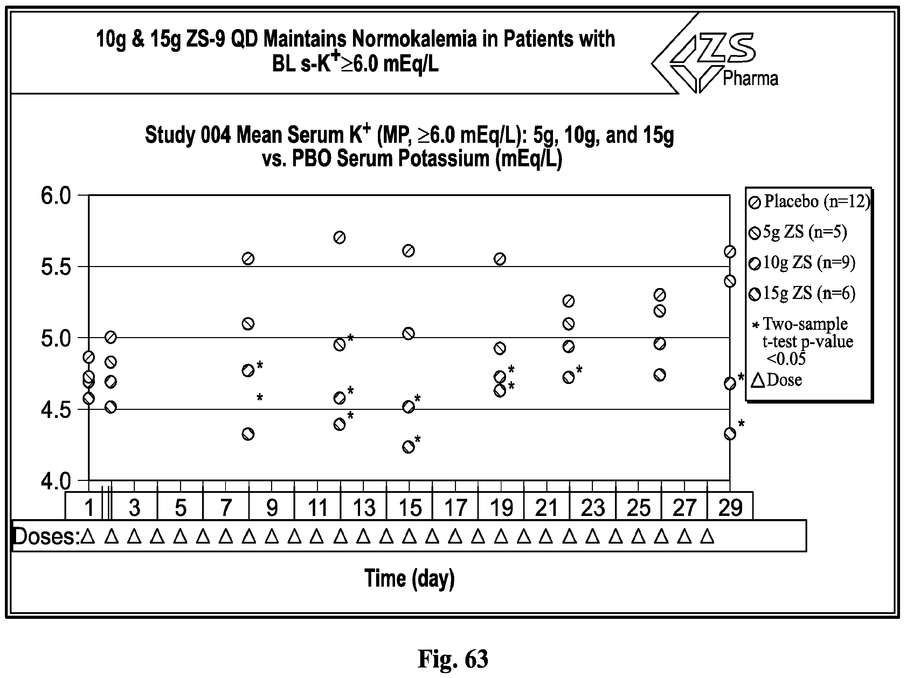

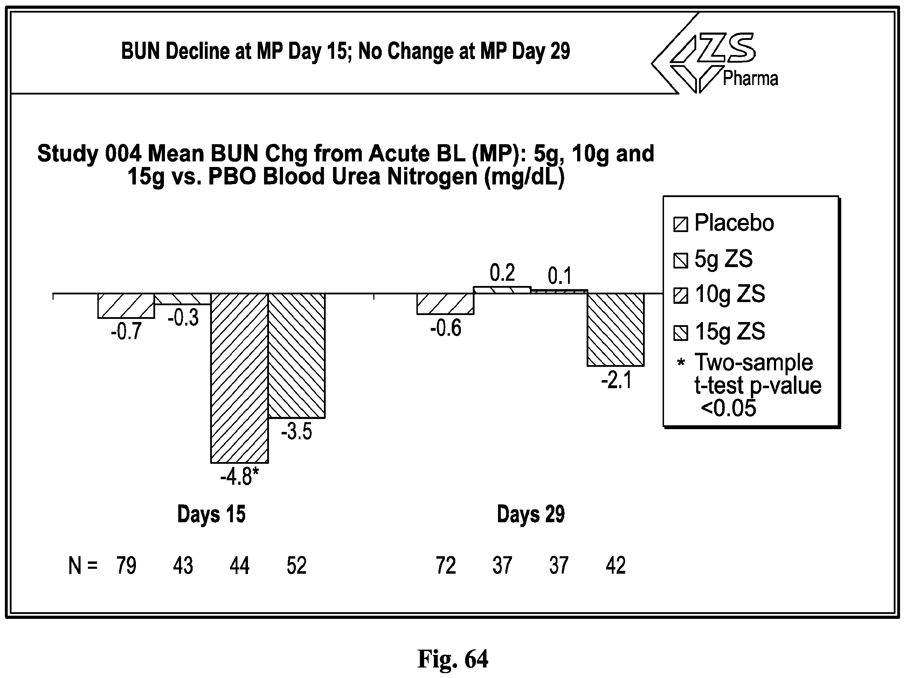

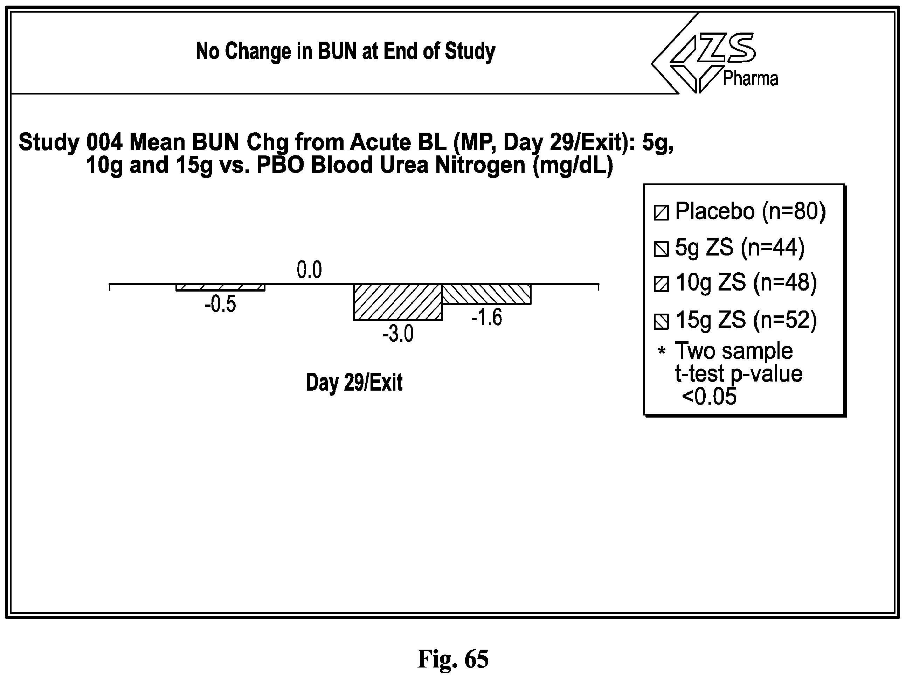

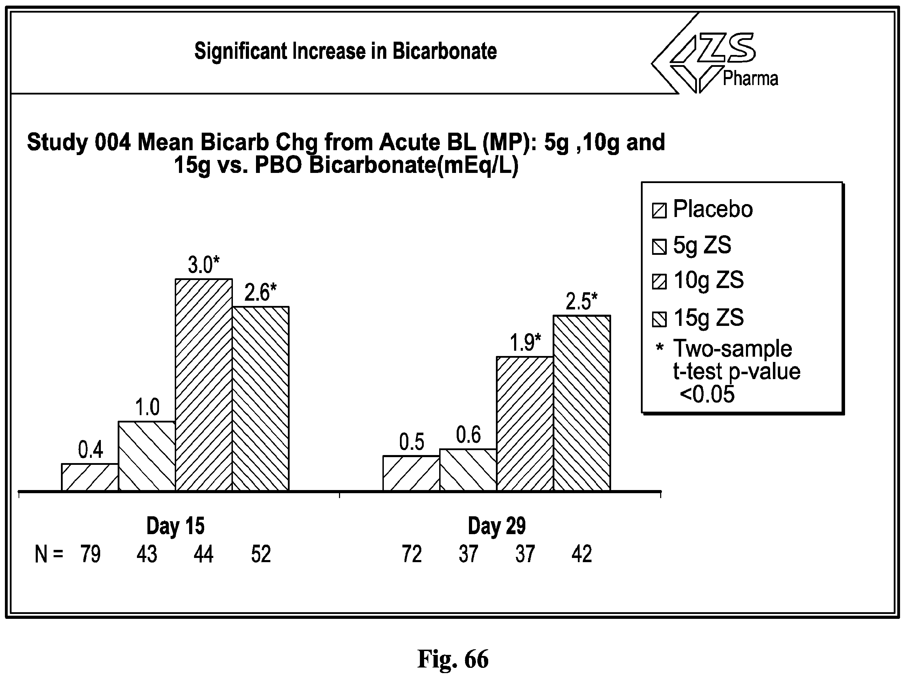

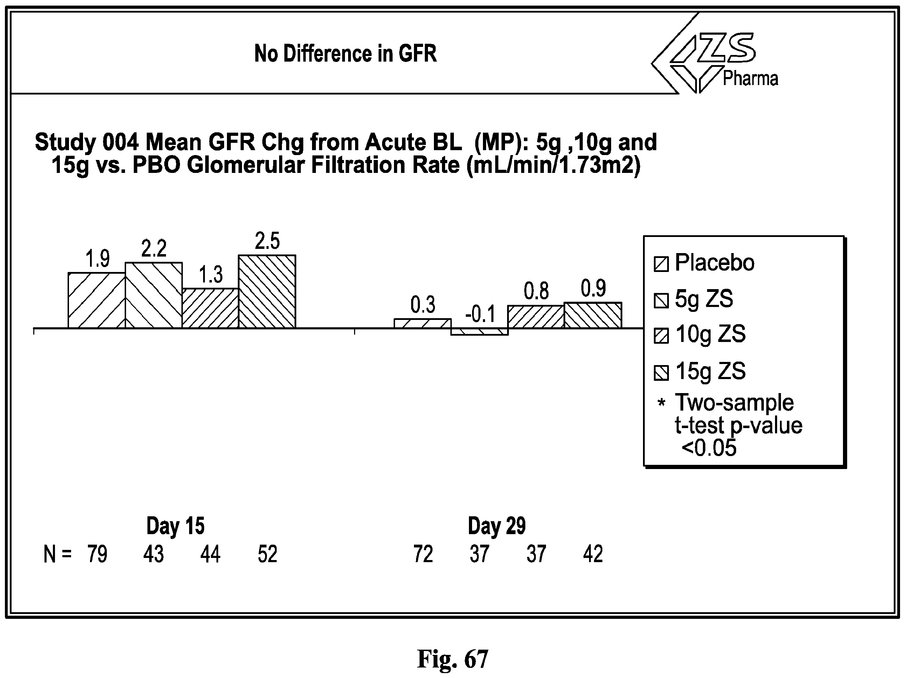

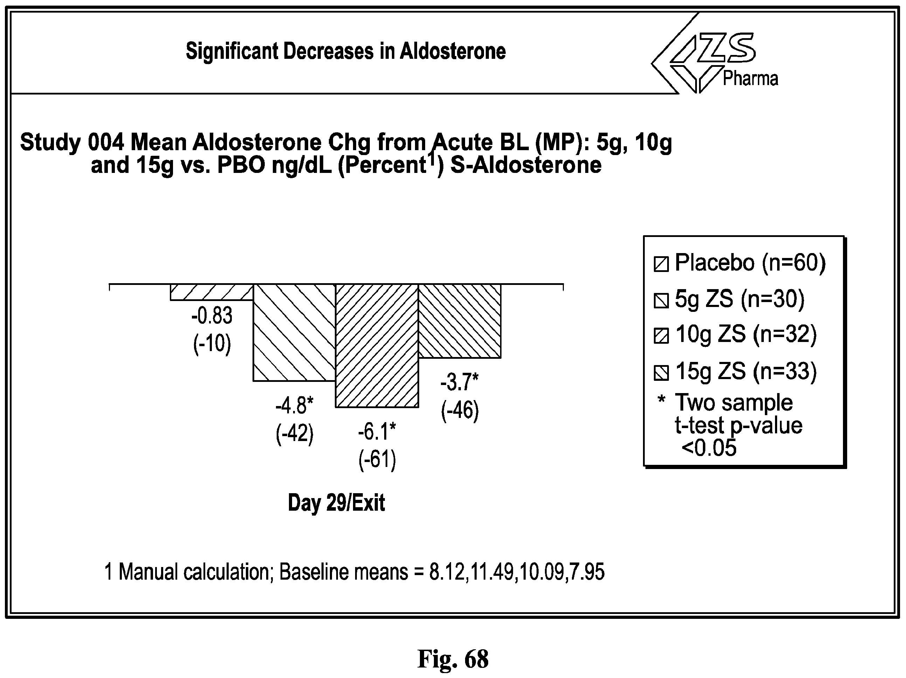

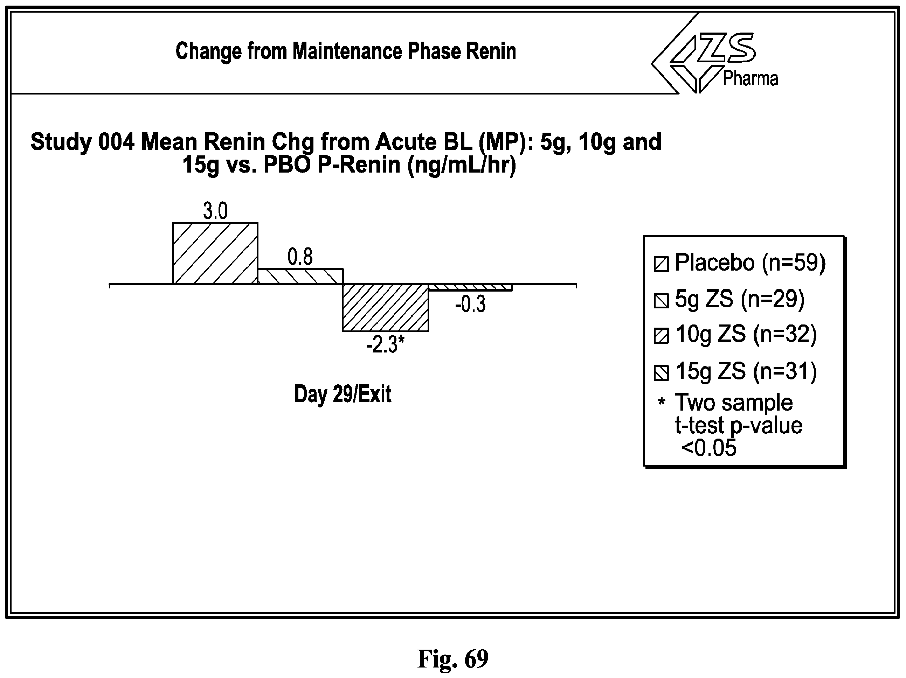

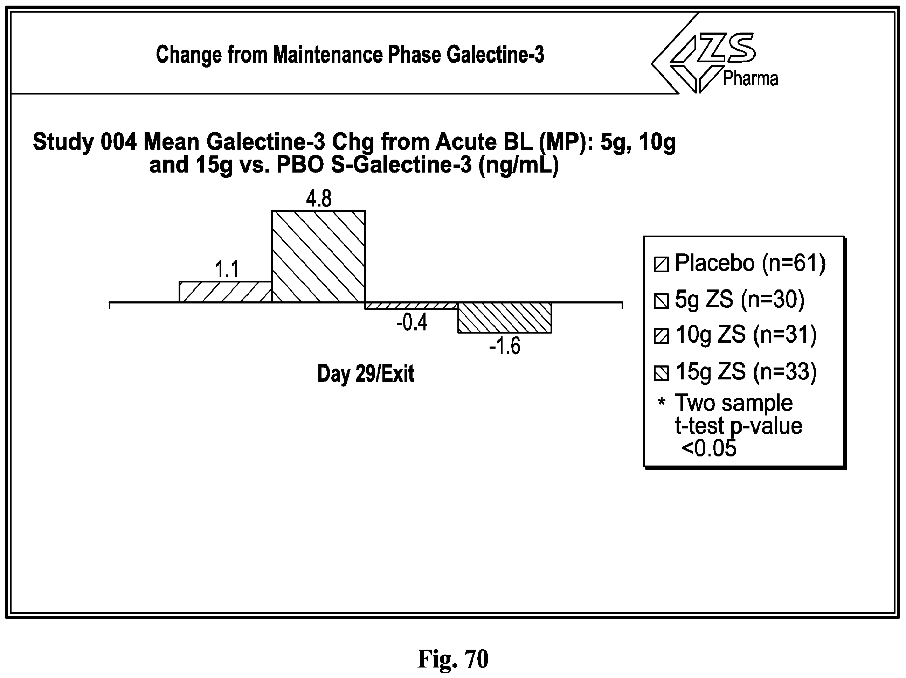

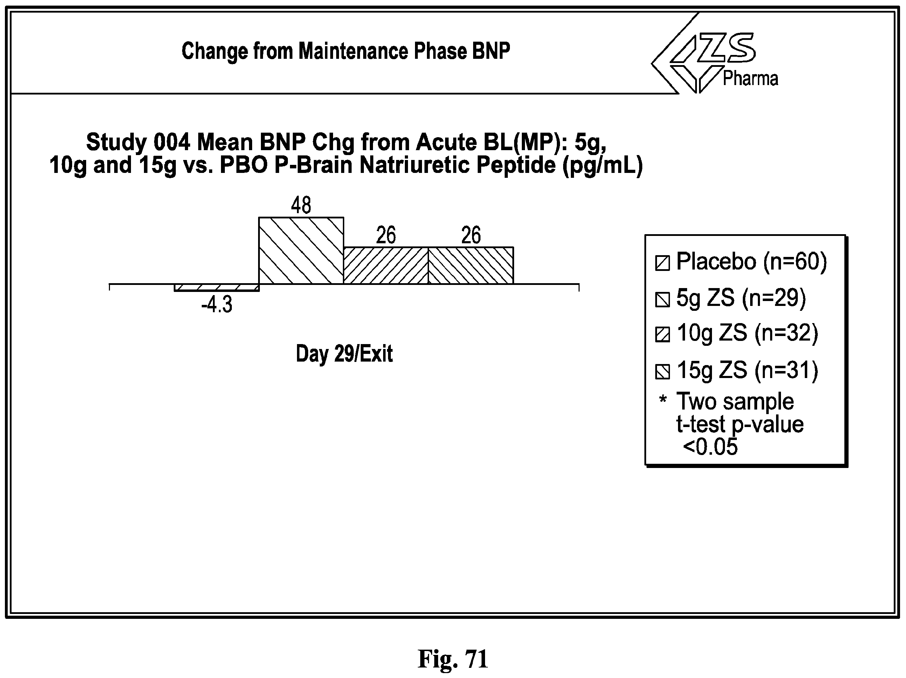

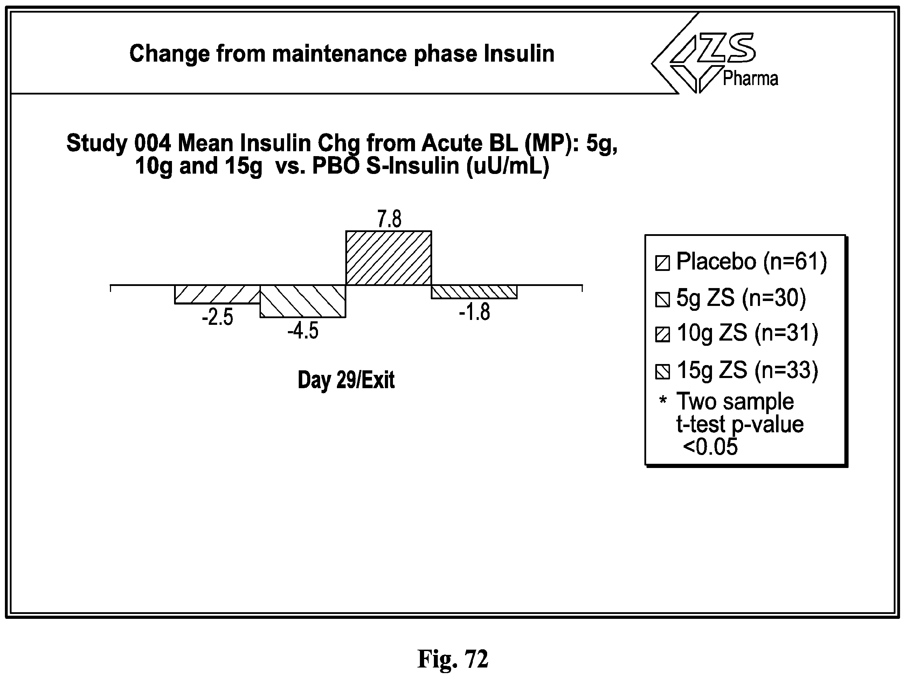

Fig. 62 shows that ZS when administered QD maintains normalkalemia at 5, 10, and 15 g doses. When patients exhibit serum potassium exceeding 6 mmol/g normalkalemia is achieved at the 10 and 15 g doses as shown in Fig. 63. The patients exhibited no change in BUN at Day 29 as shown in Figs. 64-65. The patients exhibited a significant increase in bicarbonate at day 15 and 29 as shown in Fig. 66. Fig. 67 shows there was no difference in GFR. The patient exhibited a significant decrease in aldosterone as shown in Fig. 68. The change from maintenance phase renin is shown in Fig. 69. The change from maintenance phase Gelectine-3 is shown in Fig. 70. The change in maintenance phase BNP is shown in Fig. 71. The change in maintenance phase for insulin is shown in Fig. 72.

**EXAMPLE 32**

The following example relates to the manufacture of various ZS compositions described herein into tablet formulations.

Final tablet formulation components are listed below (Table 11)

| TABLE 11: TABLET FROMULATION | | | | |
|---|---|---|---|---|
| COMPONENTS | % w/w | 500 mg TABLETS | 1000 mg TABLETS | |
| Zirconium Silicate | 66.67 | 500.00 | 1000.00 | |
| Hydroxypropyl cellulose (NF/EP) | 7.41 | 55.60 | 111.20 | |
| Silicified microcrystalline cellulose, USP/NF* | 20.42 | 153.15 | 306.30 | |
| Crospovidone (NF/EP) | 5.00 | 37.50 | 75.0 | |
| Magnesium stearate (NF/EP) | 0.50 | 3.75 | 7.50 | |
| TOTAL | 100% | 750.00 mg | 1500.00 mg | |
| * = Silicified microcrystalline cellulose, USP/NF consists of microcrystalline cellulose, NF/EP and silica, colloidal anhydrous, EP. | | | | |

ZS tablets are manufactured into either 500 or 1000 mg tablets using a high shear granulation process followed by blending and compression into the desired tablet form. The process begins by screening ZS and hydroxypropyl cellulose (NF/EP) through a 20-mesh screen with an optional step of weighing. The screened components are charged into a high shear granulator and dry mixed for approximately 3 minutes with the impellar set at approximately 150 rpm. Following the dry mixing, the chopper is set at 2000 rpm and USP purified water is charged into the granulator over a period of 5 minutes. The granulated mixture is discharged and milled followed by charging into a fluid bed dryer with an inlet air temperature of approximately 60 degrees C until the product reaches a temperature of 52 degrees C. The material continues to dry until the moisture content is less than or equal to approximately 2.5%. Once the desired moisture content is achieved, the product is cooled to a temperature of approximately less than 30 degrees C.

The cooled material is discharged from the fluid bed dryer, milled, and added to a diffusion mixer and mixed with a silicified microcrystalline cellulose (NF) and crospovidone (NF/EP) blend for approximately 10 minutes. Magnesium stearate (NF/EP, bovine free) is added to the mixer and the contents are blended for an additional 3 minutes. The blended mixture is compressed into 500 mg tablets using a 0.3300 inch X 0.6600 inch modified oval b tooling or into 1000 mg tablets using a 0.4600 inch X 0.8560 inch modified oval D tooling.

The quality attributes that are analysed on the final tablet include the following parameters: appearance, XRD identification, average tablet weight, tablet breaking force, tablet friability, KEC, dose uniformity, and disintegration. Conformance to the following criteria is required for proper quality assurance (table 12).

| TABLE 12: CRITERIA FOR QUALITY ASSURANCE | | | |
|---|---|---|---|
| TEST ATTRIBUTE | TEST METHOD REFERENCE | ACCEPTANCE CRITERIA | |
| | | 500 mg | 1000 mg |
| Appearance | n/a | White, modified oval tablet | |
| Identification: X-ray Diffraction | M-1043 | The two highest peaks occur at approximately 15.5 and 28.9, with the highest peak occurring at approximately 28.9. | |
| Average Tablet Weight | TBD | 712 mg - 788 mg (95% - 105%) | 1425 mg - 1575 mg (95% - 105%) |
| Tablet Breaking Force | TBD | 8 - 23 kp | 15 - 35 kp |
| Tablet Friability | TBD | NMT 1.0% | |
| Potassium Exchange Capacity | TM 256-012 | 2.7 - 3.7 mEq/g | |
| Dose Uniformity | TBD | Acceptance Value (AV) $\leq$ 15.0% | |

(continued)

| TABLE 12: CRITERIA FOR QUALITY ASSURANCE | | | |
|---|---|---|---|
| **TEST ATTRIBUTE** | **TEST METHOD REFERENCE** | **ACCEPTANCE CRITERIA** | |
| | | **500 mg** | **1000 mg** |
| Disintegration | TBD | NMT 15 minutes | |

Other embodiments and uses of the invention will be apparent to those skilled in the art from consideration of the specification and practice of the invention disclosed herein. All references cited herein, including all U.S. and foreign patents and patent applications, are specifically and entirely hereby incorporated herein by reference. It is intended that the specification and examples be considered exemplary only, with the true scope and spirit of the invention indicated by the following claims.

**Claims**

1. A zirconium silicate composition comprising zirconium silicate of formula (I):

$$A_p M_x Zr_{1-x} Si_n Ge_y O_m \qquad (I)$$

where

A is a potassium ion, sodium ion, rubidium ion, cesium ion, calcium ion, magnesium ion, hydronium ion or mixtures thereof,
M is at least one framework metal, wherein the framework metal is hafnium (4+), tin (4+), niobium (5+), titanium (4+), cerium (4+), germanium (4+), praseodymium (4+), terbium (4+) or mixtures thereof,

"p" has a value from about 1 to about 20,
"x" has a value from 0 to less than 1,
"n" has a value from about 1 to about 12,
"y" has a value from 0 to about 12,
"m" has a value from about 3 to about 36 and $1 \leq n + y \leq 12$,

wherein the composition comprises at least about 95% ZS-9.

2. The composition of claim 1, wherein the ZS-9 has an X-ray diffraction pattern of

| d(Å) | I |
|---|---|
| 5.9-6.7 | m |
| 5.3-6.1 | m-s |
| 2.7-3.5 | vs |
| 2.0-2.8 | w-m |
| 1.6-2.4 | w |

3. The composition of claim 1, wherein the composition comprises ZS-7 having an X-ray diffraction pattern of

| d(Å) | I |
|---|---|
| 6.8-7.6 | vs |
| 5.6-6.4 | m |

EP 4 378 577 A2

(continued)

| d(Å) | I |
|---|---|
| 3.7-4.5 | m |
| 3.6-4.4 | m |
| 2.6-3.4 | s-vs |
| 2.5-3.3 | m |
| 2.4-3.2 | vs |

4. The composition of claim 1, wherein the composition does not comprise ZS-8 having an X-ray diffraction pattern of

| d(Å) | I |
|---|---|
| 12.0-13.2 | vs |
| 3.9-4.7 | m |
| 2.8-3.6 | m |
| 2.3-3.1 | m |
| 2.2-3.0 | w |
| 2.1-2.9 | w |

5. The composition of claim 1, wherein the composition comprises ZS-9 at a weight percent ranging from about 95% to 100%.

6. The composition of claim 1, wherein the composition comprises ZS-7 at a weight percent ranging from about 0% to about 5%.

7. The composition of claim 1, wherein the ZS-9 exhibits a median particle size of greater than 3 microns and less than 7% of the particles in the composition have a diameter less than 3 microns, and the composition exhibits a sodium content below 12% by weight.

8. The composition of claim 1, wherein the ZS-9 is partially protonated.

9. The composition of claim 1, wherein the protonated ZS-9 has a potassium exchange rate of greater than 3.1 meq/g.

10. The composition of claim 1, wherein the protonated ZS-9 has a potassium exchange rate in the range of 3.2 to 3.5 meq/g.

11. The composition of claim 1, wherein the protonated ZS-9 has a potassium exchange rate of > 2.46 meq/g.

12. The composition of claim 1, wherein the protonated ZS-9 has a sodium content of less than 12%.

13. A powdered pharmaceutical cation exchange composition comprising ZS-9 having an X-ray diffraction pattern generated using a copper K-alpha radiation source of:

| d(Å) |
|---|
| 5.9-6.7 |
| 5.3-6.1 |
| 2.7-3.5 |
| 2.0-2.8 |

43

(continued)

| d(Å) |
|------|
| 1.6-2.4 |

wherein the composition comprises at least 95% ZS-9.

14. An zirconium silicate composition comprising zirconium silicate of formula (I):

$$A_pM_xZr_{1-x}Si_nGe_yO_m \qquad (I)$$

where

A is a potassium ion, sodium ion, rubidium ion, cesium ion, calcium ion, magnesium ion, hydronium ion or mixtures thereof,
M is at least one framework metal, wherein the framework metal is hafnium (4+), tin (4+), niobium (5+), titanium (4+), cerium (4+), germanium (4+), praseodymium (4+), terbium (4+) or mixtures thereof,
"p" has a value from about 1 to about 20,
"x" has a value from 0 to less than 1,
"n" has a value from about 1 to about 12,
"y" has a value from 0 to about 12,
"m" has a value from about 3 to about 36 and $1 \leq n + y \leq 12$,

wherein the zirconium silicate comprises at least about 95% ZS-9.

15. The composition of claim 14, wherein the ZS-9 has an X-ray diffraction pattern of

| d(Å) | I |
|------|---|
| 5.9-6.7 | m |
| 5.3-6.1 | m-s |
| 2.7-3.5 | vs |
| 2.0-2.8 | w-m |
| 1.6-2.4 | w |

16. The composition of claim 14, wherein the zirconium silicate comprises ZS-7 having an X-ray diffraction pattern of

| d(Å) | I |
|------|---|
| 6.8-7.6 | vs |
| 5.6-6.4 | m |
| 3.7-4.5 | m |
| 3.6-4.4 | m |
| 2.6-3.4 | s-vs |
| 2.5-3.3 | m |
| 2.4-3.2 | vs |

17. The composition of claim 14, wherein the zirconium silicate does not comprise ZS-8 having an X-ray diffraction pattern of

| d(Å) | I |
|---|---|
| 12.0-13.2 | vs |
| 3.9-4.7 | m |
| 2.8-3.6 | m |
| 2.3-3.1 | m |
| 2.2-3.0 | w |
| 2.1-2.9 | w |

18. The composition of claim 14, wherein the composition comprises ZS-9 at a weight percent ranging from about 95% to about 100%.

19. The composition of claim 14, wherein the composition comprises ZS-7 at a weight percent ranging from 0% to about 5%.

20. The composition of claim 14, wherein the ZS-9 exhibits a median particle size of greater than 3 microns and less than 7% of the particles in the composition have a diameter less than 3 microns, and the composition exhibits a sodium content below 12% by weight.

21. The composition of claim 14, wherein the ZS-9 is partially protonated.

22. The composition of claim 14, wherein the protonated ZS-9 has a potassium exchange rate of greater than 3.1 meq/g.

23. The composition of claim 14, wherein the protonated ZS-9 has a potassium exchange rate in the range of 3.2 to 3.5 meq/g.

24. The composition of claim 14, wherein the protonated ZS-9 has a potassium exchange rate of > 2.46 meq/g.

25. The composition of claim 14, wherein the protonated ZS-9 has a sodium content of less than 12%.

26. A method for treatment of hyperkalemia comprising administering a particulate pharmaceutical composition comprising a zirconium silicate of formula (I):

$$A_pM_xZr_{1-x}Si_nGe_yO_m \qquad (I)$$

where

A is a potassium ion, sodium ion, rubidium ion, cesium ion, calcium ion, magnesium ion, hydronium ion or mixtures thereof,
M is at least one framework metal, wherein the framework metal is hafnium (4+), tin (4+), niobium (5+), titanium (4+), cerium (4+), germanium (4+), praseodymium (4+), terbium (4+) or mixtures thereof,
"p" has a value from about 1 to about 20,
"x" has a value from 0 to less than 1,
"n" has a value from 1 to about 12,
"y" has a value from 0 to about 12,
"m" has a value from about 3 to about 36 and $1 \leq n + y \leq 12$,
wherein the particles exhibit a uniform microporous structure and a median particle size of greater than 3 microns and less than 7% of the particles in the composition have a diameter less than 3 microns, the composition exhibits a sodium content below 12% by weight, and wherein the zirconium silicate in the composition is at least 95% ZS-9.

27. The method of claim 26, wherein the sodium content is less than 6% by weight.

**28.** The method of claim 26, wherein the sodium content is between 0.05 to 3% by weight.

**29.** The method of claim 26, wherein the sodium content is less than 0.01% by weight.

**30.** The method of claim 26, wherein less than 3% of the particles in the composition have a diameter less than 3 microns.

**31.** The method of claim 26, wherein less than 2.5% of the particles in the composition have a diameter less than 3 microns.

**32.** The method of claim 26, wherein the median particle size ranges from 5 to 1000 microns.

**33.** The method of claim 26, wherein the median particle size ranges from 20 to 100 microns.

**34.** The method of claim 26, wherein the composition exhibits an x-ray powder diffraction pattern generated using a copper K-alpha radiation source comprising:

a first peak at 2 theta corresponding to a first d-spacing within the range of 2.7-3.5 angstroms, and
a second peak at 2 theta corresponding to a second d-spacing within the range of 5.3-6.1 angstroms.

**35.** The method of claim 26, wherein the patient is suffering from acute hyperkalemia.

**36.** The method of claim 35, wherein the patient is administered a dose of approximately 0.7 to 1,500 mg/Kg/day.

**37.** The method of claim 35, wherein the patient is administered a dose of approximately 500 to 1,000 mg/Kg/day.

**38.** The method of claim 35, wherein the patient is administered a dose of approximately 700 mg/Kg/day.

**39.** The method of claim 26, wherein the patient is suffering from chronic hyperkalemia.

**40.** The method of claim 39, wherein the patient is administered a dose of approximately 0.25 to 100 mg/Kg/day.

**41.** The method of claim 39, wherein the patient is administered a dose of approximately 50 mg/Kg/day.

**42.** The method of claim 26, wherein the patient is at risk for congestive heart failure.

**43.** The method of claim 26, wherein the patient has edema from elevated sodium levels.

**44.** The method of claim 26, wherein the FTIR spectra of the composition does not include absorption peaks at approximately 764 cm$^{-1}$.

**45.** The method of claim 26, wherein the composition does not indicate peaks at 2-theta values of 7.5, 32, or 42.5 angstroms when the x-ray diffraction spectrum is generated using a copper K-alpha radiation source.

**46.** The method of claim 26, wherein the composition exhibits a median particle size of greater than 3 microns and less than 2% of the particles in the composition have a diameter less than 3 microns.

**47.** The method of claim 26, wherein the composition exhibits a particle size in the range of about 5 to about 200 microns.

**48.** The method of claim 26, wherein the composition administered is a powered composition.

**49.** The method of claim 26, wherein the composition is formed into a shaped article.

**50.** The method of claim 49, wherein the shaped article is a tablet.

**51.** The method of claim 26, wherein the x-ray powder diffraction pattern further comprises:

a third peak at 2 theta corresponding to a third d-spacing within the range of 5.9-6.7 angstroms,
a fourth peak at 2 theta corresponding to a fourth d-spacing within the range of 2.0-2.8 angstroms, and

a fifth peak at 2 theta corresponding to a fifth d-spacing within the range of 1.6-2.4 angstroms,
the third, fourth, and fifth peaks each have intensity values that are lower than the first and second intensity values.

52. A method for treatment of hyperkalemia comprising administering to a subject in need thereof an effective amount of a powdered composition comprising ZS-9 having an x-ray diffraction pattern of:

| d(Å) |
| --- |
| 5.9-6.7 |
| 5.3-6.1 |
| 2.7-3.5 |
| 2.0-2.8 |
| 1.6-2.4 |

wherein the ZS-9 exhibits a uniform microporous structure and the composition exhibits a median particle size of greater than 3 microns and less than 3% of the particles in the composition have a diameter less than 3 microns, the composition exhibits a sodium content below 12% by weight, and wherein the zirconium silicate in the composition is at least 95% ZS-9.

53. The method of claim 52, wherein the composition further exhibits an x-ray powder diffraction pattern generated using a copper K-alpha radiation source with at least a third peak at 2 theta corresponding to a third d-spacing within the range of 5.9-6.7 angstroms, and a fourth peak at 2 theta corresponding to a fourth d-spacing within the range of 2.0-2.8 angstroms, the third peak having the third greatest relative intensity within the diffraction pattern, and the fourth peak having the fourth greatest relative intensity within the diffraction pattern.

Dark = ZrO3 (oct), Light = SiO2 (tet), Cations not shown

**Fig. 1**

| Particle Name:<br>ZS-9(0.001) | Accessory Name:<br>Hydro 2000S (A) | Pump Speed:<br>2000    RPM | Analysis model:<br>General purpose | Sensitivity:<br>Normal |
|---|---|---|---|---|
| Particle RI:<br>1.550 | Absorption:<br>0.001 | | Size range:<br>0.020  to  2000.000  um | Obscuration:<br>15.49   % |
| Dispersant Name:<br>Water | Dispersant RI:<br>1.330 | | Weighted Residual:<br>0.626      % | Result Emulation:<br>Off |

| Concentration:<br>0.0192  %Vol | | Span:<br>3.269 | | Uniformity:<br>1.04 | | Result units:<br>Volume |
|---|---|---|---|---|---|---|
| Specific Surface Area:<br>0.636   m²/g | | Surface Weighted Mean D[3,2]:<br>9.433   um | | Vol. Weighted Mean D[4,3]:<br>24.964   um | | |
| d(0.1):   3.803   um | | | d(0.5):   16.407   um | | d(0.9):   57.436   um | |

Particle Size Distribution

ZS-9 - Average, Wednesday, January 12, 2011 5:01:56 PM

| Size (µm) | Vol Under % | Size (µm) | Vol Under % | Size (µm) | Vol Under % | Size (µm) | Vol Under % | Size (µm) | Vol Under % | Size (µm) | Vol Under % |
|---|---|---|---|---|---|---|---|---|---|---|---|
| 0.020 | 0.00 | 0.142 | 0.00 | 1.002 | 0.00 | 7.096 | 24.52 | 50.238 | 86.92 | 355.656 | 100.00 |
| 0.022 | 0.00 | 0.159 | 0.00 | 1.125 | 0.00 | 7.962 | 27.66 | 56.368 | 89.60 | 399.052 | 100.00 |
| 0.025 | 0.00 | 0.178 | 0.00 | 1.262 | 0.01 | 8.934 | 30.91 | 63.246 | 91.92 | 447.744 | 100.00 |
| 0.028 | 0.00 | 0.200 | 0.00 | 1.416 | 0.07 | 10.024 | 34.28 | 70.963 | 93.88 | 502.377 | 100.00 |
| 0.032 | 0.00 | 0.224 | 0.00 | 1.589 | 0.31 | 11.247 | 37.76 | 79.621 | 95.49 | 563.677 | 100.00 |
| 0.036 | 0.00 | 0.252 | 0.00 | 1.783 | 0.76 | 12.619 | 41.36 | 89.337 | 96.79 | 632.456 | 100.00 |
| 0.040 | 0.00 | 0.283 | 0.00 | 2.000 | 1.44 | 14.159 | 45.08 | 100.237 | 97.81 | 709.627 | 100.00 |
| 0.045 | 0.00 | 0.317 | 0.00 | 2.244 | 2.38 | 15.887 | 48.91 | 112.468 | 98.58 | 796.214 | 100.00 |
| 0.050 | 0.00 | 0.356 | 0.00 | 2.518 | 3.59 | 17.825 | 52.84 | 126.191 | 99.14 | 893.367 | 100.00 |
| 0.056 | 0.00 | 0.399 | 0.00 | 2.825 | 5.06 | 20.000 | 56.87 | 141.589 | 99.53 | 1002.374 | 100.00 |
| 0.063 | 0.00 | 0.448 | 0.00 | 3.170 | 6.79 | 22.440 | 60.95 | 158.866 | 99.79 | 1124.683 | 100.00 |
| 0.071 | 0.00 | 0.502 | 0.00 | 3.557 | 8.75 | 25.179 | 65.05 | 178.250 | 99.93 | 1261.915 | 100.00 |
| 0.080 | 0.00 | 0.564 | 0.00 | 3.991 | 10.94 | 28.251 | 69.12 | 200.000 | 99.98 | 1415.892 | 100.00 |
| 0.089 | 0.00 | 0.632 | 0.00 | 4.477 | 13.33 | 31.698 | 73.10 | 224.404 | 100.00 | 1588.656 | 100.00 |
| 0.100 | 0.00 | 0.710 | 0.00 | 5.024 | 15.90 | 35.566 | 76.93 | 251.785 | 100.00 | 1782.502 | 100.00 |
| 0.112 | 0.00 | 0.796 | 0.00 | 5.637 | 18.64 | 39.905 | 80.55 | 282.508 | 100.00 | 2000.000 | 100.00 |
| 0.126 | 0.00 | 0.893 | 0.00 | 6.325 | 21.51 | 44.774 | 83.69 | 316.979 | 100.00 | | |

**Fig. 2 (ZS-9 lot 5332-04310-A)**

| Particle Name: | Accessory Name: | Pump Speed: | | Analysis model: | Sensitivity: |
|---|---|---|---|---|---|
| ZS-9(0.001) | Hydro 2000S (A) | 2000 RPM | | General purpose | Normal |
| Particle RI: | Absorption: | | | Size range: | Obscuration: |
| 1.550 | 0.001 | | | 0.020 to 2000.000 um | 15.11 % |
| Dispersant Name: | Dispersant RI: | | | Weighted Residual: | Result Emulation: |
| Water | 1.330 | | | 0.777 % | Off |

| Concentration: | Span: | Uniformity: | Result units: |
|---|---|---|---|
| 0.0172 %Vol | 3.059 | 0.9.84 | Volume |

| Specific Surface Area: | Surface Weighted Mean D[3,2]: | Vol. Weighted Mean D[4,3]: | |
|---|---|---|---|
| 0.703 m²/g | 8.538 um | 19.297 um | |

| d(0.1): 3.713 um | | d(0.5): 12.825 um | | d(0.9): 42.950 um |
|---|---|---|---|---|

### Particle Size Distribution

ZS-9 - Average, Thursday, Janaury 13, 2011 11:09:02 AM

| Size (µm) | Vol Under % | Size (µm) | Vol Under % | Size (µm) | Vol Under % | Size (µm) | Vol Under % | Size (µm) | Vol Under % | Size (µm) | Vol Under % |
|---|---|---|---|---|---|---|---|---|---|---|---|
| 0.020 | 0.00 | 0.142 | 0.00 | 1.002 | 0.00 | 7.096 | 28.93 | 50.238 | 93.13 | 355.656 | 99.98 |
| 0.022 | 0.00 | 0.159 | 0.00 | 1.125 | 0.00 | 7.962 | 32.89 | 56.368 | 94.96 | 399.052 | 99.99 |
| 0.025 | 0.00 | 0.178 | 0.00 | 1.262 | 0.00 | 8.934 | 36.94 | 63.246 | 96.42 | 447.744 | 100.00 |
| 0.028 | 0.00 | 0.200 | 0.00 | 1.416 | 0.00 | 10.024 | 41.06 | 70.963 | 97.54 | 502.377 | 100.00 |
| 0.032 | 0.00 | 0.224 | 0.00 | 1.589 | 0.16 | 11.247 | 45.22 | 79.621 | 98.37 | 563.677 | 100.00 |
| 0.036 | 0.00 | 0.252 | 0.00 | 1.783 | 0.51 | 12.619 | 49.41 | 89.337 | 98.96 | 632.456 | 100.00 |
| 0.040 | 0.00 | 0.283 | 0.00 | 2.000 | 1.11 | 14.159 | 53.62 | 100.237 | 99.37 | 709.627 | 100.00 |
| 0.045 | 0.00 | 0.317 | 0.00 | 2.244 | 2.00 | 15.887 | 57.85 | 112.468 | 99.64 | 796.214 | 100.00 |
| 0.050 | 0.00 | 0.356 | 0.00 | 2.518 | 3.22 | 17.825 | 62.08 | 126.191 | 99.81 | 893.367 | 100.00 |
| 0.056 | 0.00 | 0.399 | 0.00 | 2.825 | 4.80 | 20.000 | 66.27 | 141.589 | 99.89 | 1002.374 | 100.00 |
| 0.063 | 0.00 | 0.448 | 0.00 | 3.170 | 6.74 | 22.440 | 70.41 | 158.866 | 99.92 | 1124.683 | 100.00 |
| 0.071 | 0.00 | 0.502 | 0.00 | 3.557 | 9.04 | 25.179 | 74.44 | 178.250 | 99.93 | 1261.915 | 100.00 |
| 0.080 | 0.00 | 0.564 | 0.00 | 3.991 | 11.69 | 28.251 | 78.30 | 200.000 | 99.94 | 1415.892 | 100.00 |
| 0.089 | 0.00 | 0.632 | 0.00 | 4.477 | 14.88 | 31.698 | 81.93 | 224.404 | 99.95 | 1588.656 | 100.00 |
| 0.100 | 0.00 | 0.710 | 0.00 | 5.024 | 17.91 | 35.566 | 85.27 | 251.785 | 99.96 | 1782.502 | 100.00 |
| 0.112 | 0.00 | 0.796 | 0.00 | 5.637 | 21.40 | 39.905 | 88.28 | 282.508 | 99.97 | 2000.000 | 100.00 |
| 0.126 | 0.00 | 0.893 | 0.00 | 6.325 | 25.08 | 44.774 | 90.90 | 316.979 | 99.97 | | |

**Fig. 3 (ZS-9 lot 5332-15410-A)**

| Particle Name: | Accessory Name: | Pump Speed: | | Analysis model: | Sensitivity: |
|---|---|---|---|---|---|
| ZS-9(0.001) | Hydro 2000S (A) | 2000 RPM | | General purpose | Normal |
| **Particle RI:** | **Absorption:** | | | **Size range:** | **Obscuration:** |
| 1.550 | 0.001 | | | 0.020 to 2000.000 um | 15.25 % |
| **Dispersant Name:** | **Dispersant RI:** | | | **Weighted Residual:** | **Result Emulation:** |
| Water | 1.330 | | | 0.458 % | Off |

| Concentration: | Span: | Uniformity: | Result units: |
|---|---|---|---|
| 0.0256 %Vol | 2.003 | 0.613 | Volume |

| **Specific Surface Area:** | **Surface Weighted Mean D[3,2]:** | **Vol. Weighted Mean D[4,3]:** |
|---|---|---|
| 0.515 m²/g | 11.644 um | 17.741 um |

**d(0.1): 5.999 um**  **d(0.5): 14.374 um**  **d(0.9): 34.795 um**

ZS-9 - Average, Wednesday, January 12, 2011 4:37:09 PM

| Size (µm) | Vol Under % | Size (µm) | Vol Under % | Size (µm) | Vol Under % | Size (µm) | Vol Under % | Size (µm) | Vol Under % | Size (µm) | Vol Under % |
|---|---|---|---|---|---|---|---|---|---|---|---|
| 0.020 | 0.00 | 0.142 | 0.00 | 1.002 | 0.00 | 7.096 | 15.63 | 50.238 | 98.00 | 355.656 | 100.00 |
| 0.022 | 0.00 | 0.159 | 0.00 | 1.125 | 0.00 | 7.962 | 20.25 | 56.368 | 99.32 | 399.052 | 100.00 |
| 0.025 | 0.00 | 0.178 | 0.00 | 1.262 | 0.00 | 8.934 | 25.40 | 63.246 | 99.88 | 447.744 | 100.00 |
| 0.028 | 0.00 | 0.200 | 0.00 | 1.416 | 0.00 | 10.024 | 30.98 | 70.963 | 100.00 | 502.377 | 100.00 |
| 0.032 | 0.00 | 0.224 | 0.00 | 1.589 | 0.00 | 11.247 | 36.88 | 79.621 | 100.00 | 563.677 | 100.00 |
| 0.036 | 0.00 | 0.252 | 0.00 | 1.783 | 0.00 | 12.619 | 42.99 | 89.337 | 100.00 | 632.456 | 100.00 |
| 0.040 | 0.00 | 0.283 | 0.00 | 2.000 | 0.00 | 14.159 | 49.19 | 100.237 | 100.00 | 709.627 | 100.00 |
| 0.045 | 0.00 | 0.317 | 0.00 | 2.244 | 0.00 | 15.887 | 55.36 | 112.468 | 100.00 | 796.214 | 100.00 |
| 0.050 | 0.00 | 0.356 | 0.00 | 2.518 | 0.00 | 17.825 | 61.42 | 126.191 | 100.00 | 893.367 | 100.00 |
| 0.056 | 0.00 | 0.399 | 0.00 | 2.825 | 0.09 | 20.000 | 67.28 | 141.589 | 100.00 | 1002.374 | 100.00 |
| 0.063 | 0.00 | 0.448 | 0.00 | 3.170 | 0.34 | 22.440 | 72.82 | 158.866 | 100.00 | 1124.683 | 100.00 |
| 0.071 | 0.00 | 0.502 | 0.00 | 3.557 | 0.96 | 25.179 | 78.00 | 178.250 | 100.00 | 1261.915 | 100.00 |
| 0.080 | 0.00 | 0.564 | 0.00 | 3.991 | 1.97 | 28.251 | 82.75 | 200.000 | 100.00 | 1415.892 | 100.00 |
| 0.089 | 0.00 | 0.632 | 0.00 | 4.477 | 3.47 | 31.698 | 86.99 | 224.404 | 100.00 | 1588.656 | 100.00 |
| 0.100 | 0.00 | 0.710 | 0.00 | 5.024 | 5.55 | 35.566 | 90.65 | 251.785 | 100.00 | 1782.502 | 100.00 |
| 0.112 | 0.00 | 0.796 | 0.00 | 5.637 | 8.26 | 39.905 | 93.72 | 282.508 | 100.00 | 2000.000 | 100.00 |
| 0.126 | 0.00 | 0.893 | 0.00 | 6.325 | 11.62 | 44.774 | 96.15 | 316.979 | 100.00 | | |

**Fig. 4 (ZS-9 preclinical lot)**

| Serial Number: S3238 | | | | | | | | | | | |
|---|---|---|---|---|---|---|---|---|---|---|---|
| Range: 0.687 -995.6 um | | | *MICROTRAC – S3000* | | | | | | Ver: 9.0h Meas : Original | | |

**MICROTRAC – S3000**

Ver: 9.0h
Meas : Original

ZS9

R/M
LOT: 5332-04310A

Date: 02/04/11 Meas #: 205
Time: 10:39 Pres #: 1

POWDERSIZE, INC.
20 PACIFIC DRIVE
QUAKERTOWN, PA 18951
TEL: 215-536-5605

OPERATOR: HM
PSI: S0072

| Summary | Percentiles | | Dia | Vol% | Width |
|---|---|---|---|---|---|
| mv = 52.85 | 10% = 5.808 | 60% = 39.45 | 28.24 | 100% | 87.87 |
| mn = 3.428 | 20% = 9.597 | 70% = 54.83 | | | |
| ma = 14.62 | 30% = 14.36 | 80% = 79.46 | | | |
| cs = 0.410 | 40% = 20.25 | 90% = 136.7 | | | |
| sd = 43.93 | 50% = 28.24 | 95% = 197.5 | | | |

| SIZE | %PASS | %CHAN | SIZE | %PASS | %CHAN | SIZE | %PASS | %CHAN | SIZE | %PASS | %CHAN |
|---|---|---|---|---|---|---|---|---|---|---|---|
| 995.6 | 100.00 | 0.00 | 114.1 | 87.15 | 1.51 | 13.08 | 27.53 | 2.21 | 1.499 | 0.00 | 0.00 |
| 913.0 | 100.00 | 0.00 | 104.6 | 85.64 | 1.62 | 12.00 | 25.32 | 2.13 | 1.375 | 0.00 | 0.00 |
| 837.2 | 100.00 | 0.00 | 95.96 | 84.02 | 1.75 | 11.00 | 23.19 | 2.05 | 1.261 | 0.00 | 0.00 |
| 767.7 | 100.00 | 0.00 | 88.00 | 82.27 | 1.90 | 10.09 | 21.14 | 1.97 | 1.156 | 0.00 | 0.00 |
| 704.0 | 100.00 | 0.00 | 80.70 | 80.37 | 2.07 | 9.250 | 19.17 | 1.90 | 1.060 | 0.00 | 0.00 |
| 645.6 | 100.00 | 0.00 | 74.00 | 78.30 | 2.23 | 8.482 | 17.27 | 1.83 | 0.972 | 0.00 | 0.00 |
| 592.0 | 100.00 | 0.00 | 67.86 | 76.07 | 2.38 | 7.778 | 15.44 | 1.74 | 0.892 | 0.00 | 0.00 |
| 542.9 | 100.00 | 0.00 | 62.23 | 73.69 | 2.50 | 7.133 | 13.70 | 1.64 | 0.818 | 0.00 | 0.00 |
| 497.8 | 100.00 | 0.00 | 57.06 | 71.19 | 2.59 | 6.541 | 12.06 | 1.53 | 0.750 | 0.00 | 0.00 |
| 456.5 | 100.00 | 0.00 | 52.32 | 68.60 | 2.63 | 5.998 | 10.53 | 1.40 | | | |
| 418.6 | 100.00 | 0.18 | 47.98 | 65.97 | 2.64 | 5.500 | 9.13 | 1.28 | | | |
| 383.9 | 99.82 | 0.29 | 44.00 | 63.33 | 2.64 | 5.043 | 7.85 | 1.15 | | | |
| 352.0 | 99.53 | 0.36 | 40.35 | 60.69 | 2.62 | 4.625 | 6.70 | 1.04 | | | |
| 322.8 | 99.17 | 0.45 | 37.00 | 58.07 | 2.60 | 4.241 | 5.66 | 0.93 | | | |
| 296.0 | 98.72 | 0.58 | 33.93 | 55.47 | 2.58 | 3.889 | 4.73 | 0.83 | | | |
| 271.4 | 98.14 | 0.70 | 31.11 | 52.89 | 2.58 | 3.566 | 3.90 | 0.74 | | | |
| 248.9 | 97.44 | 0.83 | 28.53 | 50.31 | 2.59 | 3.270 | 3.16 | 0.65 | | | |
| 228.2 | 96.61 | 0.93 | 26.16 | 47.72 | 2.60 | 2.999 | 2.51 | 0.57 | | | |
| 209.3 | 95.68 | 1.03 | 23.99 | 45.12 | 2.62 | 2.750 | 1.94 | 0.49 | | | |
| 191.9 | 94.65 | 1.10 | 22.00 | 42.50 | 2.62 | 2.522 | 1.45 | 0.41 | | | |
| 176.0 | 93.55 | 1.16 | 20.17 | 39.88 | 2.60 | 2.312 | 1.04 | 0.34 | | | |
| 161.4 | 92.39 | 1.22 | 18.50 | 37.28 | 2.55 | 2.121 | 0.70 | 0.29 | | | |
| 148.0 | 91.17 | 1.27 | 16.96 | 34.73 | 2.49 | 1.945 | 0.41 | 0.25 | | | |
| 135.7 | 89.90 | 1.34 | 15.56 | 32.24 | 2.40 | 1.783 | 0.16 | 0.16 | | | |
| 124.5 | 88.56 | 1.41 | 14.27 | 29.84 | 2.31 | 1.635 | 0.00 | 0.00 | | | |

| | | |
|---|---|---|
| Distribution: Volume | Run Time: 10 seconds | Fluid: ISOPAR G |
| Progression: Geometric Root8 | Run Number 1 of 1 runs | Fluid Refractive Index: 1.42 |
| Upper Edge: 995.6 | Particle: DefaultParticle | Loading Factor: 0.1198 |
| Lower Edge: 0.687 | Particle Transparency: Trans | Transmission: 0.96 |
| Residuals: Disabled | Particle Refractive Index: 1.51 | Above Residual: 0.00 |
| Number Of Channels: 84 | Particle Shape: Irregular | Below Residual: 0.00 |

Filter: On

Database Path: C:\MTWIN90\MT2_2011.DB

**Fig. 5 (lot 5332-04310A w/o screening)**

| Serial Number: S3238 | | | | | | | | | | | |
|---|---|---|---|---|---|---|---|---|---|---|---|
| Range: 0.687 -995.6 um | | *MICROTRAC - S3000* | | | | | | Ver: 9.0h | | | |
| | | | | | | | | Meas : Original | | | |
| ZS9 | | | | > 635 MESH | | | | Date: 02/04/11 Meas #: 209 | | | |
| | | | | LOT: 5332-04310A | | | | Time: 10:52 Pres #: 1 | | | |

POWDERSIZE, INC.
20 PACIFIC DRIVE
QUAKERTOWN, PA 18951
TEL: 215-536-5605

OPERATOR: HM
PSI: S0072

**Summary**
mv = 59.18
mn = 3.413
ma = 15.18
cs = 0.395
sd = 45.17

**Percentiles**
10% = 5.998  60% = 40.51
20% = 10.16  70% = 55.59
30% = 15.40  80% = 80.63
40% = 21.66  90% = 159.8
50% = 29.69  95% = 250.3

| Dia | Vol% | Width |
|---|---|---|
| 29.69 | 100% | 90.35 |

| SIZE | %PASS | %CHAN | SIZE | %PASS | %CHAN | SIZE | %PASS | %CHAN | SIZE | %PASS | %CHAN |
|---|---|---|---|---|---|---|---|---|---|---|---|
| 995.6 | 100.00 | 0.00 | 114.1 | 86.22 | 1.26 | 13.08 | 25.81 | 2.08 | 1.499 | 0.00 | 0.00 |
| 913.0 | 100.00 | 0.00 | 104.6 | 84.96 | 1.45 | 12.00 | 23.73 | 1.98 | 1.375 | 0.00 | 0.00 |
| 837.2 | 100.00 | 0.00 | 95.96 | 83.51 | 1.64 | 11.00 | 21.75 | 1.90 | 1.261 | 0.00 | 0.00 |
| 767.7 | 100.00 | 0.00 | 88.00 | 81.87 | 1.85 | 10.09 | 19.85 | 1.83 | 1.156 | 0.00 | 0.00 |
| 704.0 | 100.00 | 0.00 | 80.70 | 80.02 | 2.05 | 9.250 | 18.02 | 1.77 | 1.060 | 0.00 | 0.00 |
| 645.6 | 100.00 | 0.00 | 74.00 | 77.97 | 2.24 | 8.482 | 16.25 | 1.69 | 0.972 | 0.00 | 0.00 |
| 592.0 | 100.00 | 0.00 | 67.86 | 75.73 | 2.40 | 7.778 | 14.56 | 1.62 | 0.892 | 0.00 | 0.00 |
| 542.9 | 100.00 | 0.00 | 62.23 | 73.33 | 2.54 | 7.133 | 12.94 | 1.52 | 0.818 | 0.00 | 0.00 |
| 497.8 | 100.00 | 0.17 | 57.06 | 70.79 | 2.64 | 6.541 | 11.42 | 1.42 | 0.750 | 0.00 | 0.00 |
| 456.5 | 99.83 | 0.30 | 52.32 | 68.15 | 2.72 | 5.998 | 10.00 | 1.31 | | | |
| 418.6 | 99.53 | 0.39 | 47.98 | 65.43 | 2.77 | 5.500 | 8.69 | 1.20 | | | |
| 383.9 | 99.14 | 0.53 | 44.00 | 62.66 | 2.79 | 5.043 | 7.49 | 1.08 | | | |
| 352.0 | 98.61 | 0.71 | 40.35 | 59.87 | 2.79 | 4.625 | 6.41 | 0.98 | | | |
| 322.8 | 97.90 | 0.88 | 37.00 | 57.08 | 2.79 | 4.241 | 5.43 | 0.88 | | | |
| 296.0 | 97.02 | 1.01 | 33.93 | 54.29 | 2.79 | 3.889 | 4.55 | 0.79 | | | |
| 271.4 | 96.01 | 1.08 | 31.11 | 51.50 | 2.78 | 3.566 | 3.76 | 0.71 | | | |
| 248.9 | 94.93 | 1.07 | 28.53 | 48.72 | 2.76 | 3.270 | 3.05 | 0.62 | | | |
| 228.2 | 93.86 | 1.03 | 26.16 | 45.96 | 2.75 | 2.999 | 2.43 | 0.55 | | | |
| 209.3 | 92.83 | 0.96 | 23.99 | 43.21 | 2.72 | 2.750 | 1.88 | 0.47 | | | |
| 191.9 | 91.87 | 0.90 | 22.00 | 40.49 | 2.67 | 2.522 | 1.41 | 0.40 | | | |
| 176.0 | 90.97 | 0.87 | 20.17 | 37.82 | 2.61 | 2.312 | 1.01 | 0.33 | | | |
| 161.4 | 90.10 | 0.87 | 18.50 | 35.21 | 2.52 | 2.121 | 0.68 | 0.28 | | | |
| 148.0 | 89.23 | 0.91 | 16.96 | 32.69 | 2.41 | 1.945 | 0.40 | 0.24 | | | |
| 135.7 | 88.32 | 0.99 | 15.56 | 30.28 | 2.29 | 1.783 | 0.16 | 0.16 | | | |
| 124.5 | 87.33 | 1.11 | 14.27 | 27.99 | 2.18 | 1.635 | 0.00 | 0.00 | | | |

Distribution: Volume
Progression: Geometric Root8
Upper Edge: 995.6
Lower Edge: 0.687
Residuals: Disabled
Number Of Channels: 84

Run Time: 10 seconds
Run Number 1 of 1 runs
Particle: DefaultParticle
Particle Transparency: Trans
Particle Refractive Index: 1.51
Particle Shape: Irregular

Fluid: ISOPAR G
Fluid Refractive Index: 1.42
Loading Factor: 0.1627
Transmission: 0.94
Above Residual: 0.00
Below Residual: 0.00

Filter: On

Database Path: C:\MTWIN90\MT2_2011.DB

**Fig. 6 (lot 5332-04310A 635 mesh)**

| Serial Number: S3238 | *MICROTRAC - S3000* | | Ver: 9.0h |
| Range: 0.687 -995.6 um | | | Meas : Original |

| ZS9 | > 450 MESH | Date: 02/04/11 Meas #: 211 |
| | LOT: 5332-04310A | Time: 11:12  Pres #: 1 |

POWDERSIZE, INC.
20 PACIFIC DRIVE
QUAKERTOWN, PA 18951
TEL: 215-536-5605

OPERATOR: HM
PSI: S0072

| Summary | | Percentiles | | Dia | Vol% | Width |
|---|---|---|---|---|---|---|
| mv = 72.51 | | 10% = 7.046 | 60% = 57.46 | 197.2 | 20% | 164.8 |
| mn = 3.331 | | 20% = 13.63 | 70% = 73.25 | 34.24 | 80% | 59.47 |
| ma = 18.80 | | 30% = 23.33 | 80% = 104.8 | | | |
| cs = 0.319 | | 40% = 34.26 | 90% = 197.5 | | | |
| sd = 61.59 | | 50% = 45.39 | 95% = 259.4 | | | |

| SIZE | %PASS | %CHAN | SIZE | %PASS | %CHAN | SIZE | %PASS | %CHAN | SIZE | %PASS | %CHAN |
|---|---|---|---|---|---|---|---|---|---|---|---|
| 995.6 | 100.00 | 0.00 | 114.1 | 81.54 | 1.58 | 13.08 | 19.32 | 1.40 | 1.499 | 0.00 | 0.00 |
| 913.0 | 100.00 | 0.00 | 104.6 | 79.96 | 1.84 | 12.00 | 17.92 | 1.37 | 1.375 | 0.00 | 0.00 |
| 837.2 | 100.00 | 0.00 | 95.96 | 78.12 | 2.18 | 11.00 | 16.55 | 1.34 | 1.261 | 0.00 | 0.00 |
| 767.7 | 100.00 | 0.00 | 88.00 | 75.94 | 2.58 | 10.09 | 15.21 | 1.32 | 1.156 | 0.00 | 0.00 |
| 704.0 | 100.00 | 0.00 | 80.70 | 73.36 | 2.97 | 9.250 | 13.89 | 1.29 | 1.060 | 0.00 | 0.00 |
| 645.6 | 100.00 | 0.00 | 74.00 | 70.39 | 3.34 | 8.482 | 12.60 | 1.24 | 0.972 | 0.00 | 0.00 |
| 592.0 | 100.00 | 0.00 | 67.86 | 67.05 | 3.60 | 7.778 | 11.36 | 1.19 | 0.892 | 0.00 | 0.00 |
| 542.9 | 100.00 | 0.00 | 62.23 | 63.45 | 3.75 | 7.133 | 10.17 | 1.14 | 0.818 | 0.00 | 0.00 |
| 497.8 | 100.00 | 0.15 | 57.06 | 59.70 | 3.77 | 6.541 | 9.03 | 1.07 | 0.750 | 0.00 | 0.00 |
| 456.5 | 99.85 | 0.25 | 52.32 | 55.93 | 3.66 | 5.998 | 7.96 | 1.00 | | | |
| 418.6 | 99.60 | 0.35 | 47.98 | 52.27 | 3.50 | 5.500 | 6.96 | 0.92 | | | |
| 383.9 | 99.25 | 0.51 | 44.00 | 48.77 | 3.26 | 5.043 | 6.04 | 0.85 | | | |
| 352.0 | 98.74 | 0.73 | 40.35 | 45.51 | 3.03 | 4.625 | 5.19 | 0.77 | | | |
| 322.8 | 98.01 | 0.99 | 37.00 | 42.48 | 2.78 | 4.241 | 4.42 | 0.70 | | | |
| 296.0 | 97.02 | 1.27 | 33.93 | 39.70 | 2.56 | 3.889 | 3.72 | 0.63 | | | |
| 271.4 | 95.75 | 1.47 | 31.11 | 37.14 | 2.35 | 3.566 | 3.09 | 0.57 | | | |
| 248.9 | 94.28 | 1.60 | 28.53 | 34.79 | 2.17 | 3.270 | 2.52 | 0.50 | | | |
| 228.2 | 92.68 | 1.63 | 26.16 | 32.62 | 2.01 | 2.999 | 2.02 | 0.45 | | | |
| 209.3 | 91.05 | 1.55 | 23.99 | 30.61 | 1.87 | 2.750 | 1.57 | 0.39 | | | |
| 191.9 | 89.50 | 1.45 | 22.00 | 28.74 | 1.76 | 2.522 | 1.18 | 0.33 | | | |
| 176.0 | 88.05 | 1.34 | 20.17 | 26.98 | 1.66 | 2.312 | 0.85 | 0.28 | | | |
| 161.4 | 86.71 | 1.26 | 18.50 | 25.32 | 1.58 | 2.121 | 0.57 | 0.23 | | | |
| 148.0 | 85.45 | 1.24 | 16.96 | 23.74 | 1.52 | 1.945 | 0.34 | 0.21 | | | |
| 135.7 | 84.21 | 1.28 | 15.56 | 22.22 | 1.47 | 1.783 | 0.13 | 0.13 | | | |
| 124.5 | 82.93 | 1.39 | 14.27 | 20.75 | 1.43 | 1.635 | 0.00 | 0.00 | | | |

Distribution: Volume
Progression: Geometric Root8
Upper Edge: 995.6
Lower Edge: 0.687
Residuals: Disabled
Number Of Channels: 84

Run Time: 10 seconds
Run Number 1 of 1 runs
Particle: DefaultParticle
Particle Transparency: Trans
Particle Refractive Index: 1.51
Particle Shape: Irregular

Fluid: ISOPAR G
Fluid Refractive Index: 1.42
Loading Factor: 0.1028
Transmission: 0.97
Above Residual: 0.00
Below Residual: 0.00

Filter: On

Database Path: C:\MTWIN90\MT2_2011.DB

**Fig. 7 (lot 5332-04310A 450 mesh)**

| Serial Number: S3238 | **MICROTRAC – S3000** | | Ver: 9.0h |
|---|---|---|---|
| Range: 0.687 -995.6 um | | | Meas : Original |

| ZS9 | >325 MESH | Date: 02/04/11 Meas #: 214 |
|---|---|---|
| | LOT: 5332-04310A | Time: 11:32 Pres #: 1 |

| POWDERSIZE, INC. | OPERATOR: HM | Summary | Percentiles | Dia | Vol% | Width |
|---|---|---|---|---|---|---|
| 20 PACIFIC DRIVE | PSI: S0072 | mv = 101.0 | 10% = 32.90 60% = 99.71 | 84.61 | 100% | 113.0 |
| QUAKERTOWN, PA 18951 | | mn = 4.942 | 20% = 49.23 70% = 118.2 | | | |
| TEL: 215-536-5605 | | ma = 47.84 | 30% = 60.46 80% = 143.2 | | | |
| | | cs = 0.125 | 40% = 71.77 90% = 188.0 | | | |
| | | sd = 56.52 | 50% = 84.61 95% = 238.3 | | | |

| SIZE | %PASS | %CHAN | SIZE | %PASS | %CHAN | SIZE | %PASS | %CHAN | SIZE | %PASS | %CHAN |
|---|---|---|---|---|---|---|---|---|---|---|---|
| 995.6 | 100.00 | 0.00 | 114.1 | 68.01 | 5.11 | 13.08 | 4.48 | 0.33 | 1.499 | 0.00 | 0.00 |
| 913.0 | 100.00 | 0.00 | 104.6 | 62.90 | 5.22 | 12.00 | 4.15 | 0.31 | 1.375 | 0.00 | 0.00 |
| 837.2 | 100.00 | 0.00 | 95.96 | 57.68 | 5.28 | 11.00 | 3.84 | 0.30 | 1.261 | 0.00 | 0.00 |
| 767.7 | 100.00 | 0.00 | 88.00 | 52.40 | 5.28 | 10.09 | 3.54 | 0.30 | 1.156 | 0.00 | 0.00 |
| 704.0 | 100.00 | 0.00 | 80.70 | 47.12 | 5.26 | 9.250 | 3.24 | 0.29 | 1.060 | 0.00 | 0.00 |
| 645.6 | 100.00 | 0.00 | 74.00 | 41.86 | 5.21 | 8.482 | 2.95 | 0.28 | 0.972 | 0.00 | 0.00 |
| 592.0 | 100.00 | 0.00 | 67.86 | 36.65 | 5.03 | 7.778 | 2.67 | 0.27 | 0.892 | 0.00 | 0.00 |
| 542.9 | 100.00 | 0.00 | 62.23 | 31.62 | 4.73 | 7.133 | 2.40 | 0.27 | 0.818 | 0.00 | 0.00 |
| 497.8 | 100.00 | 0.15 | 57.06 | 26.89 | 4.26 | 6.541 | 2.13 | 0.26 | 0.750 | 0.00 | 0.00 |
| 456.5 | 99.85 | 0.25 | 52.32 | 22.63 | 3.65 | 5.998 | 1.87 | 0.25 | | | |
| 418.6 | 99.60 | 0.32 | 47.98 | 18.98 | 3.02 | 5.500 | 1.62 | 0.25 | | | |
| 383.9 | 99.28 | 0.41 | 44.00 | 15.96 | 2.36 | 5.043 | 1.37 | 0.23 | | | |
| 352.0 | 98.87 | 0.55 | 40.35 | 13.60 | 1.82 | 4.625 | 1.14 | 0.22 | | | |
| 322.8 | 98.32 | 0.70 | 37.00 | 11.78 | 1.38 | 4.241 | 0.92 | 0.21 | | | |
| 296.0 | 97.62 | 0.88 | 33.93 | 10.40 | 1.05 | 3.889 | 0.71 | 0.19 | | | |
| 271.4 | 96.74 | 1.10 | 31.11 | 9.35 | 0.82 | 3.566 | 0.52 | 0.19 | | | |
| 248.9 | 95.64 | 1.35 | 28.53 | 8.53 | 0.67 | 3.270 | 0.33 | 0.19 | | | |
| 228.2 | 94.29 | 1.67 | 26.16 | 7.86 | 0.57 | 2.999 | 0.14 | 0.14 | | | |
| 209.3 | 92.62 | 2.05 | 23.99 | 7.29 | 0.49 | 2.750 | 0.00 | 0.00 | | | |
| 191.9 | 90.57 | 2.49 | 22.00 | 6.80 | 0.45 | 2.522 | 0.00 | 0.00 | | | |
| 176.0 | 88.08 | 3.01 | 20.17 | 6.35 | 0.41 | 2.312 | 0.00 | 0.00 | | | |
| 161.4 | 85.07 | 3.55 | 18.50 | 5.94 | 0.39 | 2.121 | 0.00 | 0.00 | | | |
| 148.0 | 81.52 | 4.09 | 16.96 | 5.55 | 0.37 | 1.945 | 0.00 | 0.00 | | | |
| 135.7 | 77.43 | 4.54 | 15.56 | 5.18 | 0.36 | 1.783 | 0.00 | 0.00 | | | |
| 124.5 | 72.89 | 4.88 | 14.27 | 4.82 | 0.34 | 1.635 | 0.00 | 0.00 | | | |

| Distribution: Volume | Run Time: 10 seconds | Fluid: ISOPAR G |
|---|---|---|
| Progression: Geometric Root8 | Run Number 1 of 1 runs | Fluid Refractive Index: 1.42 |
| Upper Edge: 995.6 | Particle: DefaultParticle | Loading Factor: 0.3073 |
| Lower Edge: 0.687 | Particle Transparency: Trans | Transmission: 0.95 |
| Residuals: Disabled | Particle Refractive Index: 1.51 | Above Residual: 0.00 |
| Number Of Channels: 84 | Particle Shape: Irregular | Below Residual: 0.00 |
| Filter: On | Database Path: C:\MTWIN90\MT2_2011.DB | |

**Fig. 8 (lot 5332-04310A 325 mesh)**

| Serial Number: S3238 | | | MICROTRAC – S3000 | | | | Ver: 9.0h | | |
|---|---|---|---|---|---|---|---|---|---|
| Range: 0.687 -995.6 um | | | | | | | Meas : Original | | |
| | ZS9 | | | | > 230 MESH | | Date: 02/04/11 Meas #: 219 | | |
| | | | | | LOT: 5332-04310A | | Time: 12:03 Pres #: 1 | | |

POWDERSIZE, INC.
20 PACIFIC DRIVE
QUAKERTOWN, PA 18951
TEL: 215-536-5605

OPERATOR: HM
PSI: S0072

Summary
mv = 173.2
mn = 97.19
ma = 138.1
cs = 0.043
sd = 85.77

Percentiles
10% = 86.70  60% = 166.1
20% = 102.0  70% = 199.0
30% = 114.9  80% = 246.1
40% = 128.4  90% = 306.3
50% = 144.3  95% = 351.0

Dia   Vol%   Width
144.3  100%   171.5

| SIZE | %PASS | %CHAN | SIZE | %PASS | %CHAN | SIZE | %PASS | %CHAN | SIZE | %PASS | %CHAN |
|---|---|---|---|---|---|---|---|---|---|---|---|
| 995.6 | 100.00 | 0.00 | 114.1 | 29.36 | 7.39 | 13.08 | 0.00 | 0.00 | 1.499 | 0.00 | 0.00 |
| 913.0 | 100.00 | 0.00 | 104.6 | 21.97 | 6.27 | 12.00 | 0.00 | 0.00 | 1.375 | 0.00 | 0.00 |
| 837.2 | 100.00 | 0.00 | 95.96 | 15.70 | 5.01 | 11.00 | 0.00 | 0.00 | 1.261 | 0.00 | 0.00 |
| 767.7 | 100.00 | 0.00 | 88.00 | 10.69 | 3.64 | 10.09 | 0.00 | 0.00 | 1.156 | 0.00 | 0.00 |
| 704.0 | 100.00 | 0.00 | 80.70 | 7.05 | 2.55 | 9.250 | 0.00 | 0.00 | 1.060 | 0.00 | 0.00 |
| 645.6 | 100.00 | 0.00 | 74.00 | 4.50 | 1.71 | 8.482 | 0.00 | 0.00 | 0.972 | 0.00 | 0.00 |
| 592.0 | 100.00 | 0.11 | 67.86 | 2.79 | 1.11 | 7.778 | 0.00 | 0.00 | 0.892 | 0.00 | 0.00 |
| 542.9 | 99.89 | 0.23 | 62.23 | 1.68 | 0.72 | 7.133 | 0.00 | 0.00 | 0.818 | 0.00 | 0.00 |
| 497.8 | 99.66 | 0.38 | 57.06 | 0.96 | 0.46 | 6.541 | 0.00 | 0.00 | 0.750 | 0.00 | 0.00 |
| 456.5 | 99.28 | 0.74 | 52.32 | 0.50 | 0.32 | 5.998 | 0.00 | 0.00 | | | |
| 418.6 | 98.54 | 1.36 | 47.98 | 0.18 | 0.18 | 5.500 | 0.00 | 0.00 | | | |
| 383.9 | 97.18 | 2.09 | 44.00 | 0.00 | 0.00 | 5.043 | 0.00 | 0.00 | | | |
| 352.0 | 95.09 | 2.97 | 40.35 | 0.00 | 0.00 | 4.625 | 0.00 | 0.00 | | | |
| 322.8 | 92.12 | 3.58 | 37.00 | 0.00 | 0.00 | 4.241 | 0.00 | 0.00 | | | |
| 296.0 | 88.54 | 3.92 | 33.93 | 0.00 | 0.00 | 3.889 | 0.00 | 0.00 | | | |
| 271.4 | 84.62 | 4.08 | 31.11 | 0.00 | 0.00 | 3.566 | 0.00 | 0.00 | | | |
| 248.9 | 80.54 | 4.02 | 28.53 | 0.00 | 0.00 | 3.270 | 0.00 | 0.00 | | | |
| 228.2 | 76.52 | 4.08 | 26.16 | 0.00 | 0.00 | 2.999 | 0.00 | 0.00 | | | |
| 209.3 | 72.44 | 4.25 | 23.99 | 0.00 | 0.00 | 2.750 | 0.00 | 0.00 | | | |
| 191.9 | 68.19 | 4.68 | 22.00 | 0.00 | 0.00 | 2.522 | 0.00 | 0.00 | | | |
| 176.0 | 63.51 | 5.35 | 20.17 | 0.00 | 0.00 | 2.312 | 0.00 | 0.00 | | | |
| 161.4 | 58.16 | 6.15 | 18.50 | 0.00 | 0.00 | 2.121 | 0.00 | 0.00 | | | |
| 148.0 | 52.01 | 7.11 | 16.96 | 0.00 | 0.00 | 1.945 | 0.00 | 0.00 | | | |
| 135.7 | 44.90 | 7.67 | 15.56 | 0.00 | 0.00 | 1.783 | 0.00 | 0.00 | | | |
| 124.5 | 37.23 | 7.87 | 14.27 | 0.00 | 0.00 | 1.635 | 0.00 | 0.00 | | | |

Distribution: Volume
Progression: Geometric Root8
Upper Edge: 995.6
Lower Edge: 0.687
Residuals: Disabled
Number Of Channels: 84

Run Time: 10 seconds
Run Number 1 of 1 runs
Particle: DefaultParticle
Particle Transparency: Trans
Particle Refractive Index: 1.51
Particle Shape: Irregular

Fluid: ISOPAR G
Fluid Refractive Index: 1.42
Loading Factor: 0.3758
Transmission: 0.98
Above Residual: 0.00
Below Residual: 0.00

Filter: On

Database Path: C:\MTWIN90\MT2_2011.DB

**Fig. 9 (lot 5332-04310A 230 mesh)**

**Fig. 10: XRD plot for ZS-9 prepared in accordance with Example 12.**

c:\pel_data\spectra\scan172.sp - 4-23-11-GMP

**Fig. 11: FTIR plot for ZS-9 prepared in accordance with Example 12.**

File Name    : Cedarburg Hauser\43557A
Sample Name  : 2724-18F                    Comment : ZS-9              1st  Scan
Date & Time  : 01-13-12 16:07:20
Condition
  X-ray Tube : Cu(1.54060 A)    Voltage : 40.0 kV    Current : 35.0 mA
  Scan Range : 4.0000 <-> 45.0000 deg    Step Size : 0.0200 deg
  Count Time : 0.60 sec    Slit  DS : 1.00 deg  SS : 1.00 deg  RS : 0.30 mm

**Fig. 12: XRD plot for ZA09 prepared in accordance with Example 14.**

Fig. 13: FTIR plot for ZS09 prepared in accordance with Example 14.

Fig. 14: Example of the Blank Solution Chromatogram

Fig. 15: Example of the Assay Standard Solution Chromatogram.

61

**Fig. 16: Exemplary Sample Chromatogram.**

## TOP VIEW

100

101

102

## SIDE VIEW

100

101

103    102

Fig. 17: 200-L reaction vessel with standard agitator arrangement.

## TOP VIEW

## SIDE VIEW

**Fig. 18: 200-L reaction vessel with baffles for production of enhanced ZS-9**

Fig. 19: Detail of baffle design for 200-L reaction vessel for production of enhanced ZS-9

Fig. 20: Serum K levels in the first 48 hours after ingestion of Placebo or ZS

Wilcoxon-Gehan Test P-value: 0.042

Fig. 21: Time of serum K decrease of placebo vs. ZS at 10 g tib.

**Fig. 22: The rate of serum K increase following ZS administration**

**Fig. 23: Rate of urine K excretion**

**Fig. 24: Daily urinary sodium excretion**

EP 4 378 577 A2

```
File Name    : Sovereign Pharma\44621-B
Sample Name  : Lot 5567-26812-A          Comment : ZS-Powders        1st  run
Date & Time  : 11-01-12 10:59:21
Condition
  X-ray Tube : Cu(1.54060 A)    Voltage : 40.0 kV    Current : 35.0 mA
  Scan Range : 4.0000 <-> 45.0000 deg    Step Size : 0.0200 deg
  Count Time : 0.60 sec    Slit  DS : 1.00 deg  SS : 1.00 deg  RS : 0.30 mm
```

**Fig. 25: XRD plot for H-ZS-9 prepared according to Example 20 batch 5602-26812**

```
File Name    : Sovereign Pharma\44779A
Sample Name  : Lot 5567-28312-A          Comment : ZS-Powders       1st  run
Date & Time  : 12-19-12 9:58:14
Condition
  X-ray Tube : Cu(1.54060 A)   Voltage : 40.0 kV   Current : 35.0 mA
  Scan Range : 4.0000 <-> 45.0000 deg   Step Size : 0.0200 deg
  Count Time : 0.60 sec    Slit  DS : 1.00 deg  SS : 1.00 deg  RS : 0.30 mm
```

Fig. 26: XRD plot for H-ZS-9 prepared in according to Example 20 batch 5602-28312

File Name    : Sovereign Pharma\44779B
Sample Name  : Lot 5567-29112-A          Comment : ZS-Powders        1st  run
Date & Time  : 12-19-12 10:50:11
Condition
  X-ray Tube  : Cu(1.54060 A)    Voltage : 40.0 kV    Current : 35.0 mA
  Scan Range  : 4.0000 <-> 45.0000 deg    Step Size : 0.0200 deg
  Count Time  : 0.60 sec    Slit  DS : 1.00 deg  SS : 1.00 deg  RS : 0.30 mm

Fig. 27: XRD plot for H-ZS-9 prepared in according to Example 20 batch 5602-29112

File Name     : Sovereign Pharma\44779C
Sample Name   : Lot 5567-29812-A          Comment : ZS Powders        1st  run
Date & Time   : 12-19-12 11:44:34
Condition
  X-ray Tube  : Cu(1.54060 A)    Voltage : 40.0 kV    Current : 35.0 mA
  Scan Range  : 4.0000 <-> 45.0000 deg    Step Size : 0.0200 deg
  Count Time  : 0.60 sec     Slit  DS : 1.00 deg  SS : 1.00 deg  RS : 0.30 mm

Fig. 28: XRD plot for H-ZS-9 prepared according to Example 20 batch 5602-29812.

Fig. 29: XRD data for ZS crystals produced accoridng to Example 20.

Fig. 30: XRD data ZS-8 impurities.

File Name    : ZS Pharma\46100A
Sample Name  : ZS130006 Composite
Date & Time  : 12-11-13 14:07:49
Condition
  X-ray Tube : Cu(1.54060 A)    Voltage : 40.0 kV    Current : 35.0 mA
  Scan Range : 4.0000 <-> 45.0000 deg    Step Size : 0.0200 deg
  Count Time : 0.60 sec    Slit  DS : 1.00 deg  SS : 1.00 deg  RS : 0.30 mm

Fig. 31: XRD for ZS having 95% or more ZS-9 Example 23.

| Particle Name: | Accessory Name: | Pump Speed: | | Analysis model: | Sensitivity: |
|---|---|---|---|---|---|
| ZS-9(0.001) | Hydro 2000S (A) | 2000 RPM | | General purpose | Normal |
| Particle RI: | Absorption: | | | Size range: | Obscuration: |
| 1.550 | 0.001 | | | 0.020 to 2000.000 um | 14.80 % |
| Dispersant Name: | Dispersant RI: | Average Result Records: | | Weighted Residual: | Result Emulation: |
| Water | 1.330 | | | 0.432 % | Off |

| Concentration: | | Span: | | Uniformity: | Result units: |
|---|---|---|---|---|---|
| 0.0247 %Vol | | 1.965 | | 0.605 | Volume |
| **Specific Surface Area:** | | **Surface Weighted Mean D[3,2]:** | | **Vol. Weighted Mean D[4,3]:** | |
| 0.506 m²/g | | 11.850 um | | 19.522 um | |

**d(0.1):** 6.265 um          **d(0.5):** 16.160 um          **d(0.9):** 38.013 um

### Particle Size Distribution

ZS-9, Wednesday, December 11, 2013 2:57:30 PM

| Size (μm) | Vol Under % | Size (μm) | Vol Under % | Size (μm) | Vol Under % | Size (μm) | Vol Under % | Size (μm) | Vol Under % | Size (μm) | Vol Under % |
|---|---|---|---|---|---|---|---|---|---|---|---|
| 0.020 | 0.00 | 0.142 | 0.00 | 1.002 | 0.12 | 7.096 | 13.23 | 50.238 | 96.59 | 355.858 | 100.00 |
| 0.022 | 0.00 | 0.159 | 0.00 | 1.125 | 0.19 | 7.962 | 15.82 | 56.368 | 98.25 | 399.052 | 100.00 |
| 0.025 | 0.00 | 0.178 | 0.00 | 1.262 | 0.25 | 8.934 | 21.00 | 63.246 | 99.43 | 447.744 | 100.00 |
| 0.028 | 0.00 | 0.200 | 0.00 | 1.416 | 0.31 | 10.024 | 25.75 | 70.963 | 99.99 | 502.377 | 100.00 |
| 0.032 | 0.00 | 0.224 | 0.00 | 1.589 | 0.39 | 11.247 | 31.03 | 79.621 | 100.00 | 563.677 | 100.00 |
| 0.036 | 0.00 | 0.252 | 0.00 | 1.783 | 0.48 | 12.619 | 36.74 | 89.337 | 100.00 | 632.456 | 100.00 |
| 0.040 | 0.00 | 0.283 | 0.00 | 2.000 | 0.60 | 14.159 | 42.00 | 100.237 | 100.00 | 709.627 | 100.00 |
| 0.045 | 0.00 | 0.317 | 0.00 | 2.244 | 0.77 | 15.887 | 49.06 | 112.468 | 100.00 | 796.214 | 100.00 |
| 0.050 | 0.00 | 0.356 | 0.00 | 2.518 | 0.99 | 17.825 | 55.41 | 126.191 | 100.00 | 893.367 | 100.00 |
| 0.056 | 0.00 | 0.399 | 0.00 | 2.825 | 1.30 | 20.000 | 61.69 | 141.589 | 100.00 | 1002.374 | 100.00 |
| 0.063 | 0.00 | 0.448 | 0.00 | 3.170 | 1.73 | 22.440 | 67.78 | 158.865 | 100.00 | 1124.683 | 100.00 |
| 0.071 | 0.00 | 0.502 | 0.00 | 3.557 | 2.34 | 25.179 | 73.54 | 178.250 | 100.00 | 1261.915 | 100.00 |
| 0.080 | 0.00 | 0.564 | 0.00 | 3.991 | 3.10 | 28.251 | 78.57 | 200.000 | 100.00 | 1415.892 | 100.00 |
| 0.089 | 0.00 | 0.632 | 0.00 | 4.477 | 4.32 | 31.698 | 83.66 | 224.404 | 100.00 | 1588.656 | 100.00 |
| 0.100 | 0.00 | 0.710 | 0.00 | 5.024 | 5.82 | 35.566 | 87.86 | 251.785 | 100.00 | 1782.502 | 100.00 |
| 0.112 | 0.00 | 0.796 | 0.00 | 5.637 | 7.77 | 39.905 | 91.42 | 282.508 | 100.00 | 2000.000 | 100.00 |
| 0.126 | 0.00 | 0.893 | 0.00 | 6.325 | 10.22 | 44.774 | 94.32 | 316.979 | 100.00 | | |

**Fig. 32: Particle size distribution for ZS having 95% or more ZS-9 (Example 23)**

**Fig. 33 Correlation between serum potassium drops and ZS-9%**

Average Binding Site Width 3A

Fig. 34

Fig. 35: Decrease in Serum Potassium upon administration of ZS-9

**Efficacy: Acute Phase**

● Longitudinal model using all data through 48 hours)

| Dose | Mean S-K Reduction | Two-sided P-value |
|---|---|---|
| 10 g tid | 0.73 mmol/L | <0.0001 |
| 5 g tid | 0.62 mmol/L | <0.0001 |
| 2.5 g tid | 0.50 mmol/L | 0.0009 |
| 1.25 g tid | 0.33 mmol/L | 0.50 |
| Placebo | 0.26 mmol/L | – – – |

**Fig. 36**

**Efficacy: Subacute Phase**

● Longitudinal model using all data through Day 15

| Dose | Two-sided P-value |
|---|---|
| 10 g qd | <0.0001 |
| 5 g qd | 0.0075 |
| 2.5 g qid (B) | 0.84 |
| 1.25 g qid (A) | 0.43 |
| A vs. B | 0.41 |

**Fig. 37**

Fig. 38

Fig. 39

Fig. 40

**Serum Potassium (mmol/L)**

Legend:
- ≤5.3
- 5.4-5.5
- >5.5 mmol/L
- * $p < 0.05$ vs placebo

Placebo: n=95, n=22, n=40 (−0.2, −0.4, −0.4)
5g ZS-9: n=87, n=36, n=29 (−0.4*, −0.7*, −0.9*)
10g ZS-9: n=92, n=26, n=22 (−0.6*, −1.0*, −1.1*)

Fig. 41

**Mean Change From Baseline in Serum Bicarbonate-ZS-9 10g (n=24) vs Placebo (n=30)**

Fig. 42

EP 4 378 577 A2

EP 4 378 577 A2

**Mean Urinary pH-ZS-9 10g (n=24) vs Placebo (n=30)**

Legend:
- ○ Placebo
- ⊘ 10g ZS tid
- △ Time of dose administration
- * p<0.01 vs placebo.

X-axis: Time (day)

**Fig. 43**

Fig. 44

Fig. 45

**If Patients Become Normokalemic,
Then Proceed to Maintenance Phase**

CKD (any stage) and non-CKD

┌─── 2 DAYS ───┐

┌───────────── 12 DAYS ─────────────┐

| Induction Phase (DB, RCT) |
| Double Blind, Randomized Extended Phase |

DOSED: TID

DOSED: QD

| Placebo | → | 1.25g |
| | | 2.5g |

| 1.25g | → | Placebo |
| | | 1.25g |

| 2.5g | → | Placebo |
| | | 2.5g |

| 5g | → | Placebo |
| | | 5g |

| 10g | → | Placebo |
| | | 10g |

◆ Rapid Enrollment

◆ Representative Demographics

 – RAASi

 – CKD

 – CHF

 – Diabetes

◆ Predominantly US sites

**Primary Endpoint**
Rate of Change in S [$K^+$]
from Baseline to 48 Hours

**Secondary Endpoint**
Rate of Change in S [$K^+$]
from 48 Hours to Day 14

**Fig. 46**

Fig. 47A

EP 4 378 577 A2

**Overall Population**

Mean change in serum $K^+$ from Baseline to 48 hours (14 hours post last dose)

| Population | Parameter | Placebo | 1.25g ZS-9 | 2.5g ZS-9 | 5g ZS-9 | 10g ZS-9 |
|---|---|---|---|---|---|---|
| DM | P-value | NA | 0.87 | 0.0147 | <0.0001 | <0.0001 |
| | Mean $\Delta K^+$, mEq/L[a] | -0.25 | -0.25 | -0.47 | -0.52 | -0.74 |
| Overall | P-value | NA | 0.5 | 0.0009 | <0.0001 | <0.0001 |
| | Mean $\Delta K^+$, mEq/L[a] | -0.25 | -0.30 | -0.46 | -0.54 | -0.73 |

**Fig. 47B**

EP 4 378 577 A2

**Fig. 48A**

EP 4 378 577 A2

**Fig. 48B**

**Fig. 48C**

**Fig. 49**

**Patients with AEs Percent**

Fig. 50

## DM Subgroup

## Overall Population

| P-value | 5g ZS-9 | 10g ZS-9 |
|---|---|---|
| DM Subgroup | <0.0001 | <0.0001 |
| Overall Population | 0.0075 | <0.0001 |

**Fig. 51**

Fig. 52

FIG. 53

Fig. 54

Study 004 Mean Serum K+ (MP, All Subjects ): 5g, 10g, and 15g vs. PBO Serum Potassium (mEq/L)

Legend:
- ⊘ Placebo (n=82)
- ⊘ 5g ZS (n=45)
- ⊘ 10g ZS (n=50)
- ⊘ 15g ZS (n=54)
- \* Two-sample t-test p-value $<0.05$
- △ Dose

Time (day)

**Fig. 55**

EP 4 378 577 A2

**Fig. 56**

EP 4 378 577 A2

**Fig. 57**

Fig. 58

EP 4 378 577 A2

Fig. 59

**Acute Phase Proportion of Subjects Normokalemia by Kaplan- Meier Estimate**

ZS Pharma

Secondary Endpoint :

Proportion of Subjects Who Achieve Normokalemia, 24 and 48 Hours

|  | 24 hour | 48 hour |
|---|---|---|
| **Percent Normalized (%)** (kaplan-Meier estimate) | 84.28 | 97.62 |

10g ZS-9 TID met this secondary endpoint

**Fig. 60**

EP 4 378 577 A2

**Acute Phase Time to Normalization**

ZS Pharma

Secondary Endpoint :
Time to Normalization, AP Phase 48hr

| | 10g (n=258) |
|---|---|
| **Median Time-to-Normalization** (hours) | 2.17 |

Median Normalization in 130 Minutes and 12 Seconds!

**Fig. 61**

**Fig. 62**

EP 4 378 577 A2

**Fig. 63**

**Fig. 64**

**No Change in BUN at End of Study**

*ZS Pharma*

**Study 004 Mean BUN Chg from Acute BL (MP, Day 29/Exit): 5g, 10g and 15g vs. PBO Blood Urea Nitrogen (mg/dL)**

Legend:
- ☒ Placebo (n=80)
- ☒ 5g ZS (n=44)
- ☒ 10g ZS (n=48)
- ☒ 15g ZS (n=52)
- * Two sample t-test p-value <0.05

0.0
-0.5
-3.0
-1.6

**Day 29/Exit**

**Fig. 65**

**Fig. 66**

Fig. 67

Fig. 68

EP 4 378 577 A2

**Change from Maintenance Phase Renin**

ZS Pharma

**Study 004 Mean Renin Chg from Acute BL (MP): 5g, 10g and 15g vs. PBO P-Renin (ng/mL/hr)**

3.0

0.8

-2.3*

-0.3

**Day 29/Exit**

☑ Placebo (n=59)

◹ 5g ZS (n=29)

☑ 10g ZS (n=32)

◿ 15g ZS (n=31)

\* Two sample t-test p-value <0.05

**Fig. 69**

EP 4 378 577 A2

**Fig. 70**

Fig. 71

**Fig. 72**

R.6023

R.1150

.3300

.6600

.0600
CUP DEPTH

R.2433

.0050 BLENDED LAND

A

DETAIL A
SCALE 14:1

R1.7000

R.2430

UPPER
LOWER

**Fig. 73**

ACTUAL SCALE

.4600

R.1650

R.7296

.8560

.0450
CUP DEPTH

R.2000

R1.1250

.0060 BLENDED LAND

R.2500    R4.0000

.2538
WEIGHT = 1240mg
APPROX.

LOWER

**Fig. 74**

ACTUAL SCALE

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 61901886
- US 61914354 B
- US 61930328 B
- US 61930336 B
- US 62005484 B
- US 62015215 B
- US 62053732 B
- US 6579460 B
- US 6099737 A
- US 6332985 B
- US 6814871 B
- US 5891417 A
- US 5888472 A
- US 371080
- US 20120213847 A1
- US 829415
- US 20130334122
- US 61670415

**Non-patent literature cited in the description**

- **REMUZZI et al.** The role of renin-angiotensin-aldosterone system in the progression of chronic kidney disease. *Kidney Int'l,* 2005, vol. 68 (99), S57-S65
- **ZHANG et al.** Aldosterone induces epithelial-mesenchymal transition via ROS of mitochondrial origin. *Am J Physiol Renal Physiol,* 2007, vol. 293
- **PONDA et al.** Aldosterone Antagonism in Chronic Kidney Disease. *Clin J Am Soc Nephol,* 2006, vol. 1, 668-677
- **U. WENZEL.** Aldosterone and Progression of Renal Disease. *Current Opinion in Nephrology and Hypertension,* 2008, vol. 17, 44-50
- **REMUZZI et al.** The Aggravating Mechanisms of Aldosterone on Kidney Fibrosis. *J Am Soc Nephrol,* 2008, vol. 19, 1459-1462
- **NAVANEETHAN et al.** Aldosterone Antagonists for Preventing the Progression of Chronic Kidney Disease: A Systematic Review and Meta-analysis. *Am Soc Neph,* 2008
- **BRIET et al.** Aldosterone: effects on the kidney and cardiovascular system. *Nature Reviews: Nephrology,* 2010, vol. 6, 261-273
- **R TOTO.** Aldosterone blockade in chronic kidney disease: can it improve outcome?. *Current Opinion in Nephrology and Hypertension,* 2010, vol. 19, 444-449
- **TURNER et al.** Treatment of chronic kidney disease. *Kidney Int'l,* 2012, vol. 81, 351-362
- **ROCHA et al.** Selective Aldosterone Blockade Prevents Angiotensin II/Salt-Induced Vascular Inflammation in the Rat Heart. *Endocrinology,* 2002, vol. 143 (12), 4828-4836
- **ROCHA et al.** Aldosterone Induces a Vascular Inflammatory Phenotype in the Rat Heart. *Am J Phsiol Heat Circ Physiol,* 2002, vol. 283, H1802-H1810
- **TOMASCHITZ et al.** Plasma aldosterone levels are associated with increased cardiovascular mortality: the Ludwigshafen Risk and Cardiocascular Health (LURIC) study. *European Heart Journal,* 2010, vol. 31, 1237-1247
- **HORN ; HANSTEN.** Hyperkalemia Due to Drug Interactions. *Pharmacy Times,* January 2004, 66-67
- **DESAI.** Hyperkalemia Associated with Inhibitors of the Renin-Angiotensin-Aldosterone System: Balancing Risk and Benefit. *Circulation,* 2008, vol. 118, 1609-1611
- **FERREIRA et al.** *Inor Chim Acta,* 2003, vol. 356, 19